(19) Europäisches Patentamt
European Patent Office
Office européen des brevets

(11) EP 4 293 020 A1

(12) EUROPEAN PATENT APPLICATION
published in accordance with Art. 153(4) EPC

(43) Date of publication:
**20.12.2023 Bulletin 2023/51**

(21) Application number: **22752300.8**

(22) Date of filing: **09.02.2022**

(51) International Patent Classification (IPC):
***C07D 403/14*** (2006.01) ***A61P 37/02*** (2006.01)
***A61K 47/54*** (2017.01)

(52) Cooperative Patent Classification (CPC):
**A61K 9/06; A61K 9/08; A61K 9/10; A61K 9/107; A61K 9/12; A61K 9/70; A61K 31/519; A61K 47/54; A61P 1/00; A61P 3/00; A61P 9/00; A61P 17/00; A61P 17/06; A61P 19/02; A61P 21/04;** (Cont.)

(86) International application number:
**PCT/CN2022/075714**

(87) International publication number:
**WO 2022/171140 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.02.2021 CN 202110182307**

(71) Applicants:
- **Minghui Pharmaceutical (Hangzhou) Limited**
  **Hangzhou, Zhejiang 310018 (CN)**
- **Minghui Pharmaceutical (Shanghai) Limited**
  **Pilot Free Trade Zone**
  **Pudong New Area Shanghai 201203 (CN)**

(72) Inventors:
- **YAO, Yuanshan**
  **Shanghai 201203 (CN)**
- **LI, Ao**
  **Shanghai 201203 (CN)**
- **SHI, Junwei**
  **Shanghai 201203 (CN)**
- **CAO, Guoqing**
  **Shanghai 201203 (CN)**

(74) Representative: **Mewburn Ellis LLP**
**Aurora Building**
**Counterslip**
**Bristol BS1 6BX (GB)**

# (54) PRODRUG COMPOUND, PREPARATION METHOD THEREFOR AND USE THEREOF

(57) The present invention provides a prodrug compound, a preparation method, and the use thereof. In particular, the present invention provides a compound as represented by formula (I), a preparation method, and the use thereof as a prodrug for preparing topical formulations.

EP 4 293 020 A1

(52) Cooperative Patent Classification (CPC): (Cont.)
**A61P 25/28; A61P 29/00; A61P 35/00;
A61P 37/02; A61P 37/06; C07D 403/14;
C07D 487/04**

## Description

### Technical field

The present invention relates to the field of small molecule drugs, specifically, the present invention provides a class of compounds which can be used as kinase inhibitor prodrugs, and pharmaceutically acceptable salts, hydrates or solvates thereof, as well as pharmaceutical compositions (preferably topical formulations) thereof.

### Background

JAK-STAT signaling pathway is a cytokine-stimulated signal transduction pathway discovered in recent years. JAK plays an important role in cytokine signal transduction. The downstream substrates of the JAK kinase family include signal transducer and activator of transcription (STAT). JAK proteins are important members of this pathway, and the abnormal increases in their activity often lead to disease occurrence. Many diseases are associated with abnormal cellular responses to the JAK-STAT signaling pathway, including autoimmune, inflammatory, bone disease, metabolic disease, neuropathy and neurodegenerative disease, cancer, cardiovascular disease, allergic reactions and asthma, Alzheimer's disease.

Rheumatoid arthritis (RA) is a common chronic autoimmune disease in clinic, characterized by joint swelling, pain, stiffness, deformity and severely impaired functionality, etc, and the prevalence in the population is 0.5%-1.0%. Since the pathogenesis of RA has not yet been clarified, its pathological process is difficult to control, and causes high disability rate, which seriously damages the physical and mental health of patients and reduces the quality of life. At present, the drugs used to treat RA mainly include nonsteroidal anti-inflammatory drug (NSAID), disease-modifying antirheumatic drug (DMARD), and antibody drugs. For a long time, the first-line drugs for the treatment of RA are DMARDs. In 1988, the 1st DMARD, methotrexate (MTX), was approved by the FDA for the treatment of RA, which is an important milestone in the history of RA treatment. The drug is widely used due to its effectiveness, tolerability, and safety, but it also causes adverse effects including nausea, vomiting, stomach discomfort, and hepatotoxicity. In contrast, the newly developed antibody-based drugs have better efficacy and safety indexes for treating moderate-to-severe RA. However, they target to specific cytokines, which significantly limited the scope of beneficial patients. Meanwhile, the cost of treatment and the injection mode of drug delivery have also limited the promotion of these drugs.

In the past 20 years of development, RA treatment has made significant progress, and the patient's condition can be effectively controlled with existing treatments. Nonetheless, RA patients still experience problems such as disease recurrence, suboptimal treatment effectiveness, poor long-term tolerability, and a number of adverse effects. More importantly, the quality of life of RA patients, including organ function such as joints, has not been truly improved by existing treatments. Therefore, there is still a huge unmet clinical need in this field to focus on restoring normal function of patients.

Studies have shown that the core pathogenicity of RA relates with the large number of cytokines produced by autocrine secretion of monocytes/macrophages and lymphocytes infiltrated in the synovial tissues and cells of RA. These cytokines interact with each other and activate the JAK/STAT signaling pathway (Janus kinase/Signal transducer and activators of transcription signaling pathway) by different means. The cascade amplification of these cytokines can be blocked by specifically inhibiting the JAK/STAT signaling pathway, so as to improve the symptoms of the damaged joints of RA patients. Therefore, the JAK/STAT signaling pathway has become a potential target for the treatment of RA.

Since JAK kinase is involved in various important in vivo physiological processes, extensive inhibition of different isoforms may lead to adverse effects. Tofacitinib is used in patients with moderate-to-severe RA who have inadequate or intolerant response to MTX, and certain adverse effects has been observed in clinical trials, including infections, tuberculosis, tumors, anemia, liver injury, and cholesterol increase. Tofacitinib showed significant inhibitory activity against JAK1, JAK2, and JAK3 isoforms. Since JAK2 activity is associated with erythrocyte differentiation as well as lipid metabolism processes, some of the above-mentioned adverse effects are considered to relate with the non-selective inhibitory characteristics of the drug. Therefore, the search for selective JAK1 and/or JAK3 inhibitors will become a new direction in the research of RA drugs. At present, JAK inhibitors have been proved to be used as therapeutic agents for hematologic diseases, oncology, rheumatoid arthritis and psoriasis. However, there is still a very limited number of JAK inhibitor compositions in the field that can be used for topical administration.

### Summary

In a first aspect of the present invention, it provides a prodrug molecule of a pharmaceutical compound G', and pharmaceutically acceptable salts, hydrates or solvates thereof, characterized in that the hydrophobicity coefficient CLogP of the drug molecule G' is less than 4. And the prodrug molecule has the structure shown in formula (I):

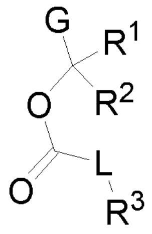

(I)

wherein, G is a partial structural fragment formed by the loss of H atoms in the drug molecule G', and connected to

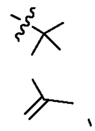

via any N, O or S atoms within the molecule;

$R^1$ and $R^2$ are each independently selected from the group consisting of H, D, substituted or unsubstituted C1-C6 alkyl, substituted or unsubstituted C1-C6 heteroalkyl, substituted or unsubstituted C3-C8 cycloalkyl, and substituted or unsubstituted 3-8 membered heterocyclyl, or $R^1$ and $R^2$, together with the carbon atoms connected thereto, form a C3-C8 carbocycle or heterocycle;

L is selected from the group consisting of chemical bond, substituted or unsubstituted C1-C6 alkylene, and substituted or unsubstituted C1-C6 heteroalkylene;

$R^3$ is selected from the group consisting of substituted or unsubstituted C1-C20 alkyl (linear or branched), substituted or unsubstituted C3-C20 cycloalkyl, substituted or unsubstituted C1-C20 heteroalkyl, substituted or unsubstituted 3-20 membered heterocyclyl, and substituted or unsubstituted C6-C14 aryl, or $R^3$ is connected to $R^1$ or $R^2$ so as to form a substituted or unsubstituted 5-20 membered lactone ring or heterolactone ring, wherein the heterolactone ring refers to the cyclic backbone of the lactone ring comprising 1-3 heteroatoms selected from the group consisting of N, O and $S(O)_p$;

wherein, the heteroalkyl refers to one or more carbon atoms on the carbon chain being replaced by heteroatom selected from the group consisting of N, O and $S(O)_p$;

the heterocyclyl comprises 1-3 heteroatoms selected from the group consisting of N, O and $S(O)_p$;

p is selected from 0, 1 or 2;

unless otherwise specified, the "substituted" means being substituted by one or more (e.g. 2, 3, 4, etc.) substituents selected from the group consisting of deuterium, halogen, C1-C6 alkyl, halogenated C1- C6 alkyl, C1-C6 alkoxy, halogenated C1-C6 alkoxy, C3-C8 cycloalkyl, halogenated C3-C8 cycloalkyl, C3-C8 heterocyclyl, oxo, -CN, hydroxyl, amino, carboxyl, amide, sulfonamide, sulfonyl, and a group which is unsubstituted or substituted by one or more substituents, wherein the group is selected from C6-C10 aryl, halogenated C6-C10 aryl, 5-10 membered heteroaryl having 1-3 heteroatoms selected from N, S and O, and halogenated 5-10-membered heterocyclyl having 1-3 heteroatoms selected from N, S and O; and the substituents selected from the group consisting of: halogen, C1-C6 alkyl, C1-C6 alkoxy, and =O.

In another preferred embodiment, the drug molecule G' is selected from the group consisting of JAK inhibitors, MEK inhibitors, and BTK inhibitors.

In another preferred embodiment, the JAK inhibitor is selected from the group consisting of INCB-52793, ATI-502, deuterium-modified ruxolitinib analog, ATI-501, R-348, NS-018, Jaktinib, KL-130008, DTRMHS-07, WXSH-0150, TQ05105, WXFL10203614, and a molecule selected from the following group, or pharmaceutically acceptable salts, hydrates, or solvates thereof:

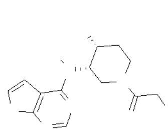

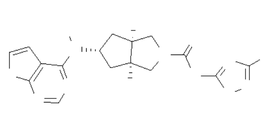

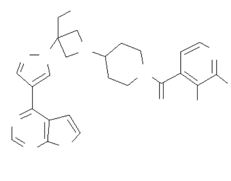

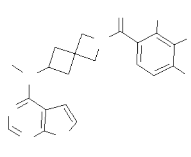

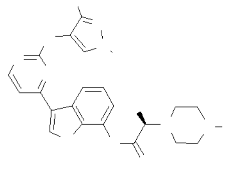 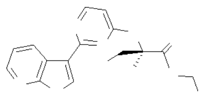 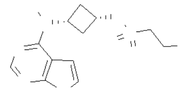 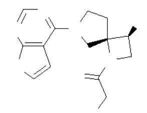

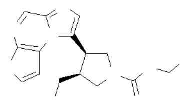 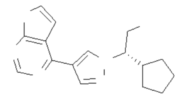 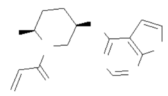 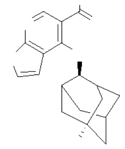

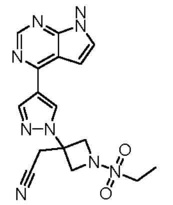 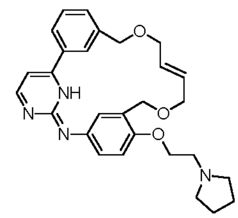 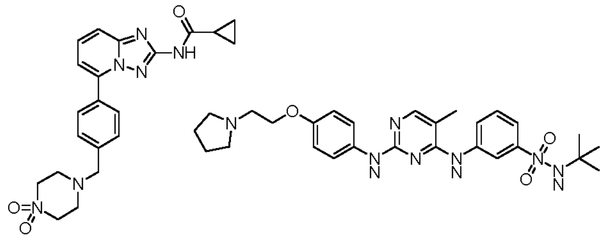

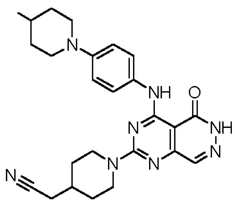 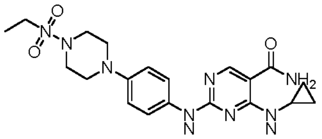 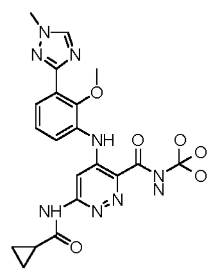 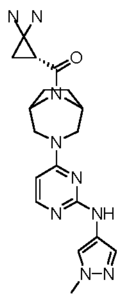

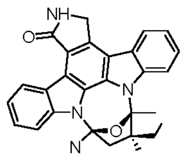 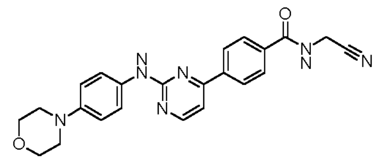 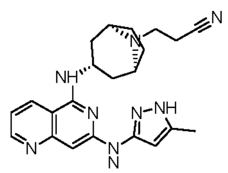 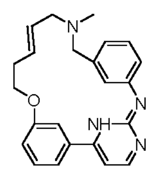

In another preferred embodiment, the MEK inhibitor is a molecule selected from the following group, or pharmaceutically acceptable salts, hydrates, or solvates thereof:

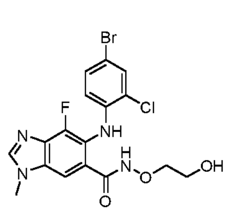 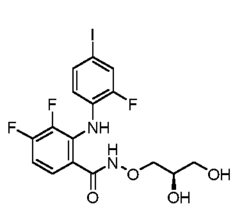 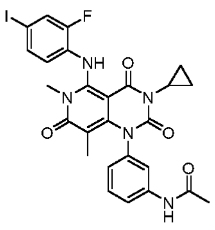 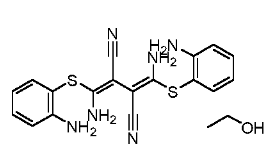

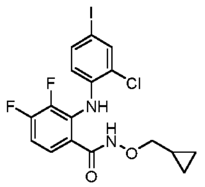 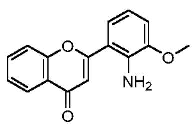 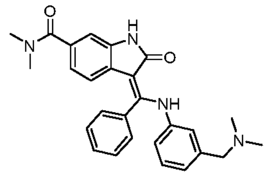 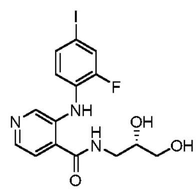

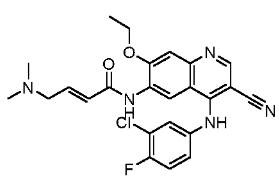 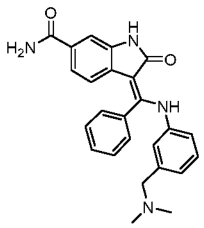 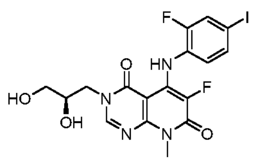 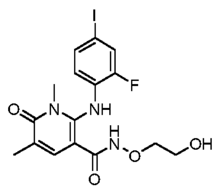

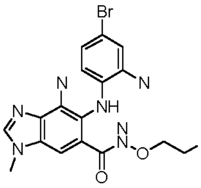 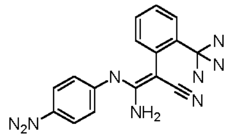 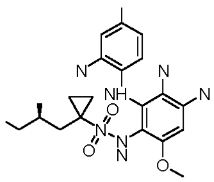 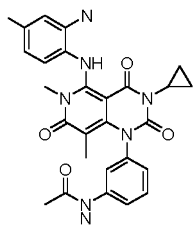 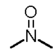

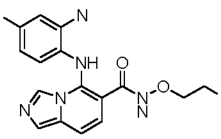 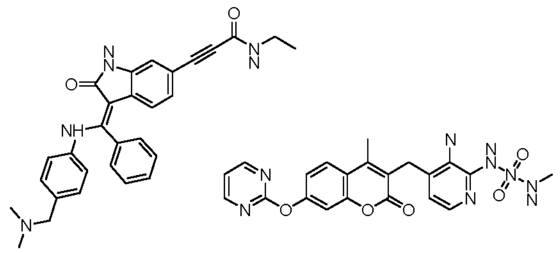 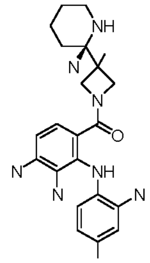

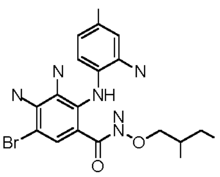 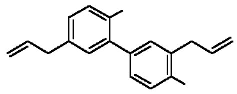 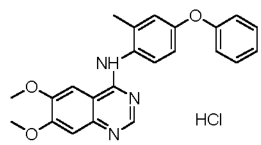 HCl 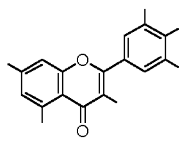 .

In another preferred embodiment, the BTK inhibitor is a molecule selected from the following group, or pharmaceutically acceptable salts, hydrates, or solvates thereof:

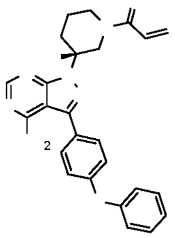 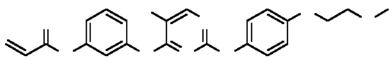 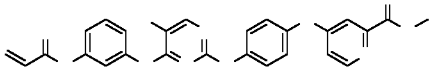

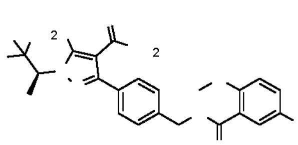
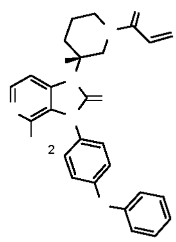
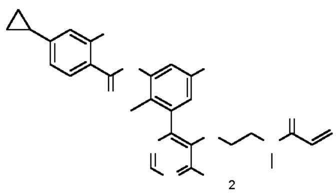
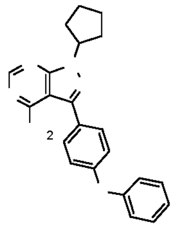

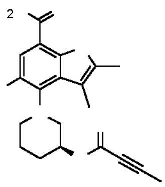
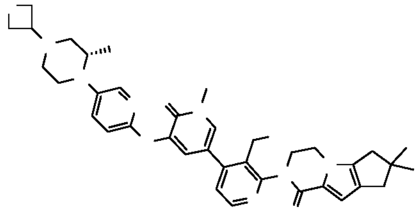
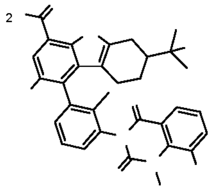
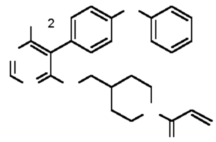

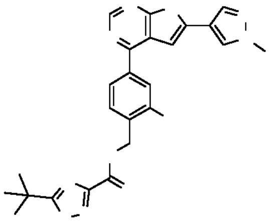
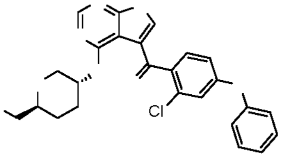
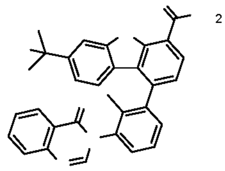
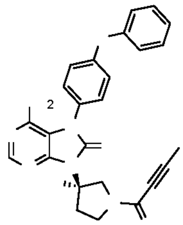

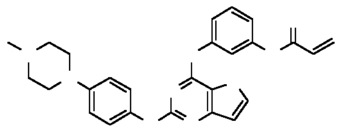
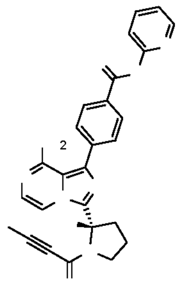
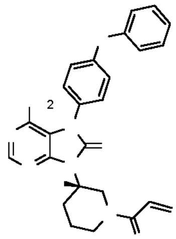
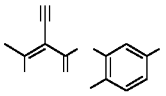

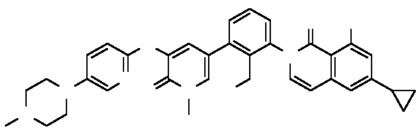
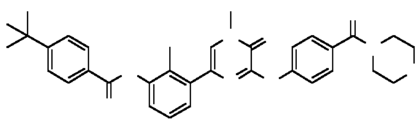

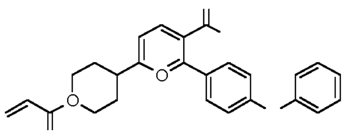
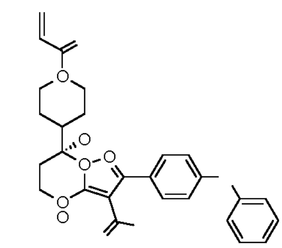
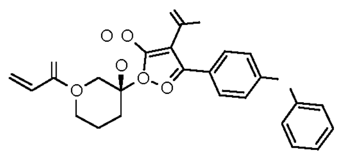

.

In another preferred embodiment, the drug molecule G' comprises structure A, and A is an optionally substituted 5-20 membered heteroaryl or an optionally substituted 5-20 membered heterocyclyl, wherein the heteroaryl or heterocyclyl comprises one or more heteroatoms selected from N, S and O, and G' is connected to

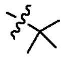

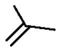,

via one of the heteroatoms in A.

In another preferred embodiment, G' has a CLogP of less than 3.

In another preferred embodiment, G' has a CLogP of less than 2.

In another preferred embodiment, G' has a molecular weight of less than 900 Da.

In another preferred embodiment, G' has a molecular weight of less than 700 Da.

In another preferred embodiment, G' has a molecular weight of less than 500 Da.

In another preferred embodiment, G' has a molecular weight of no less than 900 Da.

In another preferred embodiment, G' is connected to

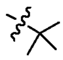

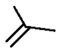,

via a N atom.

In another preferred embodiment, G' is connected to

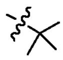

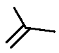,

via a S atom.

In another preferred embodiment, G' is connected to

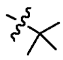

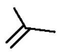,

via an O atom.

In another preferred embodiment, the structure of A is 5-20 membered heteroaryl.

In another preferred embodiment, $R^1$ and $R^2$ are each independently H, D, or C1-C6 alkyl, L is chemical bond or C1-C6 alkylene, and $R^3$ is selected from the group consisting of C1-C20 alkyl, C3-C20 cycloalkyl, C6-C14 aryl; wherein the alkyl, alkylene, cycloalkyl, and aryl can be optionally substituted by substituents selected from the group consisting of halogen, and C1-C4 alkyl.

In another preferred embodiment, $R^1$ and $R^2$ are each independently H, L is chemical bond, and $R^3$ is selected from the group consisting of C1-C20 alkyl; wherein the alkyl can be optionally substituted by substituents selected from the group consisting of halogen, and C1-C4 alkyl.

In another preferred embodiment, $R^3$ is optionally substituted $C_{1-20}$ alkyl or substituted phenyl.

In another preferred embodiment, $R^3$ is optionally substituted $C_{1-12}$ alkyl.

In another preferred embodiment, $R^3$ is optionally substituted $C_{10-15}$ alkyl.

In another preferred embodiment, $R^3$ is a linear chain alkyl.

In another preferred embodiment, $R^3$ is a branched chain alkyl.

In another preferred embodiment, G group is selected from the group consisting of:

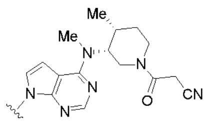
Tofacitinib

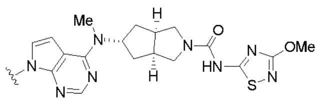
SHR-0302

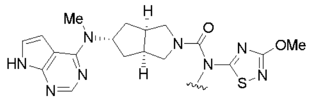
SHR-0302

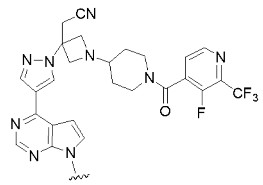
Itacitinib

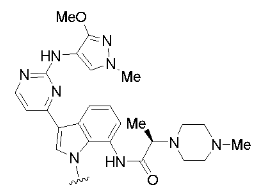
AZD-4205
2091134-68-6

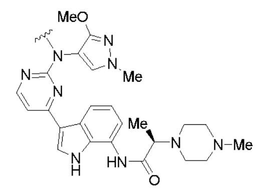
AZD-4205
2091134-68-6

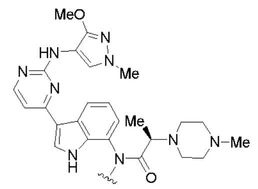
AZD-4205
2091134-68-6

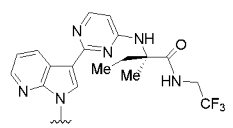
Decernotinib

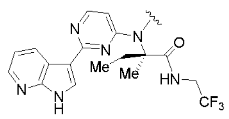
Decernotinib

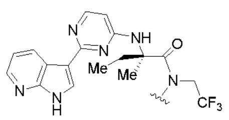
Decernotinib

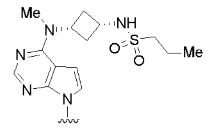
PF-04965842(Abrocitinib)

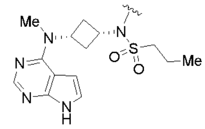
PF-04965842(Abrocitinib)

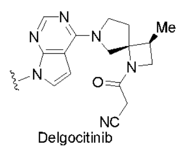
Delgocitinib

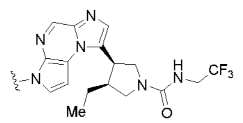
Upadacitinib

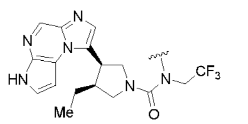
Upadacitinib

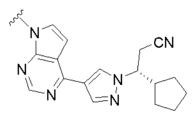
Ruxolitinib

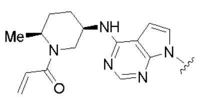
PF-06651600

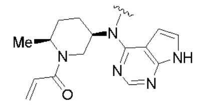
PF-06651600

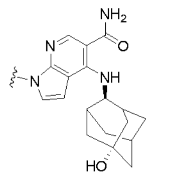
Peficitinib

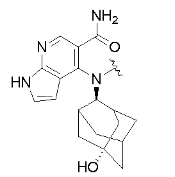
Peficitinib

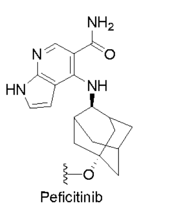
Peficitinib

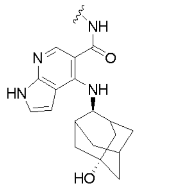
Peficitinib

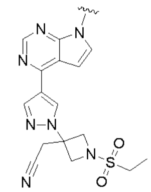
Baricitinib

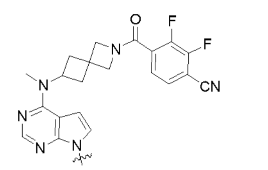

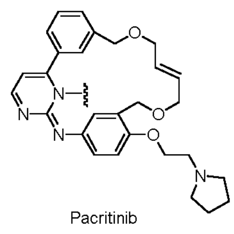
Pacritinib

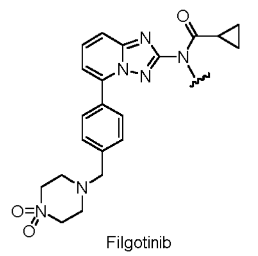
Filgotinib

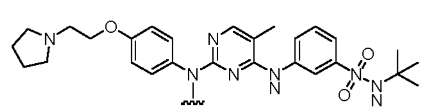
Fedratinib

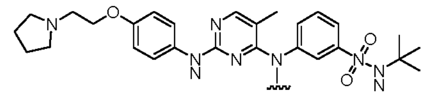

Fedratinib

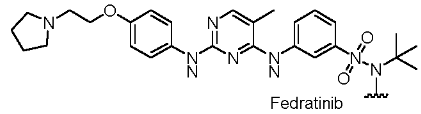

Fedratinib

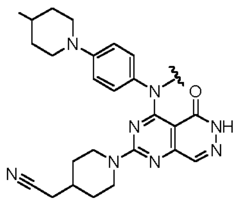

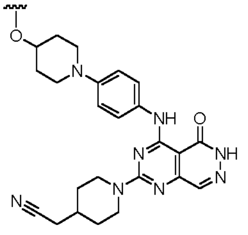

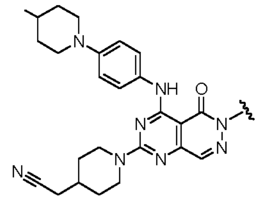

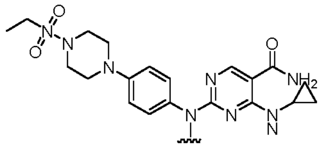

Cerdulatinib

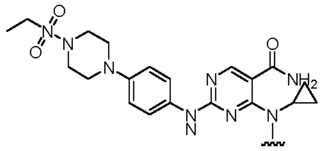

Cerdulatinib

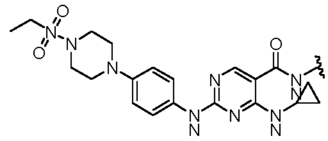

Cerdulatinib

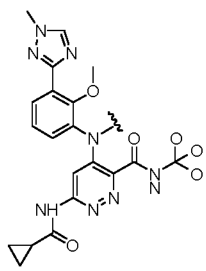

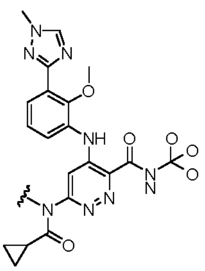

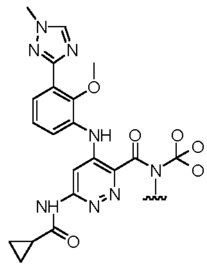

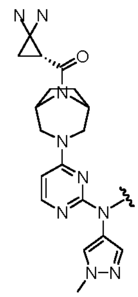

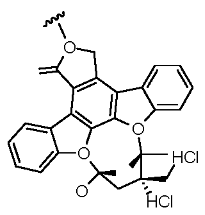

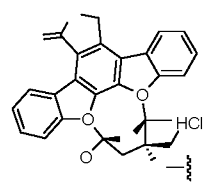

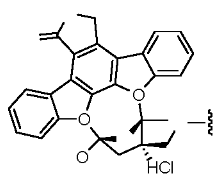

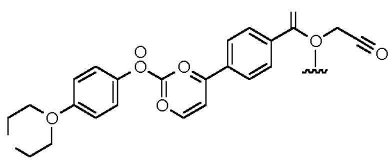

Momelotinib

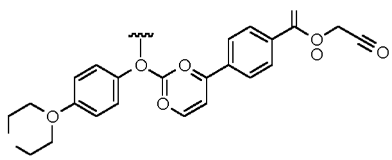

Momelotinib

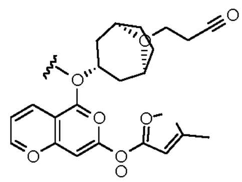

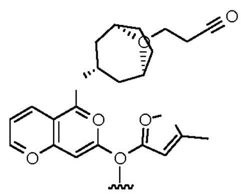

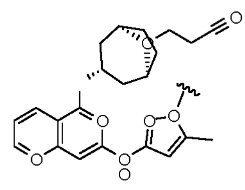

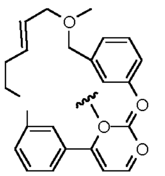

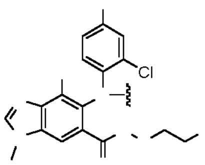
Selumetinib

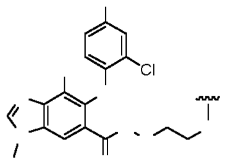
Selumetinib

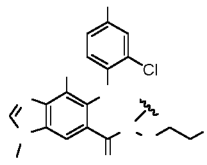
Selumetinib

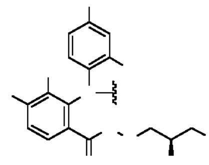
Mirdametinib

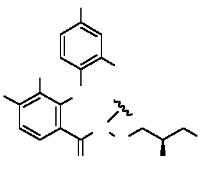
Mirdametinib

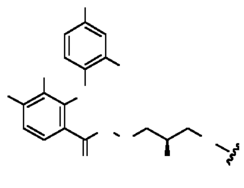
Mirdametinib

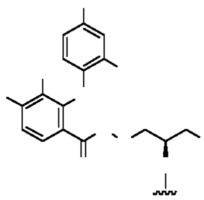
Mirdametinib

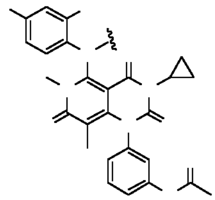

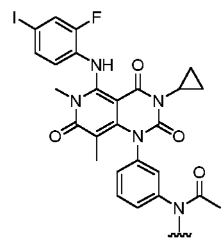
Trametinib (GSK1120212)

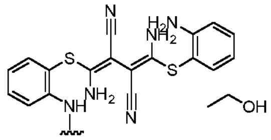
U0126

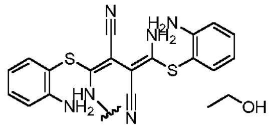
U0126

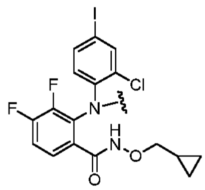
PD184352 (CI-1040)

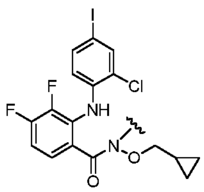
PD184352 (CI-1040)

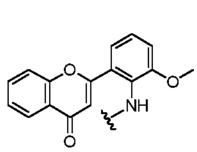
PD98059

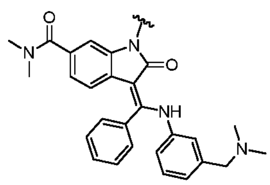
BIX 02189

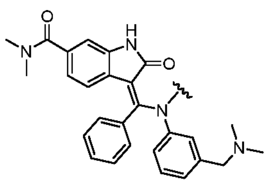
BIX 02189

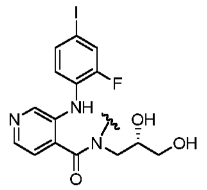
Pimasertib (AS-703026)

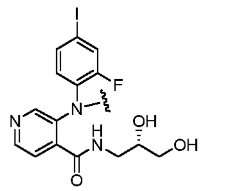
Pimasertib (AS-703026)

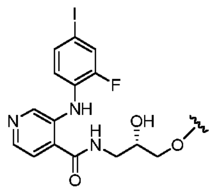
Pimasertib (AS-703026)

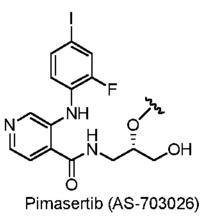
Pimasertib (AS-703026)

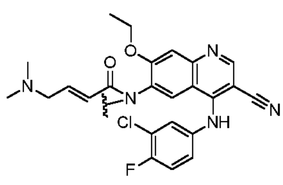
Pelitinib (EKB-569)

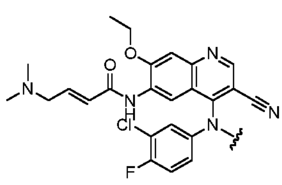
Pelitinib (EKB-569)

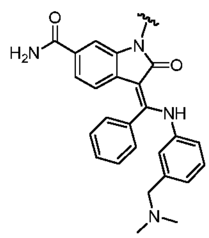
BIX 02188

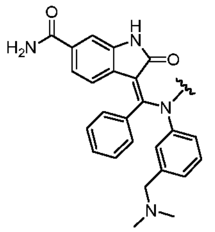
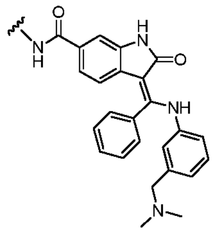

BIX 02188 BIX 02188

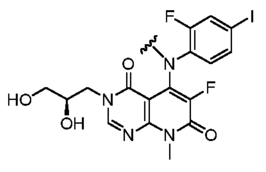
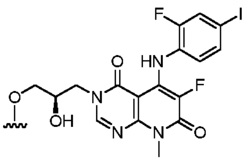

TAK-733 TAK-733

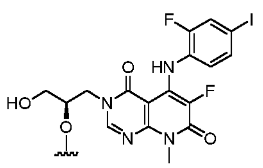
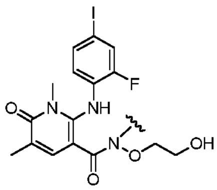
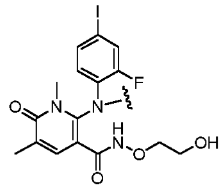
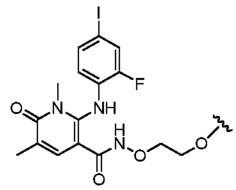

TAK-733 AZD8330 AZD8330 AZD8330

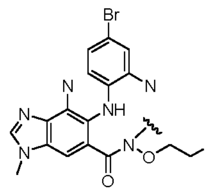
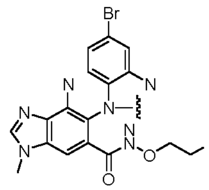
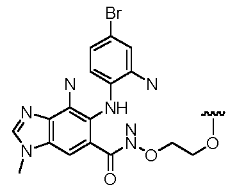
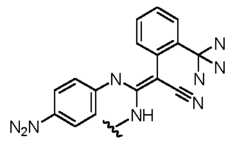

Binimetinib (MEK162) Binimetinib (MEK162) Binimetinib (MEK162)

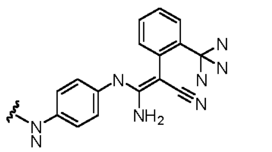
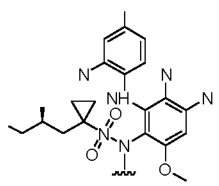
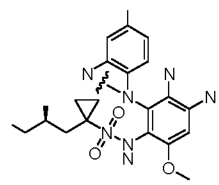
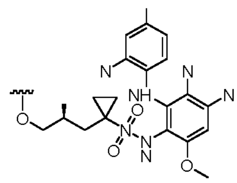

Cerdulatinib Cerdulatinib Cerdulatinib

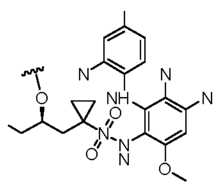
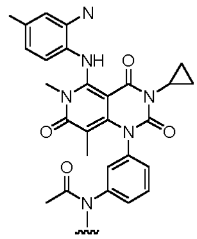
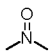
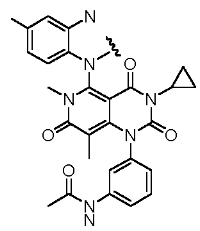
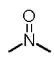
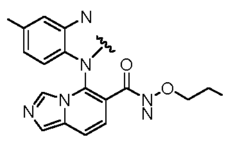

Cerdulatinib Binimetinib

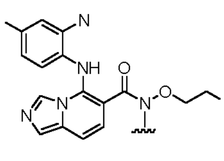
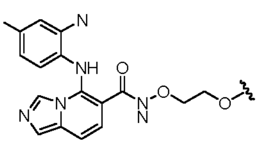
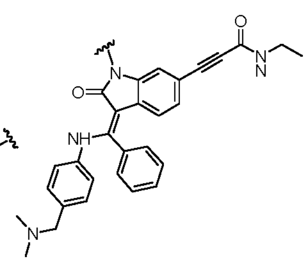
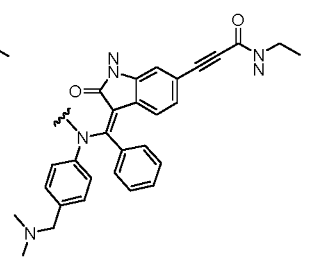

Binimetinib Binimetinib

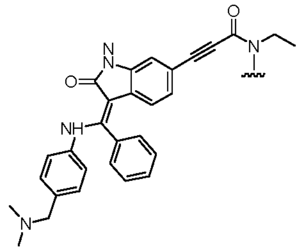

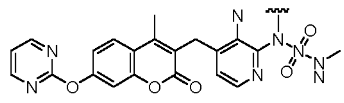

Cerdulatinib

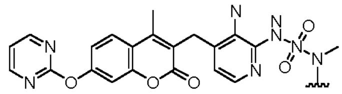

Cerdulatinib

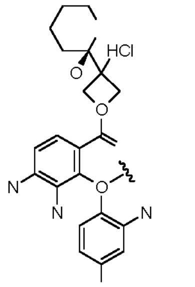

(GDC-0973)

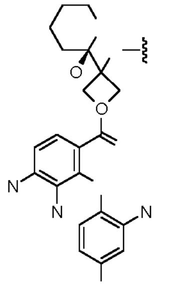

(GDC-0973)

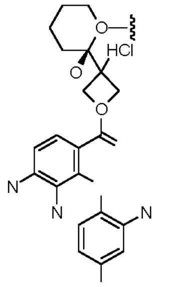

(GDC-0973)

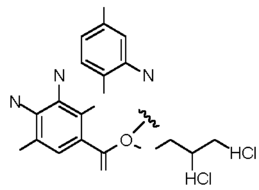

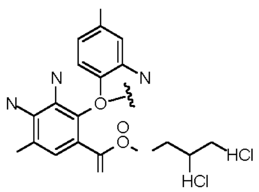

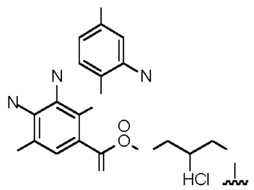

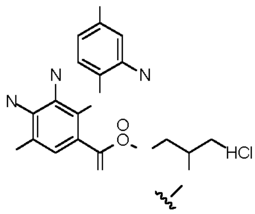

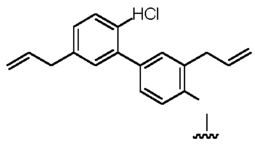

293100)

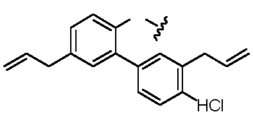

293100)

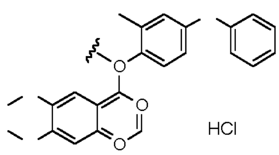

HCl

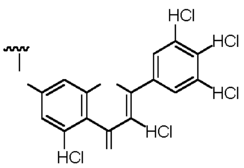

Myricetin

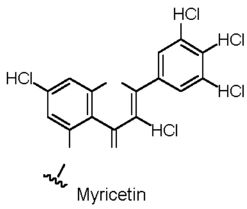

Myricetin

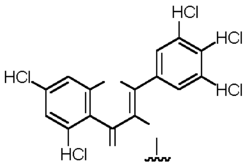

Myricetin

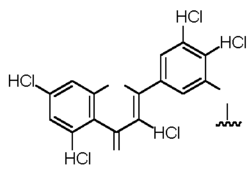

Myricetin

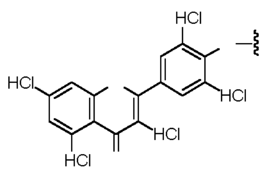

Myricetin

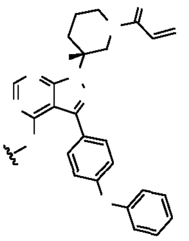

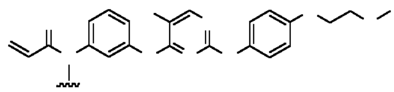

Filgotinib

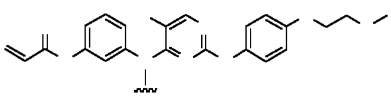

Filgotinib

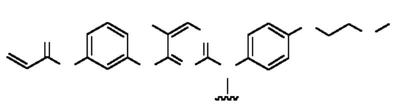

Filgotinib

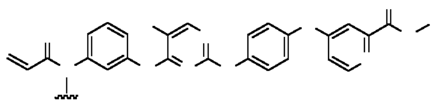

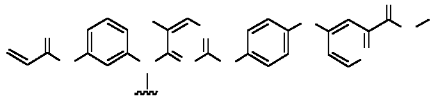

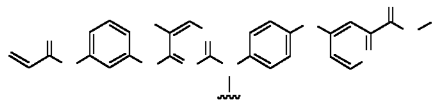

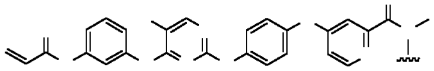

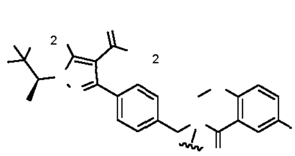

Selumetinib

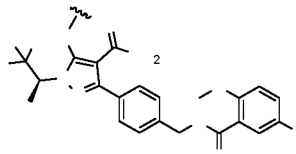

Pirtobrut

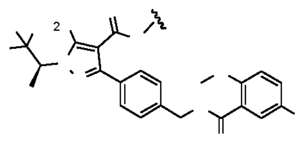

Selumetinib

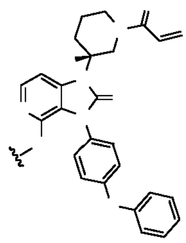

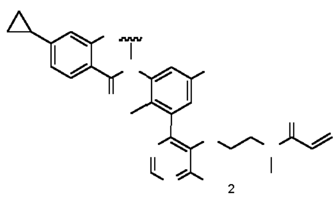

Remibrutinib (LOU064)

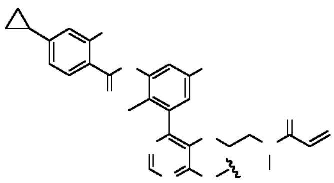

Remibrutinib (LOU064)

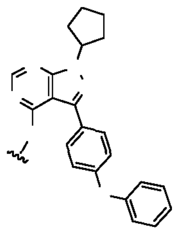

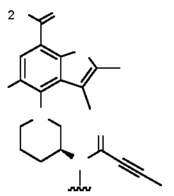

Branebrutinib (BMS-986195)

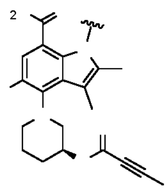

Branebrutinib (BMS-986195)

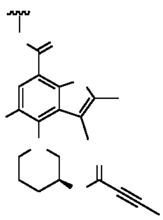

Branebrutinib (BMS-986195)

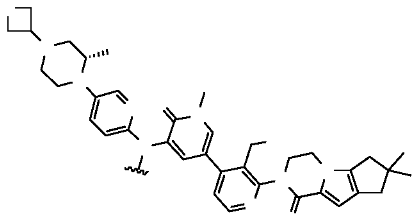

Remibrutinib

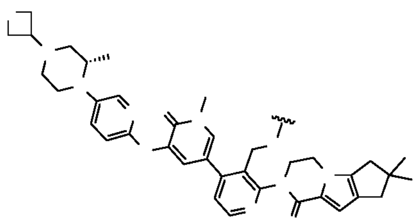

Remibrutinib

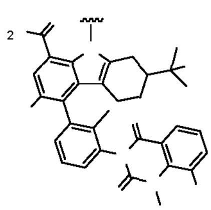

Mirdametinib

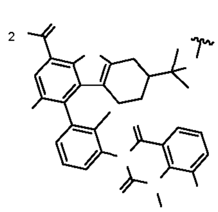

Mirdametinib

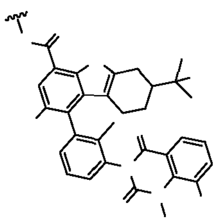

Mirdametinib

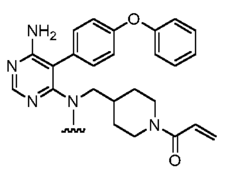

Evobrutinib (M-2951)

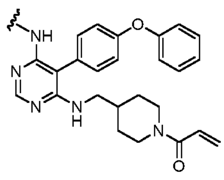

Evobrutinib (M-2951)

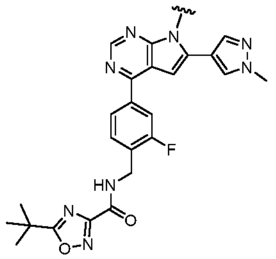

CAS: 2230724-66-8

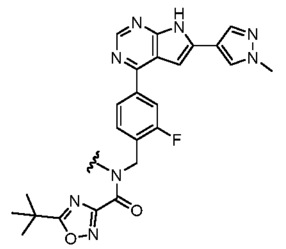

CAS: 2230724-66-8

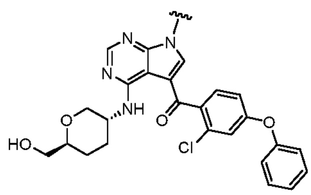

Nemtabrutinib (ARQ 531)

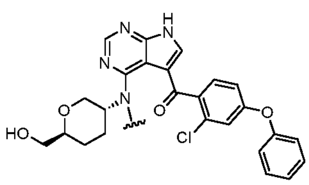

Nemtabrutinib (ARQ 531)

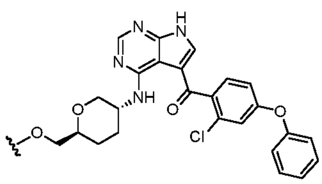

Nemtabrutinib (ARQ 531)

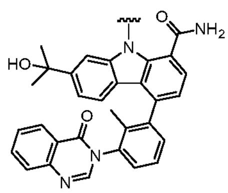

BMS-935177

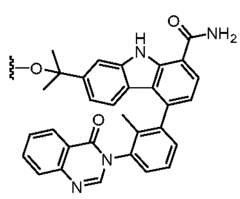

BMS-935177

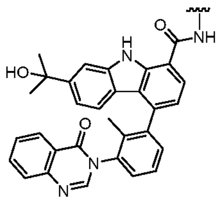

BMS-935177

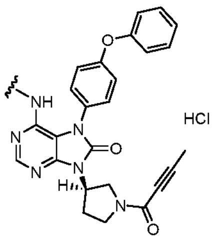

HCl

tirabrutinib(ONO-4059) hydrochloride

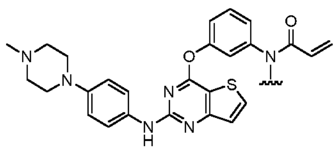

Olmutinib (BI 1482694)

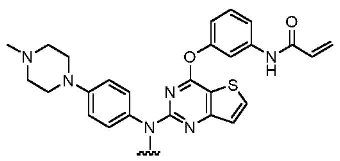

Olmutinib (BI 1482694)

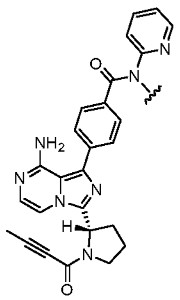

Acalabrutinib (ACP-196)

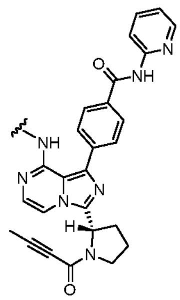

Acalabrutinib (ACP-196)

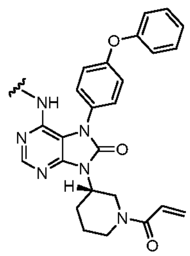

ONO-4059 analogue

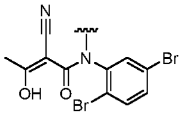

LFM-A13

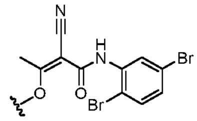

LFM-A13

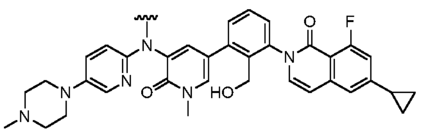

RN486

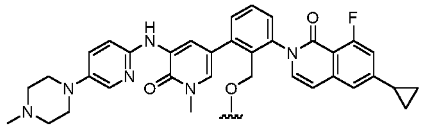
RN486

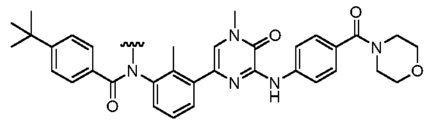
CGI1746

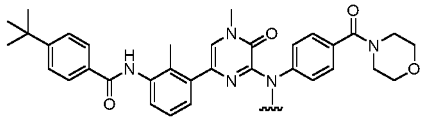
CGI1746

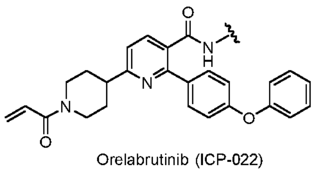
Orelabrutinib (ICP-022)

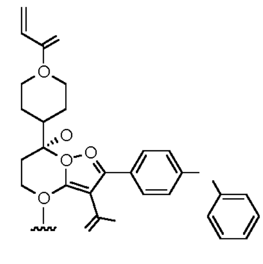
Zanubrutinib

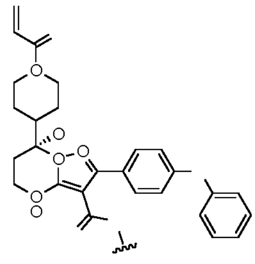
Zanubrutinib

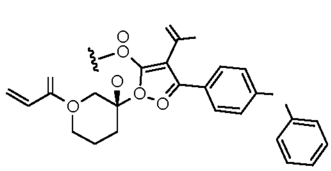

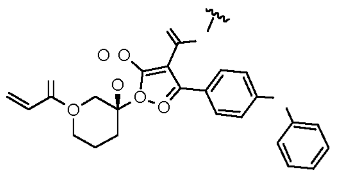
.

In another preferred embodiment, G group is selected from the group consisting of:

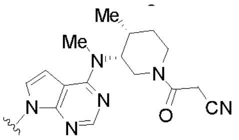

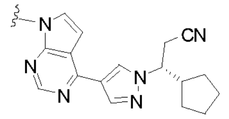

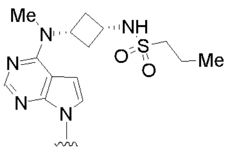

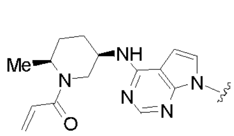

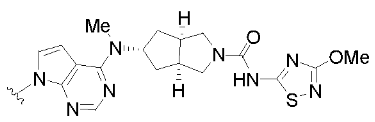

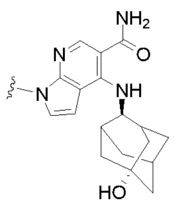

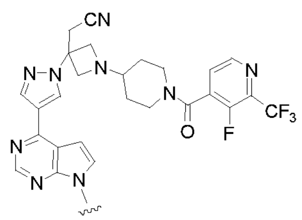

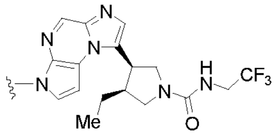

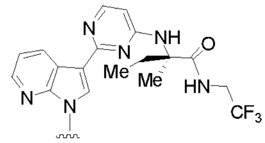

In another preferred embodiment, the compound of formula (I) has the structure shown in formula (IIB), (IIC) or (IID):

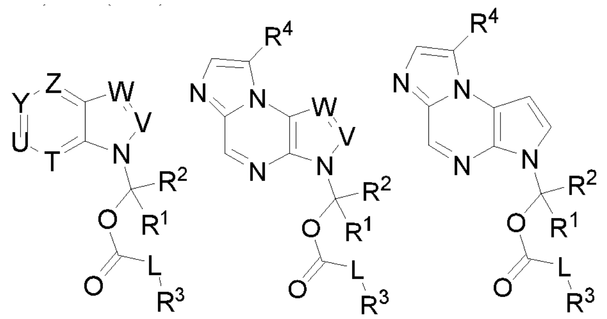

**(IIB)** **(IIC)** **(IID)**

wherein,

Z, T, U, V, and W are each independently N or $CR^4$;
Y is N or $CR^5$;
wherein $R^4$ and $R^5$ are each independently selected from the group consisting of H, halogen, -CN, $-C(O)NH_2$, and

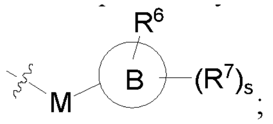

M is selected from the group consisting of chemical bond, C(O), C(O)O, S(O), $S(O)_2$, $NR^8$, 5-7 membered heteroaryl, and 5-7 membered heteroaryl-$(CHR^8)$-;
Ring B is selected from the group consisting of 5-7 membered heteroaryl, 4-10 membered heterocyclyl, C4-C10 cycloalkyl, and 4-10-membered heterocyclyl substituted with 4-10 membered heterocyclyl (wherein, the heteroaryl, cycloalkyl, or heterocyclyl comprises monocyclic, fused, spiro or bridged ring);
$R^8$ is selected from the group consisting of H, C1-C4 alkyl, and C2-C6 cyanoalkyl;
$R^6$ is selected from the group consisting of C1-C4 alkyl, and C2-C6 cyanoalkyl, - $C(O)CH_2CN$, $-C(O)CH=CH_2$, $-C(O)NHR^7$, $-NHS(O)_2R^7$, $-NHC(R^8)_2C(O)NHR^7$, and - $C(O)NHR^7$;
$R^7$ is selected from the group consisting of -OH, halogen, C1-C6 alkyl, C1-C6 haloalkyl, C1-C6 alkoxy, 5-7 membered heteroaryl, and C2-C6 cyanoalkyl; wherein, the heteroaryl can be substituted with one or more substituents selected from the group consisting of -OH, halogen, C1-C6 alkyl, C1-C6 haloalkyl, and C1-C6 alkoxy.
s is selected from 0 or 1.

In another preferred embodiment,

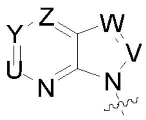

is selected from the following structure:

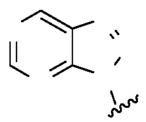 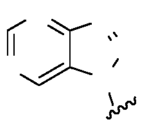 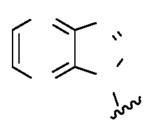 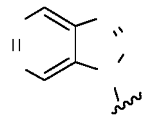,

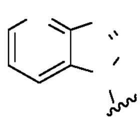 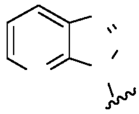 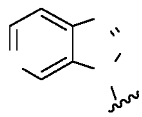 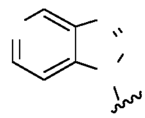, and 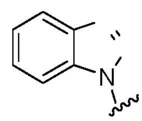.

In another preferred embodiment, $R^4$ or $R^5$ is each independently selected from the group consisting of:

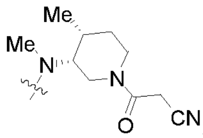, 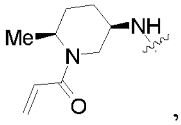, 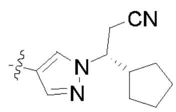,

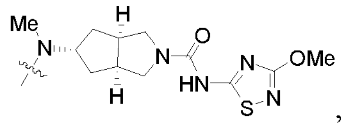, 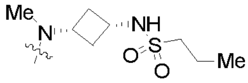, 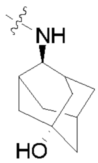, 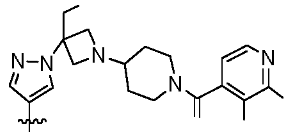, 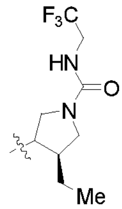,

and

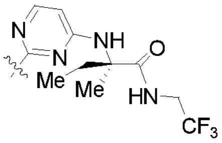.

In another preferred embodiment,

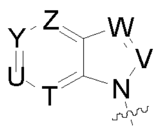

is selected from the following structure:

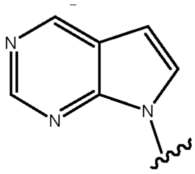, 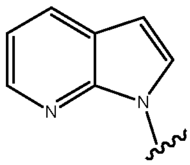, 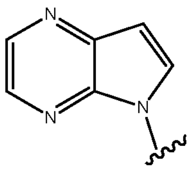, 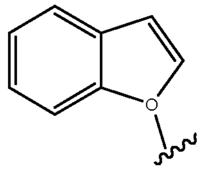

and

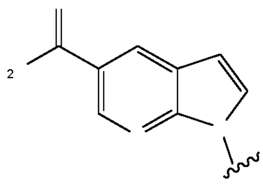.

In another preferred embodiment, the compound of formula (I) has the structure shown in the following formula:

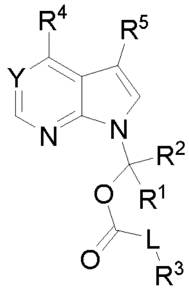

wherein,
Y is N, CH or C-C(O)$NH_2$;

In another preferred embodiment, the compound of formula (I) has the structure shown in formula (IIA):

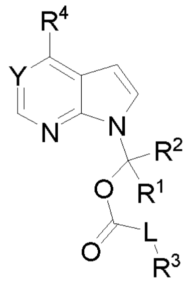

**(IIA)**

wherein,

Y is N or C-C(O)$NH_2$;
$R^4$ is selected from the group consisting of

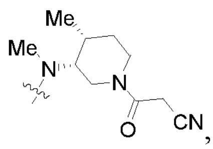, 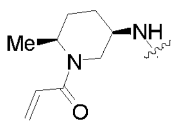,

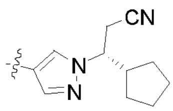, 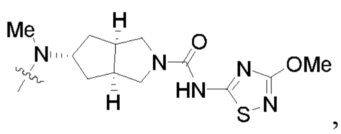, 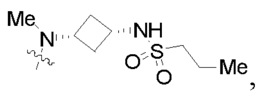, 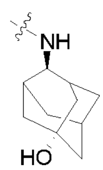,

and

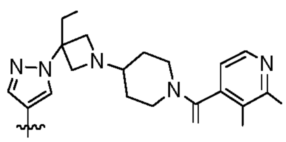.

In another preferred embodiment, the compound of formula (I) has the structure shown in formula (II):

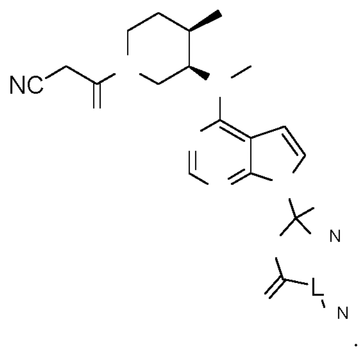

In another preferred embodiment, the compound of formula (I) has the following structure:

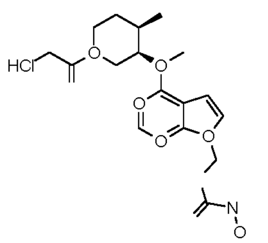

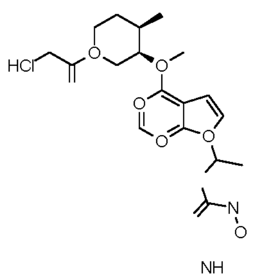

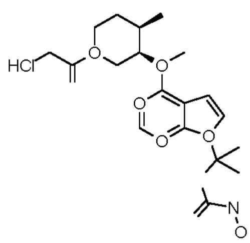

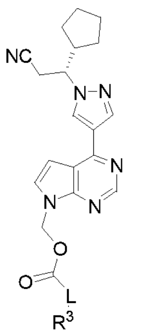

**II-A**

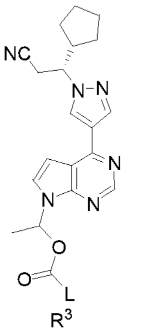

**II-B**

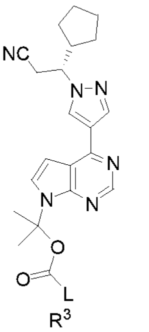

**II-C**

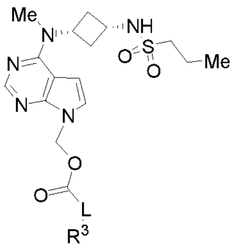

**III-A**

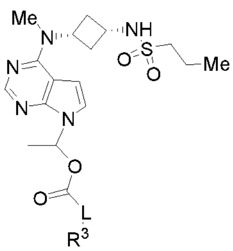

**III-B**

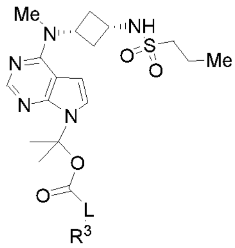

**III-C**

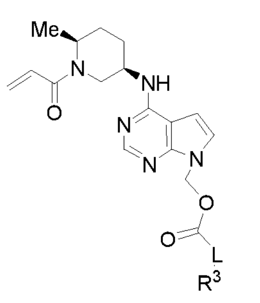

**IV-A**

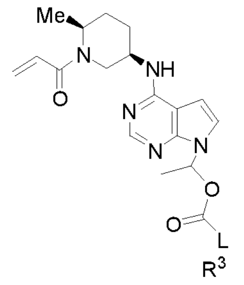

**IV-B**

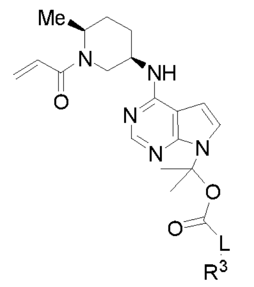

**IV-C**

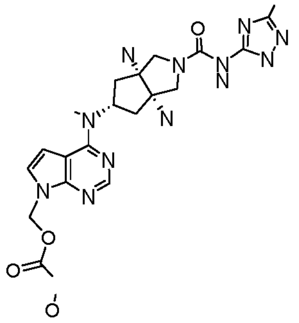

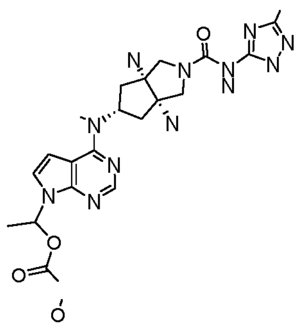

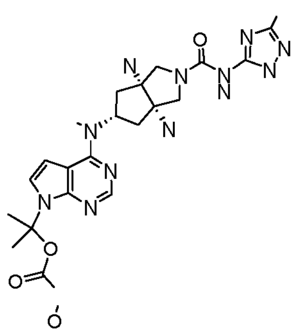

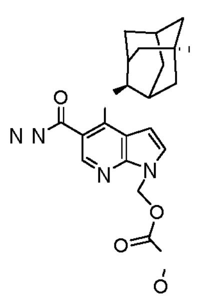

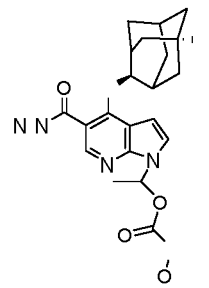

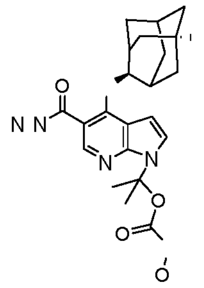

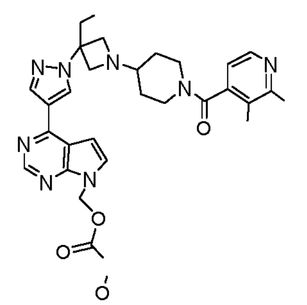

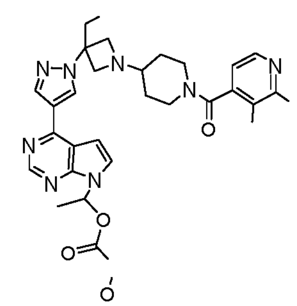

**VII-B**

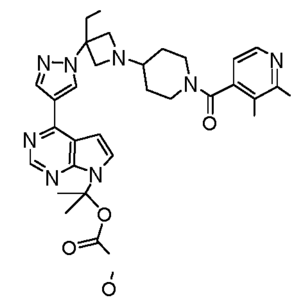

**VII-B**

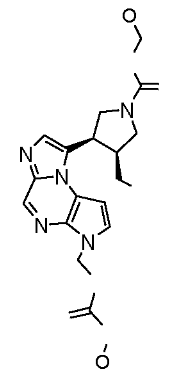 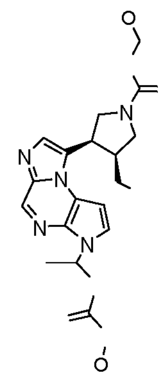 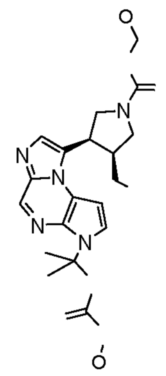

VIII-C

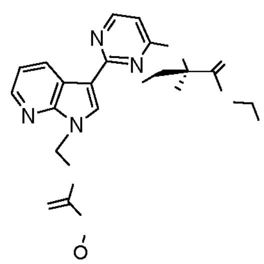 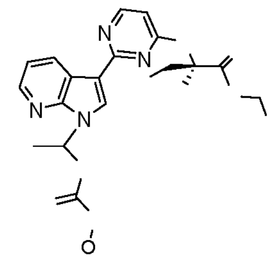 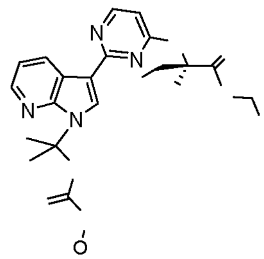

In another preferred embodiment, the compound of formula (I) has the structure selected from the group consisting of:

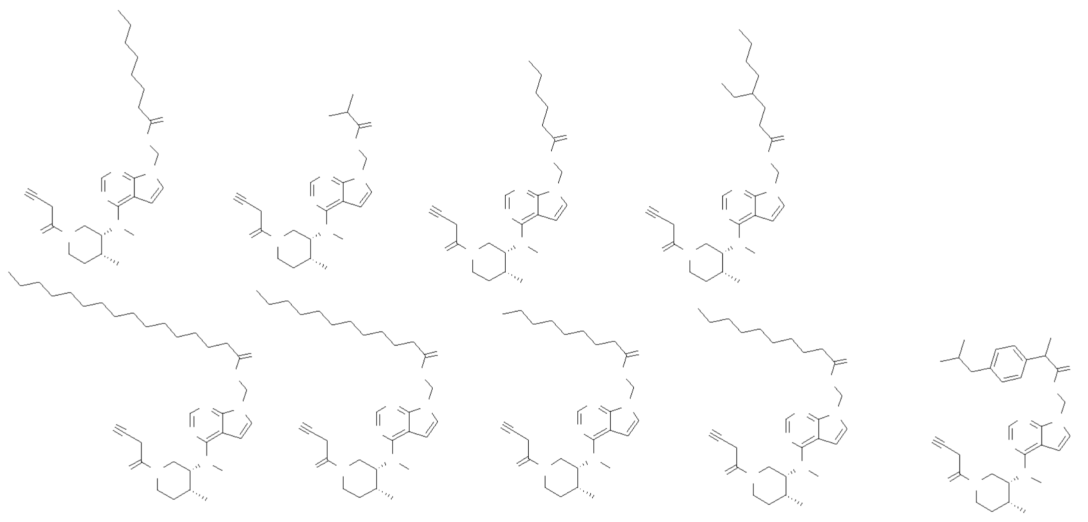

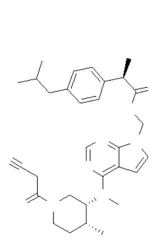 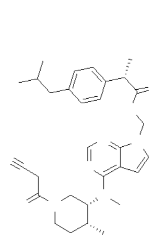 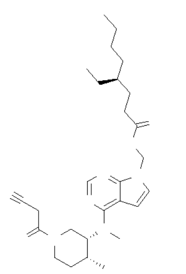 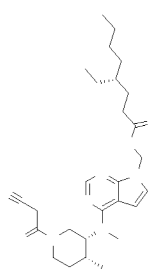

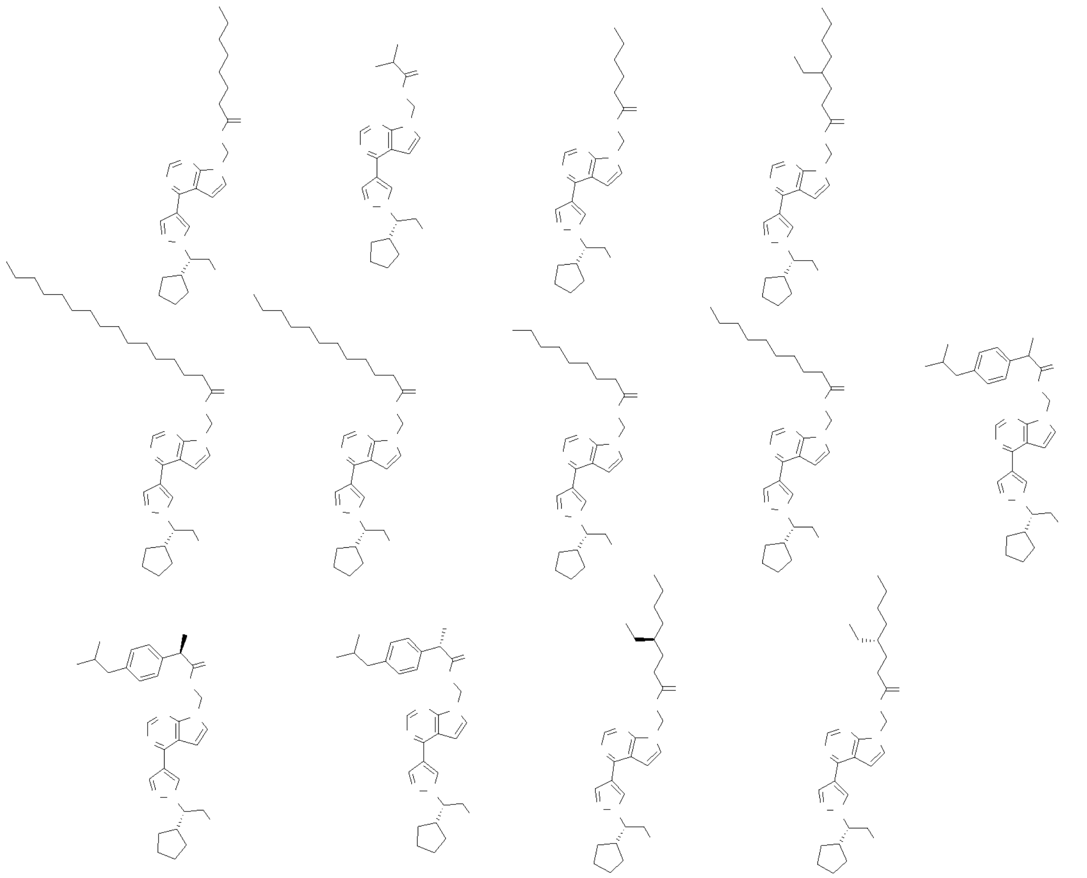

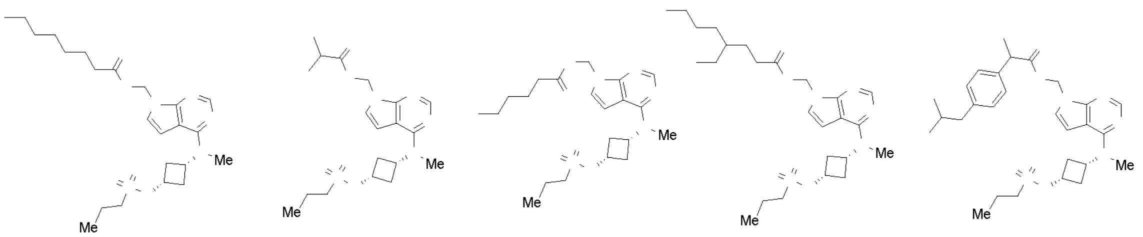

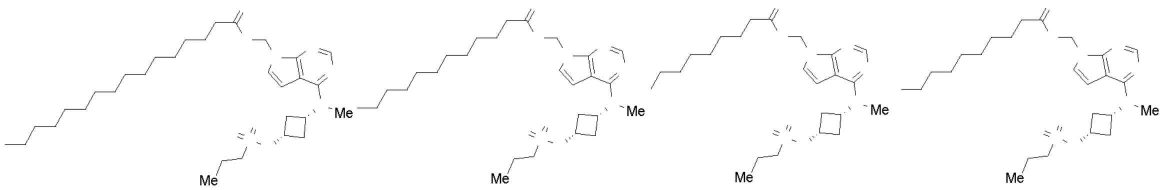

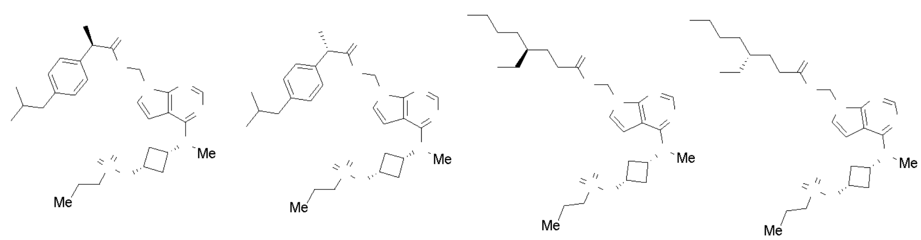

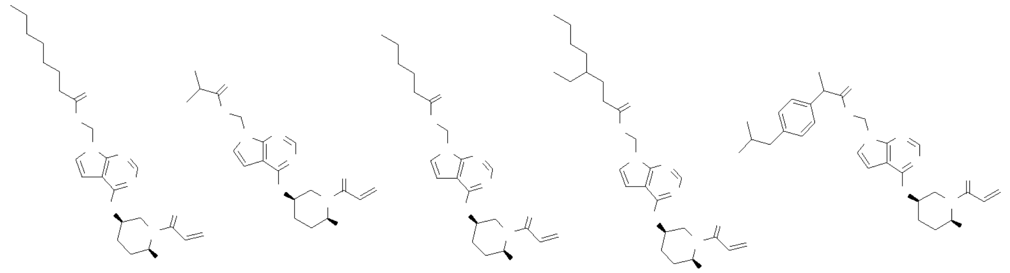

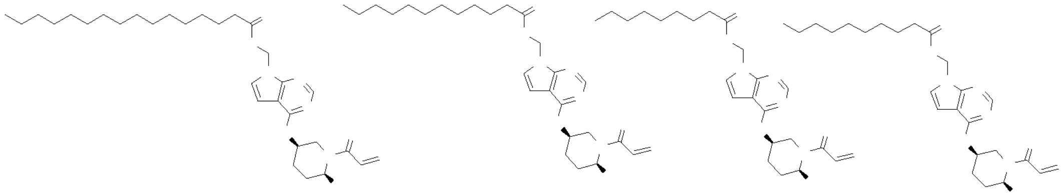

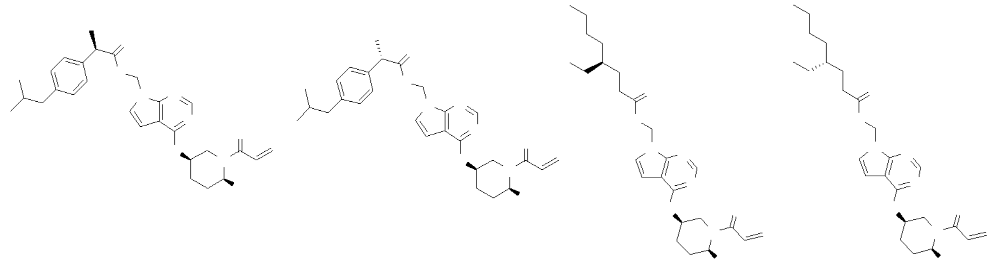

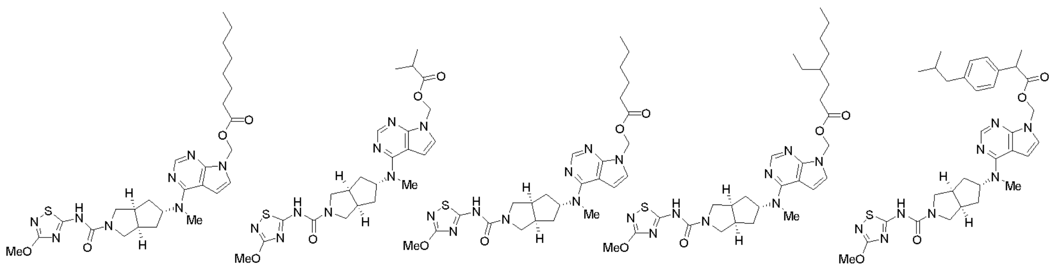

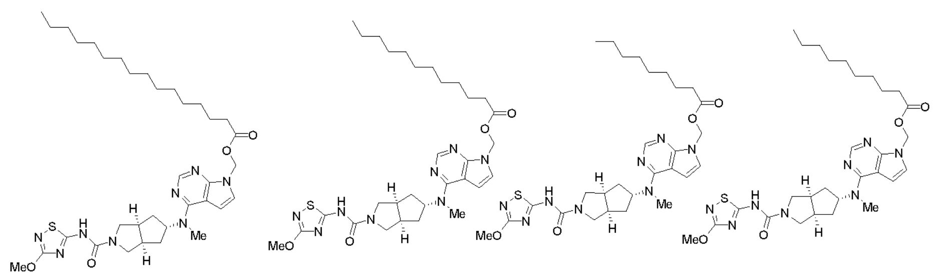

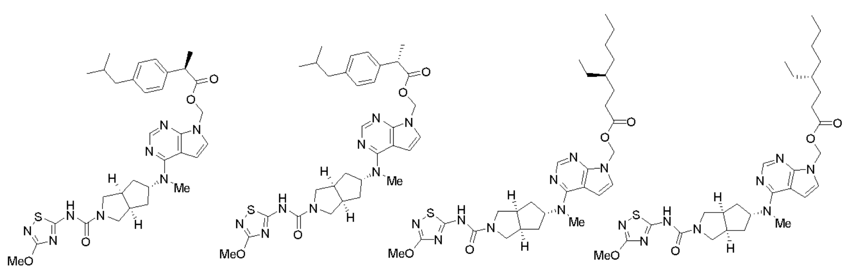

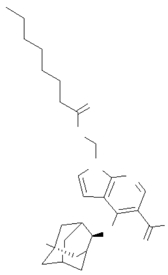
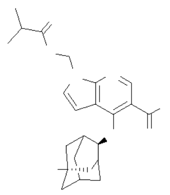
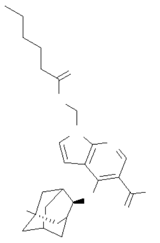
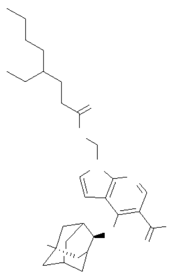
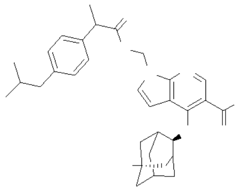

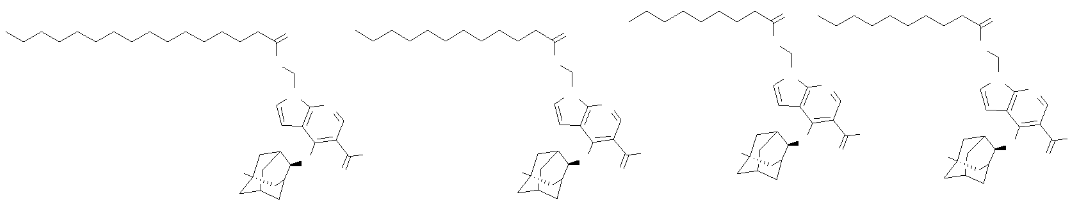

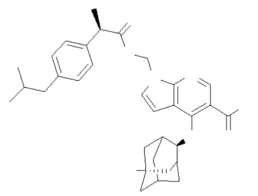
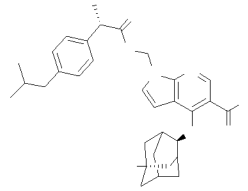
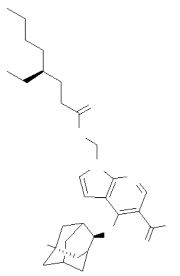
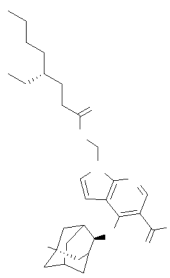

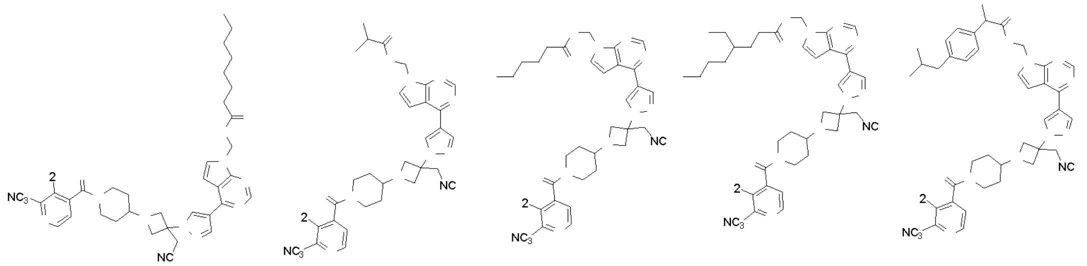

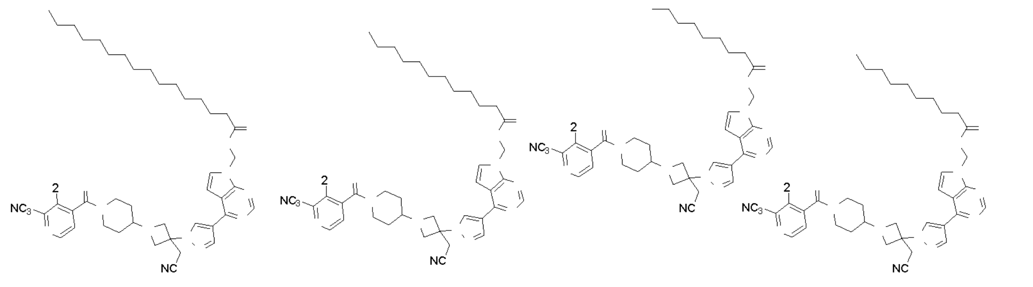

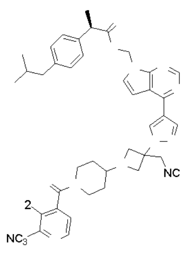
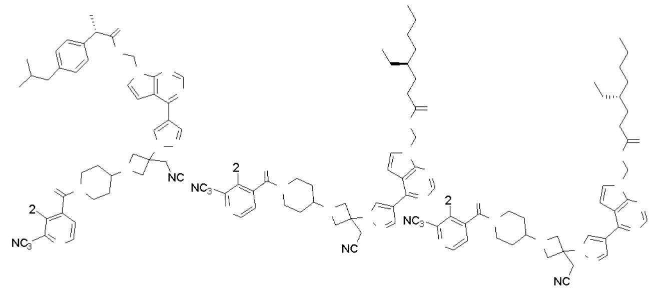

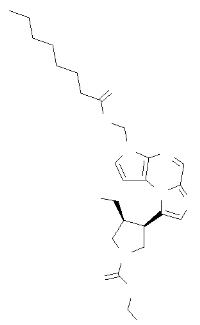
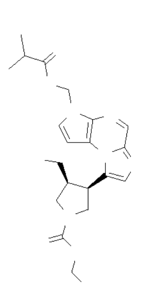
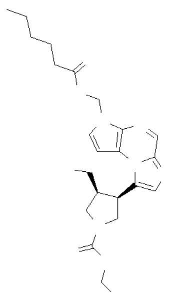
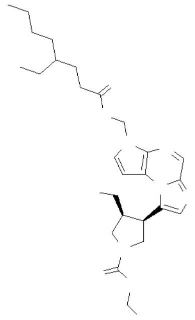
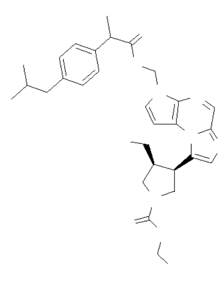

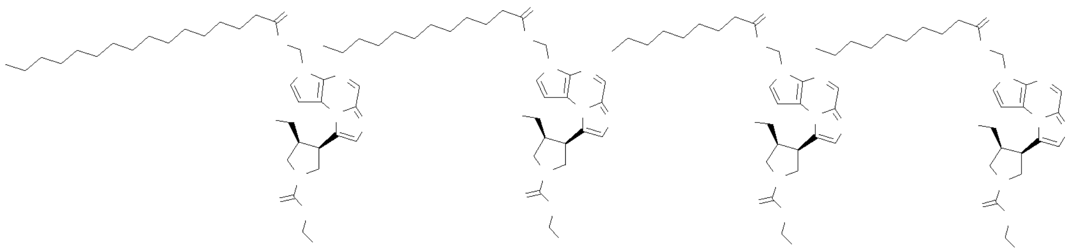

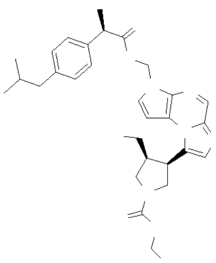
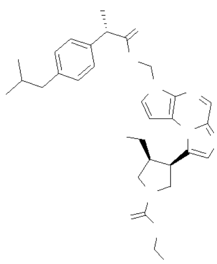
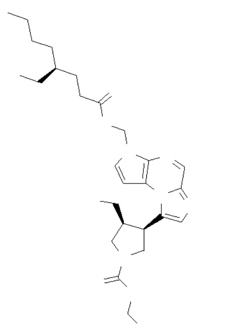
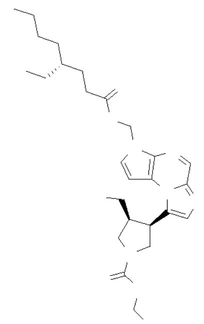

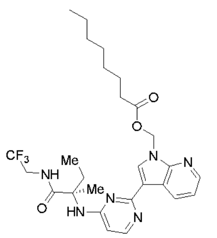
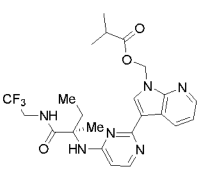
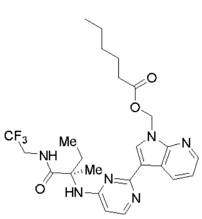
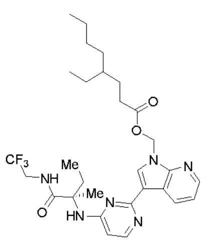
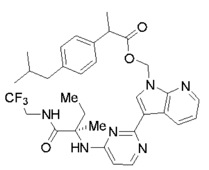

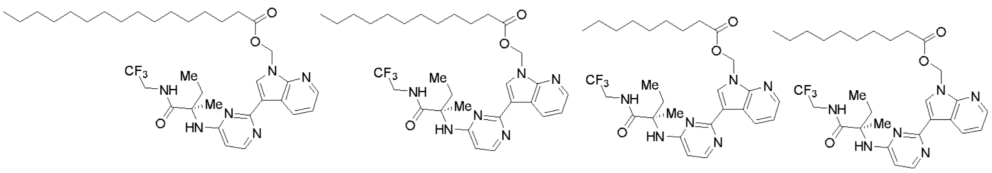

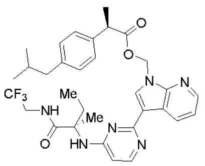

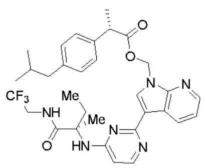

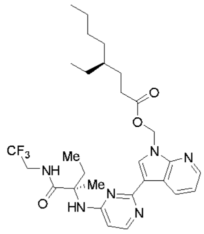

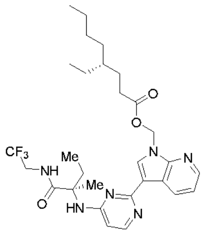

In another preferred embodiment, the pharmaceutically acceptable salt is hydrochloride.

In a second aspect of the present invention, it provides a method for preparing the compound as described in the first aspect of the present invention, wherein the drug molecule G' connect the N, O atoms in the molecule to

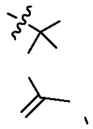

by the following scheme:

(1) scheme 1:

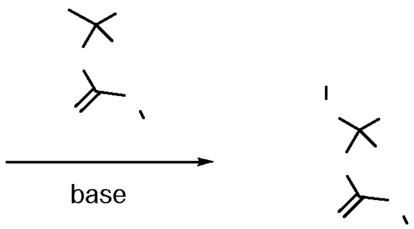


in the above reaction formula, A'-NH is equivalent to G' and A'-N is equivalent to G. The chloroalkyl ester reacts with G' under alkaline conditions to attach the N atom to the prodrug moiety of the present invention.

(2) scheme 2:

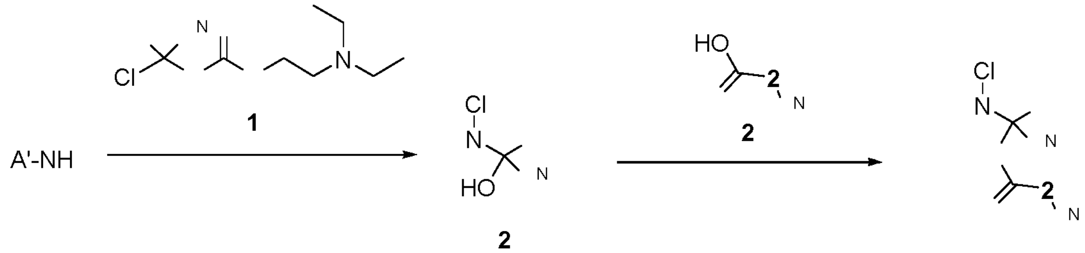


in the above reaction formula, A'-NH is equivalent to G' and A'-N is equivalent to G. By a two-step process, chloroalkyl carbonates **1** firstly reacts with G' to give intermediate **2,** which is then condensed with **3** so as to attach N atom to the prodrug moiety of the invention.

(3) scheme 3:

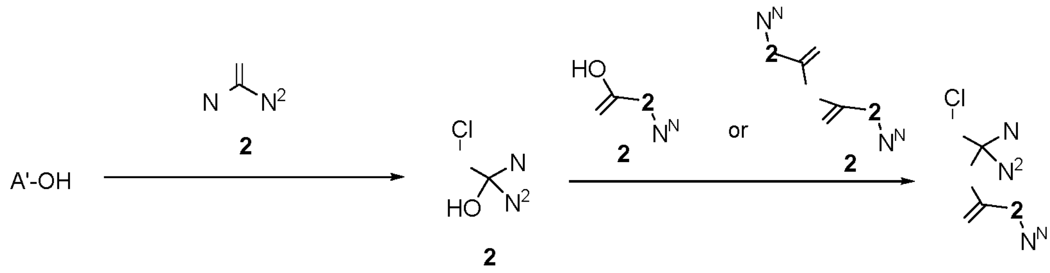


In the above reaction formula, A'-OH is equivalent to G' and A'- O is equivalent to G. Under alkaline or acidic conditions, A'-OH is condensed with carbonyl compound **4** to give intermediate **5,** which is then condensed with acid **3** or anhydride **6** so as to attach the O atom to the prodrug moiety of the invention.

In another preferred embodiment, the method of preparing compound (IIA) comprises the following steps:

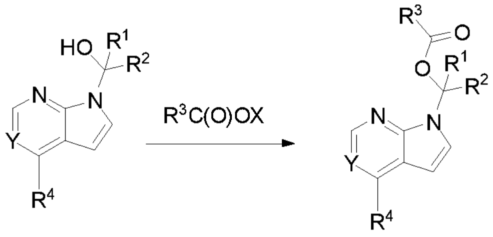


2e

(IIA)

Compound of formula **2e** reacts with $R^3C(O)X$ in an inert solvent to obtain compound of formula (IIA); wherein Y is N or $C\text{-}C(O)NH_2$, X is OH or activating group (preferably halogen or $OC(O)R^3$), and each of the remaining groups is as defined as above.

In another preferred embodiment, it comprises the following steps:

(1) scheme 1:

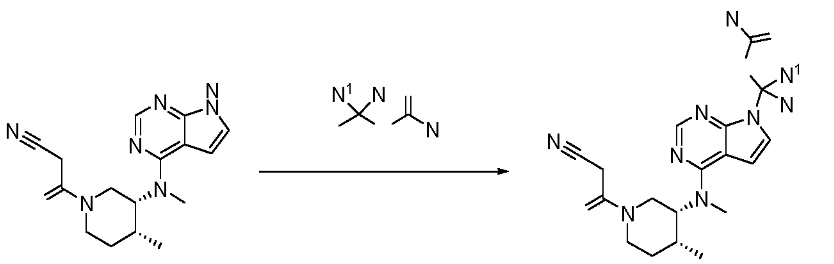

The free base of tofacitinib reacts with $R^3C(O)CR^1R^2Cl$ in an inert solvent to obtain compound of formula (II); or

(2) scheme 2:

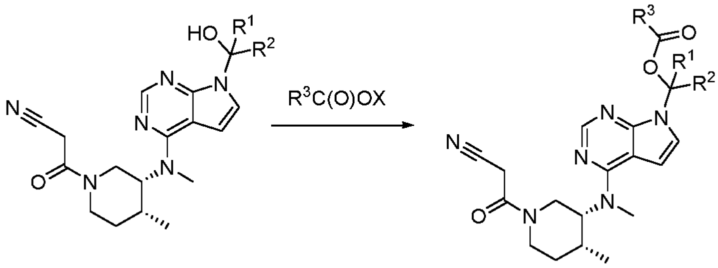


2e'

(II)

Compound of formula **2e'** reacts with $R^3C(O)X$ in an inert solvent to obtain compound of formula (II); wherein X is OH or an activating group (preferably halogen or $OC(O)R^3$), and each of the remaining groups is defined as described in the first aspect of the present invention.

In another preferred embodiment, the method further comprises the following steps:

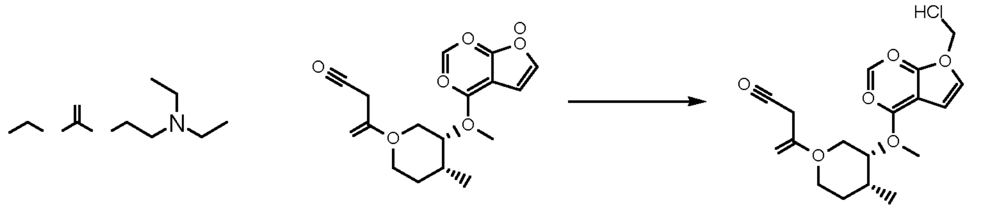

Compound **1c** reacts with compound **1d** to obtain compound **1e**.

In another preferred embodiment, the method further comprises the following steps:

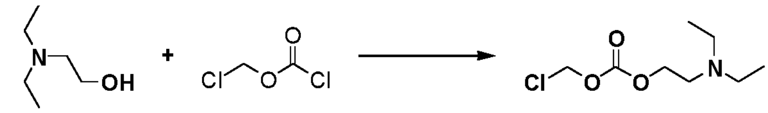

**1a** **1b** **1c**

Compound **1a** reacts with chloromethyl chloroformate to obtain compound **1c.**

In a third aspect of the present invention, it provides an intermediate as shown in formula **2e**:

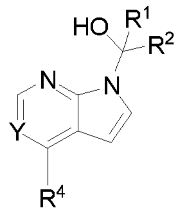

2e ;

wherein, Y is N or C-C(O)$NH_2$; each of the remaining groups are as defined as above.

In another preferred embodiment, the compound of formula **2e** has the structure shown in formula **1e**:

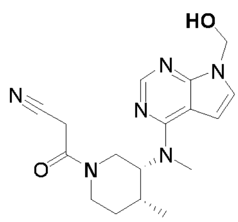

**1e**

In a fourth aspect of the present invention, it provides a pharmaceutical composition, which comprising a pharmaceutically acceptable carrier and a compound of the first aspect of the present invention, or pharmaceutically acceptable salts, or hydrates or solvates thereof.

In another preferred embodiment, the pharmaceutical composition is a topical formulation.

In another preferred embodiment, the pharmaceutical composition also has skin penetration enhancers (e.g., surfactants, dimethyl sulfoxide, decyl methyl sulfoxide, azone accelerators, alcohol accelerators, volatile oils, amino acids, phospholipids, oleic acid).

In another preferred embodiment, after transdermal administration of the pharmaceutical composition, the compound of formula I is metabolized in vivo to form tofacitinib.

In another preferred embodiment, the pharmaceutical composition is for use in the treatment or prevention of diseases associated with the activity or expression of JAK kinase; preferably, the disease is selected from the group consisting of: cancer, myeloproliferative disease, inflammation, immune diseases, organ transplantation, viral disease, cardiovascular disease or metabolic disease, autoimmune disease in humans or animals, rheumatoid arthritis, dermatological conditions, multiple sclerosis, rheumatoid arthritis, psoriatic arthritis, inflammatory bowel disease, myasthenia gravis, and psoriasis.

In a fifth aspect of the present invention, it provides a use of the compound of the first aspect of the present invention, or pharmaceutically acceptable salts or hydrates thereof, in the preparation of a pharmaceutical composition for the treatment or prevention of a disease associated with the activity or expression of JAK kinase.

In another preferred embodiment, the disease is selected from the group consisting of: cancer, myeloproliferative disease, inflammation, immune diseases, organ transplantation, viral disease, cardiovascular disease or metabolic disease, autoimmune disease in humans or animals, rheumatoid arthritis, dermatological conditions, multiple sclerosis, rheumatoid arthritis, psoriatic arthritis, inflammatory bowel disease, myasthenia gravis, and psoriasis.

In a sixth aspect of the present invention, it provides a topical formulation, comprising:

The compound of the first aspect of the present invention;
Optional skin penetration enhancer, preferably, the skin penetration enhancer is selected from the group consisting of surfactants, dimethylsulfoxide and its analogs, azones, pyrrolidone derivatives, alcohols, ethers, fatty acids and fatty acid esters, and combinations thereof;
Optional supporting layer.

In another preferred embodiment, the drug is present in a single phase or in multiple phases, in solution or in suspension.

In another preferred embodiment, the formulation is administered in the form of solutions, suspensions, gels, emulsions, creams, foams and the like.

In another preferred embodiment, the supporting layer is a membrane polymer or a backbone polymer.

In another preferred embodiment, the supporting layer is selected from the group consisting of: pressure-sensitive adhesive material, backing material, anti-adhesive material, and drug storage material.

In another preferred embodiment, the topical formulation further comprises a peeling layer.

In another preferred embodiment, the formulation is a transdermal drug delivery formulation.

In another preferred embodiment, the formulation is a sustained-release release formulation.

In another aspect of the present invention, it provides a method for improving the membrane permeability of drug molecule G', which comprises steps of: the drug molecule G' is modified so as to introduce fragment

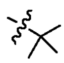

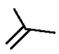,

into the molecule to form

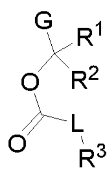

(I)

with CLogP > 4, while the CLogP of the modified prodrug molecule (I) is increased by at least 1 over the CLogP of the drug molecule G'.

In another preferred embodiment, the CLogP of the modified prodrug molecule (I) is increased by at least 2 over the CLogP of the drug molecule G'.

In another preferred embodiment, the CLogP of the modified prodrug molecule (I) is increased by at least 3 over the CLogP of the drug molecule G'.

In another preferred embodiment, a method of modifying a drug molecule G' with

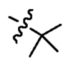

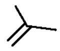,

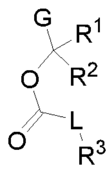

fragment to form (I) , comprising of coupling the drug molecule G' with

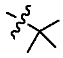

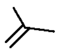,

.

In another preferred embodiment, the Skin-Pampa Pe value of the prodrug molecule

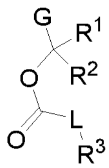

(I)

is increased by 2-100 folds over the parent drug molecule G'.

In another preferred embodiment, the Skin-Pampa Pe value of the prodrug molecule

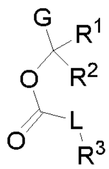

(I)

is increased by 4-20 folds over the parent drug molecule G'.

It should be understood that, in the present invention, each of the technical features specifically described above and below (such as those in the examples) can be combined with each other, thereby constituting new or preferred technical solutions which are not necessarily specified one by one herein.

## Detailed Description of the Invention

After long and intensive research, the inventors developed a compound represented by formula (I). The compound comprises a hydrophilic drug molecule end and a hydrophobic end, so that they have good transdermal properties and can be metabolized in the body to form a prototypical drug molecule after topical administration to complete the drug delivery process. Based on the above findings, the inventors have completed the present invention.

## Definition

As used herein, the term "alkyl" includes a linear or branched alkyl. For example, C1-C6 alkyl represents a linear or branched chain alkyl having 1 to 6 carbon atoms, such as methyl, ethyl, propyl, isopropyl, butyl, isobutyl, tert-butyl, and the like, "C1-C20 alkyl" has a similar meaning. The term "alkylene" refers to an alkyl group that has lost one hydrogen atom, e.g., C1-C6 alkylene refers to a linear or branched alkylene with 1-6 carbon atoms. The term "heteroalkyl" refers to one or more carbon atoms on the carbon chain being replaced by heteroatoms selected from the group consisting of N, O or S(O)p; the term "heteroalkylene" has a similar meaning.

As used herein,the term "C3-C8 cycloalkyl" refers to an cycloalkyl group having 3 -8 carbon atoms. It may be monocyclic, such as cyclopropyl, cyclobutyl, cyclopentyl, cyclohexyl, or the like. It can also be bicyclic, such as a bridged ring, a fused ring or a spiro ring. "C3-C20 cycloalkyl" has a similar definition.

As used herein, the term "C6-C14 aryl" refers to an aryl having 6 -14 carbon atoms, e.g., phenyl or naphthyl and the like.

As used herein, the term "5-10 membered heteroaryl having 1-3 heteroatoms selected from the group consisting of N, S and O" refers to a cyclic aromatic group having 5-10 atoms and wherein 1-3 atoms are heteroatoms selected from N, S and O. It can be monocyclic, and it can also be fused. Specific examples may be pyridyl, pyridazinyl, pyrimidinyl, pyrazinyl, triazinyl, pyrrolyl, pyrazolyl, imidazolyl, (1,2,3)-triazolyl, and (1,2,4)-triazolyl, tetrazolyl, furyl, thienyl, isoxazolyl, thiazolyl, oxazolyl, etc..

As used herein, the term "3-20 membered heterocyclyl" refers to saturated or partially saturated cyclic group having 3-20 cyclic atoms and wherein 1-3 atoms are heteroatoms selected from N, S and O, preferably 3-10 membered heterocyclyl, or 4-7 membered heterocyclyl or 9-15 membered heterocyclyl. It can be monocyclic, and it can also be bicyclic, such as a bridged ring or a spiro ring. Specific examples may be oxetanyl, azetidinyl, tetrahydro-2H-pyranyl, piperidinyl, tetrahydrofurfuryl, morpholinyl and pyrrolyl, etc.."3-8 membered heterocyclyl" has a similar definition.

Unless specifically stated that the groups described in the present invention are "substituted or unsubstituted", the groups of the present invention can be substituted by substituents selected from the group consisting of halogen, cyano, nitro, hydroxyl, amino, C1-C6 alkyl-amino, C1-C6 alkyl, C2-C6 alkenyl, C2-C6 alkynyl, C1-C6 alkoxy, halogenated C1-C6 alkyl, halogenated C2-C6 alkenyl, halogenated C2-C 6 alkynyl, halogenated C1-C6 alkoxy, allyl, benzyl, C6-C12 aryl, C1-C6 alkoxy-C1-C6 alkyl, C1-C6alkoxy-carbonyl, phenoxycarbonyl, C2-C6 alkynyl-carbonyl, C2 -C6 alkenyl-carbonyl, C3-C6 cycloalkyl-carbonyl, C1-C6 alkyl-sulfonyl, etc.

As used herein, the term "halogen" or "halogen atom" refers to F, Cl, Br and I. More preferably, the halogen or halogen atom is selected from F, Cl and Br. "Halogenated" means being substituted by atom(s) selected from F, Cl, Br, and I.

Unless otherwise specified, the structural formula described in the present invention is intended to include all isomeric forms (e. g., enantiomeric, diastereomeric, and geometric (or conformational) isomers): for example, R and S configurations of asymmetric centers, (Z) and (E) isomers of double bonds, etc. Thus, a single stereochemical isomer of the compound of the invention or a mixture of its enantiomers, diastereomers or geometric isomers (or conformational isomers) is within the scope of the invention.

As used herein, the term "hydrate" refers to a complex formed by the coordination of a compound of the present invention with water.

The compounds of the present application can be prepared by a variety of synthetic methods well known to those skilled in the art, including the specific embodiments listed below, the embodiments formed by the combination of specific embodiments and other chemical synthesis methods, and equivalents well-known for those skilled in the art, preferred embodiments include, but are not limited to, the Examples of the present application.

The solvent used in the present application can be commercially available. Abbreviations used in the present application such as: aq for aqueous solution; HATU for O-(7-azabenzotriazol-1-yl)-N,N,N',N'-tetramethyluronium hexafluorophosphate; EDCI for N-(3-dimethylaminopropyl)-N'-ethylcarbodiimide hydrochloride; m-CPBA stands for 3-chloroperoxybenzoic acid; eq stands for equivalent, equivocal; CDI stands for carbonyldiimidazole; DCM stands for dichloromethane; PE stands for petroleum ether; DIAD stands for diisopropyl azodicarboxylate; DMF stands for N,N-dimethylformamide; DMSO stands for dimethylsulfoxide; EtOAc stands for ethyl acetate; EtOH stands for ethanol; MeOH stands for methanol; Cbz stands for benzyloxycarbonyl, an amino protecting group; Boc stands for tert-butoxycarbonyl, an amino protecting group; HOAc stands for acetic acid; NaCNBH3 stands for sodium cyanoborohydride; r.t. stands for room temperature; THF stands for tetrahydrofuran; TFA stands for trifluoroacetic acid; DIPEA stands for diisopropylethylamine; Boc2O stands for di-tert-butyldicarbonate; and LDA stands for lithium diisopropylammonium.

Compounds were named manually or by ChemDraw® software, and commercially available compounds were named by supplier catalog.

## Prodrug compounds suitable for topical administration

In the present invention, it provides a prodrug molecule of a pharmaceutical compound, and pharmaceutically acceptable salts, hydrates or solvates thereof, characterized in that the prodrug molecule is metabolized in vivo after administration to form a drug molecule G'; and the drug molecule G' has a hydrophobicity coefficient CLogP of less than 3. And the prodrug molecule has a structure shown in formula (I):

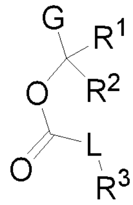

(I)

wherein, G is a partial structural fragment formed by the loss of functional groups or H atoms of the drug molecule G', and connectes to

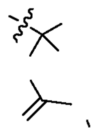

via any N, O or S atom within the molecule.

In the present invention, a lipophilic group represented as

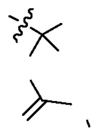

can effectively increase the transdermal efficiency of the compound, and thus prepare new compounds that can be administered topically and then metabolized into prototypical compound molecules. The drug molecule G' suitable for prodrug molecule may be of any structure, and in preferred embodiments, the drug molecule has a hydrophobicity coefficient CLogP of less than 3.

Since such drug molecules have a lipophilic end and a hydrophilic drug molecule end, they have very good membrane permeability, especially improving the transdermal property of drug molecules during dermal administration. Preferably, the drug molecule is used in dermatological disorders, e.g., a JAK inhibitor, a MEK inhibitor, a BTK inhibitor, and the like.

$R^1$ and $R^2$ are each independently selected from the group consisting of H, D, substituted or unsubstituted C1-C6 alkyl, substituted or unsubstituted C1-C6 heteroalkyl, substituted or unsubstituted C3-C8 cycloalkyl, and substituted or unsubstituted 3-8 membered heterocyclyl, or $R^1$ and $R^2$, together with the carbon atoms connected thereto, form C3-C8 carbocycle or heterocycle;

L is selected from the group consisting of chemical bond, substituted or unsubstituted C1-C6 alkylene, and substituted or unsubstituted C1-C6 heteroalkylene;

$R^3$ is selected from the group consisting of substituted or unsubstituted C1-C20 alkyl, substituted or unsubstituted C3-C20 cycloalkyl, substituted or unsubstituted C1-C20 heteroalkyl, substituted or unsubstituted 3-20-membered heterocyclyl, and substituted or unsubstituted C6-C14 aryl, or $R^3$ is connected to $R^1$ or $R^2$ so as to form a substituted or unsubstituted 5-20 membered lactone ring or heterolactone ring; wherein the heterolactone ring refers to the cyclic backbone of the lactone ring comprising 1-3 heteroatoms selected from the group consisting of N, O and $S(O)_p$;

p is selected from 0, 1 or 2;

wherein, the heteroalkyl refers to one or more carbon atoms on the carbon chain being substituted by heteroatom selected from the group consisting of N, O and $S(O)_p$;

the heterocyclyl comprises 1-3 heteroatoms selected from the group consisting of: N, O and $S(O)_p$;

unless otherwise specified, the "substituted" means being substituted by one or more (e.g. 2, 3, 4, etc.) substituents selected from the group consisting of halogen, C1-C6 alkyl, halogenated C1- C6 alkyl, C1-C6 alkoxy, halogenated C1-C6 alkoxy, C3-C8 cycloalkyl, halogenated C3-C8 cycloalkyl, C3-C8 heterocyclyl, oxo, -CN, hydroxyl, amino, carboxyl, amide, sulfonamide, sulfonyl, and a group which is unsubstituted or substituted by one or more substituents, wherein the group is selected from C6-C10 aryl, halogenated C6-C10 aryl, 5-10-membered heteroaryl having 1-3 heteroatoms selected from N, S and O, halogenated 5-10-membered heterocyclic group having 1-3 heteroatoms

selected from N, S and O; and the substituents is selected from the group consisting of halogen, C1-C6 alkyl, C1-C6 alkoxy, and =O.

**Pharmaceutical composition and mode of administration**

Since the compound of the present invention are capable of being metabolized in vivo to form a therapeutically active ingredient after topical administration, the compound of the present invention and its various crystal forms, pharmaceutically acceptable inorganic or organic salts, hydrates or solvates, and the pharmaceutical composition containing the compound of the present invention as the main active ingredient can be used to prevent and/or treat (stabilize, alleviate or cure) a variety of autoimmune and inflammation-related diseases, including cancer, myeloproliferative disease, inflammation, immune disease, organ transplantation, viral disease, cardiovascular disease, or metabolic disease, etc..

The pharmaceutical composition of the present invention comprises a safe and effective amount of the compound of the present invention and a pharmaceutically acceptable excipient or carrier. Wherein, "safe and effective amount" refers to the amount of compound which is sufficient to significantly improve the condition, and not to generate severe side effects. Generally, the pharmaceutical composition contains 1-2000 mg compound of the invention per dose, preferably, 1-200mg compound of the invention per dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatible" herein refers to the ability of each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween®), wetting agent (such as lauryl sodium sulfate), colorant, flavoring, stabilizer, antioxidant, preservative, pyrogen-free water, etc.

The prodrug compound of the present invention may be conveniently formulated into a pharmaceutical composition comprising one or more of the compounds of the present invention and pharmaceutical carriers. See Remington: The Science and Practice of Pharmacy 19th edition (Easton, PA, Mack Publishing Co., 1995), which discloses exemplary carriers and common methods of preparing pharmacy compositions, the methods being used as described or modified to make pharmaceutical compositions containing the compound of the present invention. As mentioned above, the compounds of the present invention can also be administered in the form of pharmaceutical salts and the like.

The compound of the present invention may be administered in the form of pharmaceutical agents containing commonly used non-toxic pharmaceutical carriers, accessories and excipients, and administered orally, parenterally, topically, rectally, nasally, orally, vaginally, or via implanted reservoirs. Preferably, the compound of the present invention can be administered through the skin or mucosal tissues by commonly used topical delivery systems, wherein the agent is contained in a multilayered structure, the multilayered structure being secured to the skin and functioning as a drug delivery device. In such a structure, the pharmaceutical composition is contained in a layer below the upper backing layer, i.e., the "reservoir" layer. The multilayer structure may contain a single reservoir or a plurality of reservoirs. In one example, the reservoir comprises a polymeric matrix of a pharmaceutical press-fit adhesive substance, the adhesive substance acts to secure the system to the skin during administration. Suitable skin press-fit adhesive substances include, but are not limited to, polyethylene, polysiloxane, polyisobutylene, polyacrylates, polyurethanes, and the like. Alternatively, the drug-containing reservoir and the skin press-fit adhesive may be present as separate and distinct layers, in which case, the adhesive is underneath the reservoir, and the reservoir may be a polymer matrix as described above, a liquid or hydrogel reservoir, or other forms.

In these multilayers, the backing layer, which is the upper surface of the device, acts as the main structural element of the multilayer structure, providing much of the flexibility of the device. The substance selected for the backing layer should be substantially impermeable to the active substance and to any other substances present; the backing layer is preferably made of a soft elastic sheet or membrane. Examples of polymers suitable for use as backing layers include polyethylene, polypropylene, polyester, and the like.

During storage and prior to use, the multilayer structure includes a peel-off layer. The layer is removed from the device before use, exposing either its underside or the drug reservoir or the separate press-fit adhesive layer so that the system can be secured to the skin. The peel-off layer should be made of a material that will not be permeable to the drug/excipient.

Topical drug delivery devices can be fabricated by common techniques known in the art, for example, by pouring a fluid mixture of adhesive, drug, and excipient over a backing layer and then laminating the peel-off layer. Similarly, the adhesive mixture can be poured over the peel-off layer and then laminated to the backing layer. Alternatively, drug reservoirs can be made without drug or excipient and then filled with the drug/excipient mixture by immersion.

Multi-layered topical delivery system may also contain skin penetration enhancers. That is, since the skin's inherent permeability to certain drugs may be too small to allow therapeutic amount of drugs to pass through a certain area of unbroken skin, there is a need to administer skin penetration enhancers along with these drugs. Suitable enhancers are well known in the art and include, for example, dimethylsulfoxide (DMSO), dimethylformamide (DMF), N,N-dimethylacetamide (DMA), decylmethylsulfoxide (C10MSO), C2-C6 chain alkanediols, and 1-substituted azepan-2-one, alcohols and the like.

The compounds of the present invention may be administered alone or in combination with other pharmaceutically acceptable therapeutic agents.

When administered in combination, the pharmaceutical composition further comprises one or more (2, 3, 4, or more) other pharmaceutically acceptable therapeutic agents. One or more (2, 3, 4, or more) other pharmaceutically acceptable therapeutic agent may be used simultaneously, separately, or sequentially with the compounds of the present invention for the prevention and/or treatment of cytokine and/or interferon-mediated diseases.

When the pharmaceutical compositions are used, a safe and effective amount of compound of the present invention is applied to a mammal (such as human) in need thereof, wherein the dose of administration is a pharmaceutically effective dose. For a person weighed 60 kg, the daily dose is usually 1- 2000 mg, preferably 1 -500 mg. Of course, the particular dose should also depend on various factors, such as the route of administration, patient healthy status, which are well within the skills of an experienced physician.

The present invention was further described hereafter in combination with specific embodiments. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are calculated by weight.

**Examples**

**Example 1**

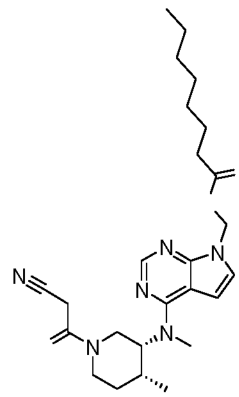

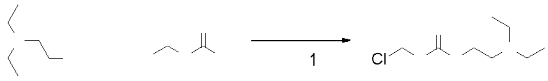


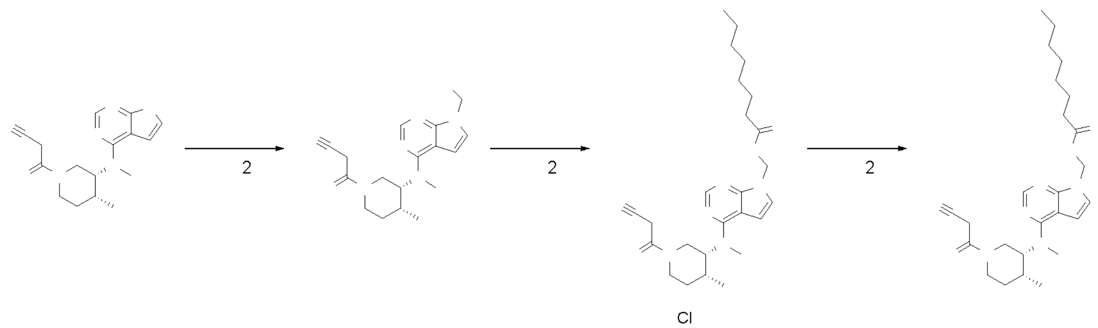

**Step 1**

1a (4.00 g, 34.13 mmol) and pyridine (5.40 g, 68.26 mmol) were dissolved in dichloromethane (50 mL), chloromethyl chloroformate (4.90 g, 38.00 mmol) was added dropwise to the above solution at 0°C under $N_2$ atmosphere, after the addition, the mixture was slowly warmed up to room temperature and continued to stir for 4 h. After the reaction was over, the reaction solution was concentrated under reduced pressure to to give crude product **1c** (12.00 g).

**Step 2**

Crude product **1c** (12.00 g, 34.13 mmol), **1d** (5.00 g, 16.01 mmol) and potassium carbonate (6.60 g, 47.75 mmol) were dissolved in *N*,*N*-dimethylformamide (50 mL) under $N_2$ atmosphere, and the resultant solution was stirred at 60°C overnight. The reaction was cooled down to room temperature and purified directly by reversed-phase column chromatography (acetonitrile: water = 0-100%) to give **1e** (3.00 g), yield: 55%.
MS-ESI calcd.for $[M+H]^+$ 343, found 343.

**Step 3**

**1e** (1.10 g, 3.21 mmol) and triethylamine (650 mg, 6.42 mmol) were dissolved in dichloromethane (30 mL), octanoyl chloride (750 mg, 4.61 mmol) was added to the above solution at 0°C under $N_2$ atmosphere. The reaction was stirred for 0.5 h after addition. At the end of the reaction, the reaction solution was diluted with dichloromethane (30 mL) and washed with water (50 mL × 1). The organic phase was dried over anhydrous sodium sulfate, filtered and concentrated under reduced pressure to obtain the residue, which was purified by preparative HPLC (acetonitrile: water = 0-100%) to give **1f** (600 mg), yield: 40%.
MS-ESI calcd.for $[M+H]^+$469, found 469.

**Step 4**

**1f** (600 mg, 1.28 mmol) was dissolved in ethyl acetate (30 mL), hydrogen chloride ethyl acetate solution (5 M, 0.30 mL, 1.50 mmol) was slowly added to the above solution under $N_2$ atmosphere. After the dropwise addition, the mixture was continued to stir for 1 h, the solid was obtained when concentrating under reduced pressure by maintaining the temperature below 20°C and lyophilised in a solution of acetonitrile (10 mL) and water (100 mL) to give 1 (620 mg), yield: 96%.
$^1$H NMR(400 MHz, DMSO-$d_6$) δ 8.29-8.26 (m, 1H), 7.40-7.40 (m, 1H), 6.77 (br s, 1H), 6.15-6.14 (m, 2H), 4.77 (br s, 1H), 4.16-3.94 (m, 3H), 3.88-3.65 (m, 2H), 3.42-3.41 (m, 1H), 3.29 (s, 3H), 2.41-2.33 (m, 1H), 2.29 (t, *J* = 7.6 Hz, 2H), 1.89-1.74 (m, 1H), 1.62-1.56 (m, 1H), 1.51-1.44 (m, 3H), 1.24-1.16 (m, 8H), 1.04-1.01 (m, 3H), 0.83-0.80 (m, 3H).
MS-ESI calcd.for $[M+H]^+$469, found 469.

**Example 2**

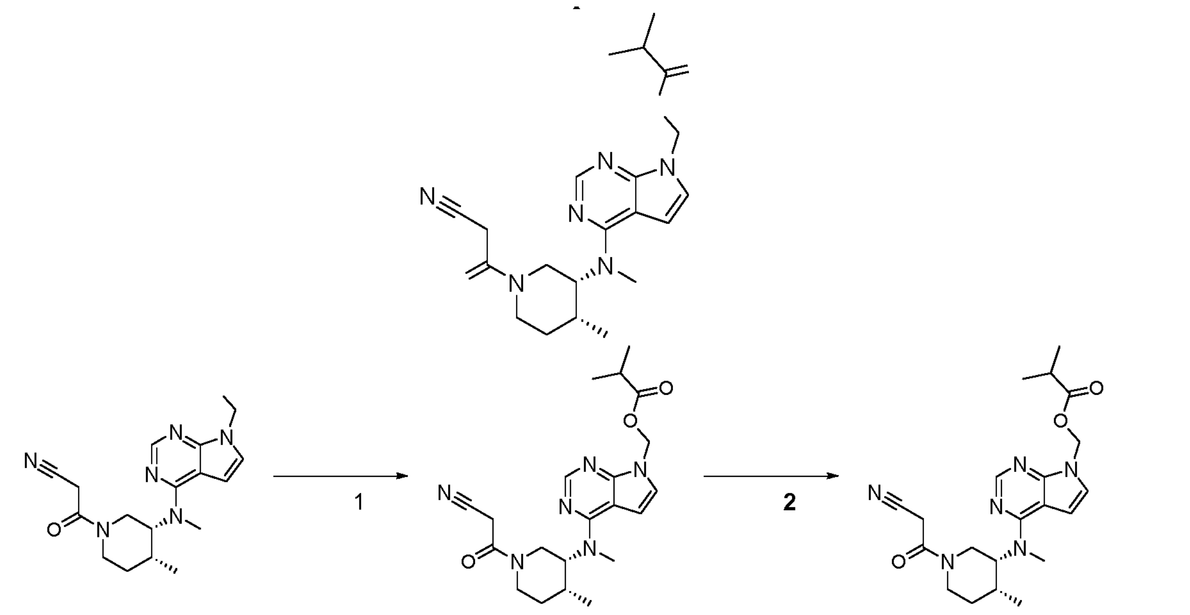


**2** (500 mg) was obtained from **1e** by a two-step reaction with reference to the synthetic method of example **1,** two-step yield: 39%.

$^1$H NMR(400 MHz, DMSO-$d_6$)δ8.40-8.36 (m, 1H), 7.53 (br s, 1H), 6.87 (brs, 1H),6.20-6.18 (m, 2H), 4.67 (br s, 1H), 4.19-3.73 (m, 5H), 3.43-3.41 (m, 1H), 3.33 (s, 3H), 2.56-2.51 (m, 1H), 2.41-2.40 (m, 1H), 1.88-1.76 (m, 1H), 1.61-1.58 (m, 1H), 1.12-1.10 (m, 9H).

MS-ESI calcd.for $[M+H]^+$413, found 413.

Example 3

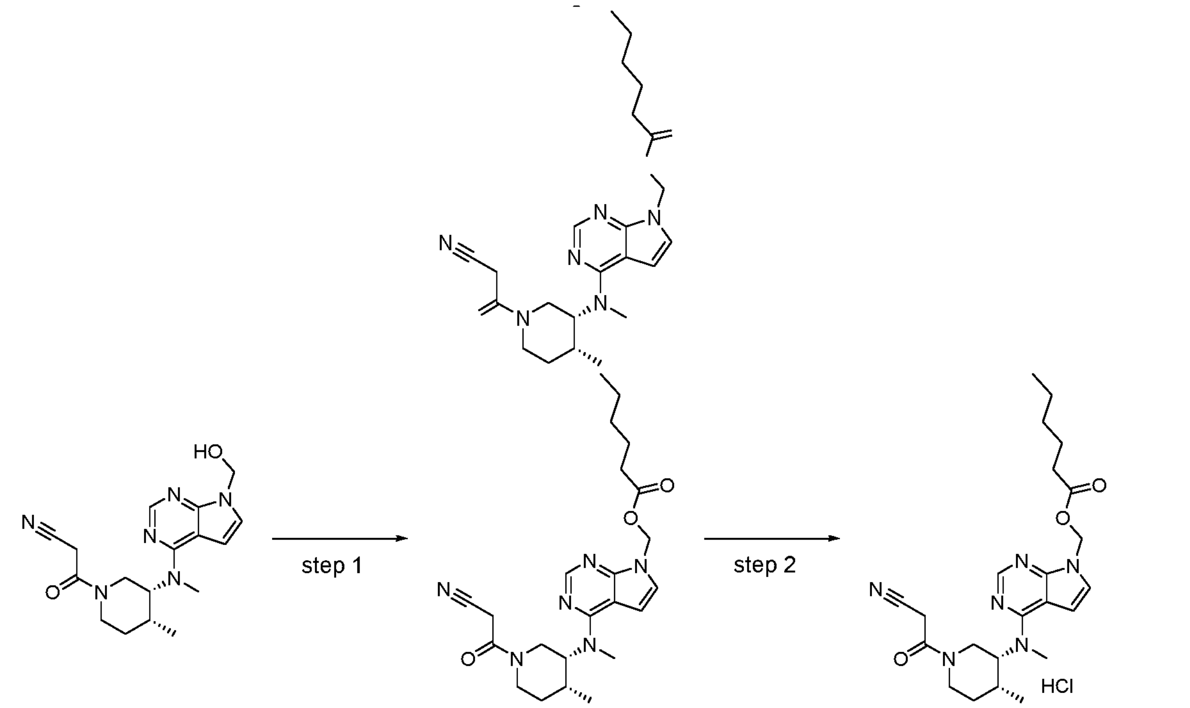


**1e** **3a** **3**

**3** (500 mg) was obtained from **1e** by a two-step reaction with reference to the synthetic method of example **1,**

two-step yield: 37%.

$^1$H NMR(400 MHz, DMSO-$d_6$)δ8.38-8.34 (m, 1H), 7.50 (br s, 1H), 6.85(br s, 1H), 6.19-6.17 (m, 2H), 4.68(br s, 1H), 4.19-3.74 (m, 4H), 3.43-3.32 (m, 5H), 2.42-2.39 (m, 1H), 2.29 (t, J= 7.6 Hz, 2H), 1.89-1.78 (m, 1H), 1.57-1.46 (m, 3H), 1.24-1.16 (m, 4H), 1.07-1.02 (m, 3H), 0.82-0.78 (m, 3H).

MS-ESI calcd.for $[M+H]^+$441, found 441.

**Example 4**

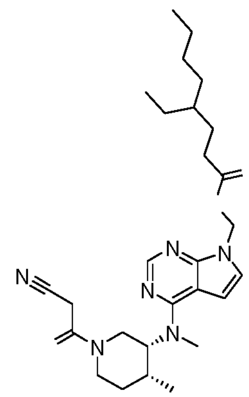

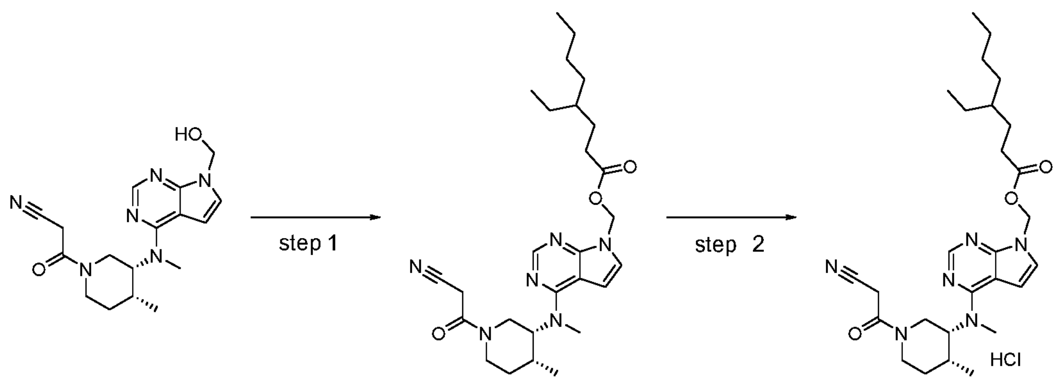


**1e** **4a** **4**

**4** (4.47 g) was obtained from **1e** by a two-step reaction with reference to the synthetic method of example **1,** two-step yield: 64%.

$^1$H NMR(400 MHz, DMSO-$d_6$)δ8.38-8.34 (m, 1H), 7.52-7.50 (m, 1H), 6.86-6.85 (m, 1H), 6.20-6.18 (m, 2H), 4.69 (br s, 1H), 4.19-3.74 (m, 5H), 3.43-3.41 (m, 1H), 3.33 (s, 3H), 2.44-2.41 (m, 1H), 2.28 (t, *J* = 7.6 Hz, 1H), 1.91-1.80 (m, 1H), 1.62-1.57 (m, 1H), 1.46-1.44 (m, 2H), 1.20-1.02 (m, 12H), 0.84-0.80 (m, 3H), 0.76-0.72 (m, 3H).

MS-ESI calcd.for $[M+H]^+$497, found 497.

**Example 5**

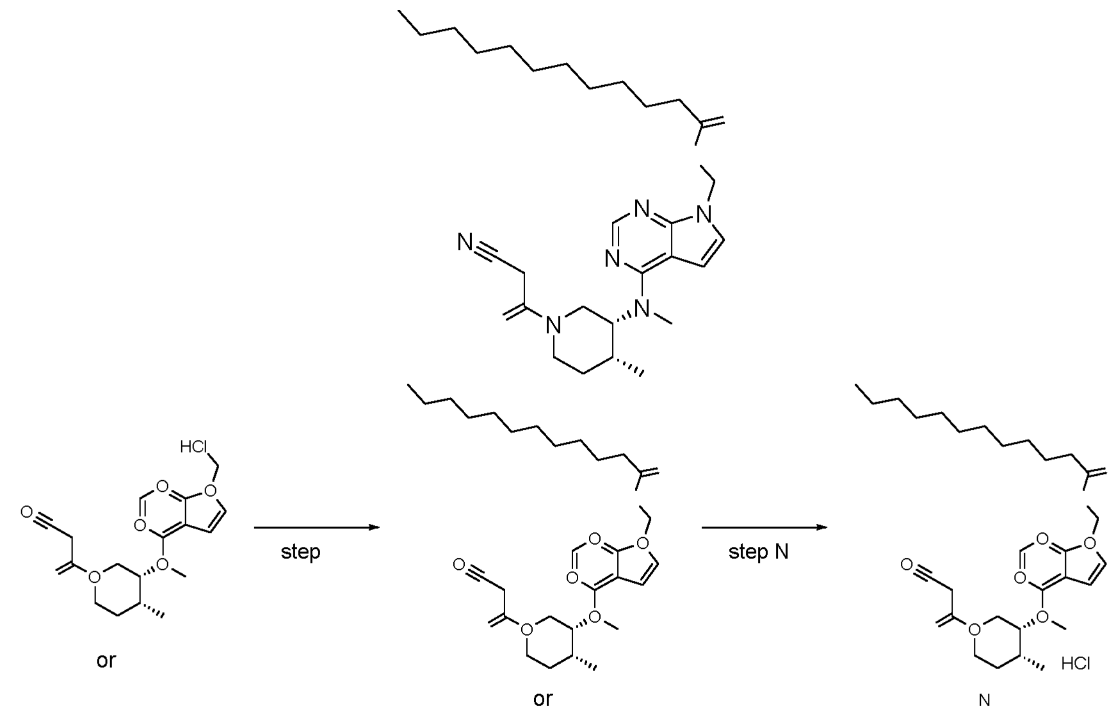


**5** (815 mg) was obtained from **1e** by a two-step reaction with reference to the synthetic method of example **1,** two-step yield: 42%.

$^1$H NMR(400 MHz, DMSO-$d_6$)δ8.27-8.26 (m, 1H), 7.40-7.39 (m, 1H), 6.76-6.75 (m, 1H), 6.15-6.14 (m, 2H), 4.77 (br s, 1H), 4.17-3.70 (m, 5H), 3.43-3.20 (m, 5H), 2.41-2.33 (m, 1H), 2.28 (t, *J* = 7.6 Hz, 2H), 1.89-1.74 (m, 1H), 1.59-1.45 (m, 3H), 1.28-1.17 (m, 16H), 1.04-1.01 (m, 3H), 0.87-0.83 (m, 3H).

MS-ESI calcd.for $[M+H]^+$525, found 525.

**Example 6**

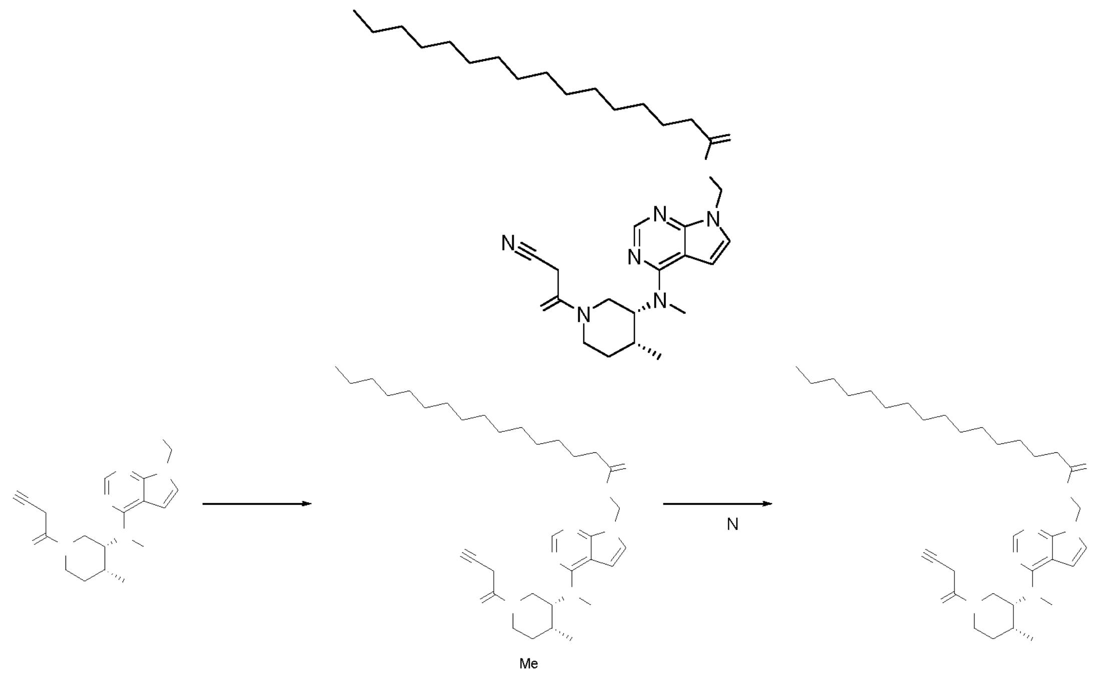

**6** (510 mg) was obtained from **1e** by a two-step reaction with reference to the synthetic method of example **1,** two-step yield: 35%.

$^{1}$H NMR (400 MHz, DMSO-$d_6$) δ8.30-8.27 (m, 1H), 7.42-7.41 (m, 1H), 6.78-6.77 (m, 1H), 6.16-6.15(m, 2H), 4.73(br s, 1H), 4.17-3.70 (m, 5H), 3.42-3.40 (m, 1H), 3.29 (s, 3H), 2.40-2.38 (m, 1H), 2.28 (t, *J* = 7.6 Hz, 2H), 1.89-1.76 (m, 1H), 1.62-1.45 (m, 3H), 1.27-1.17 (m, 24H), 1.05-1.01 (m, 3H), 0.87-0.83 (m, 3H).

MS-ESI calcd.for $[M+H]^+$581, found 581.

**Example 7**

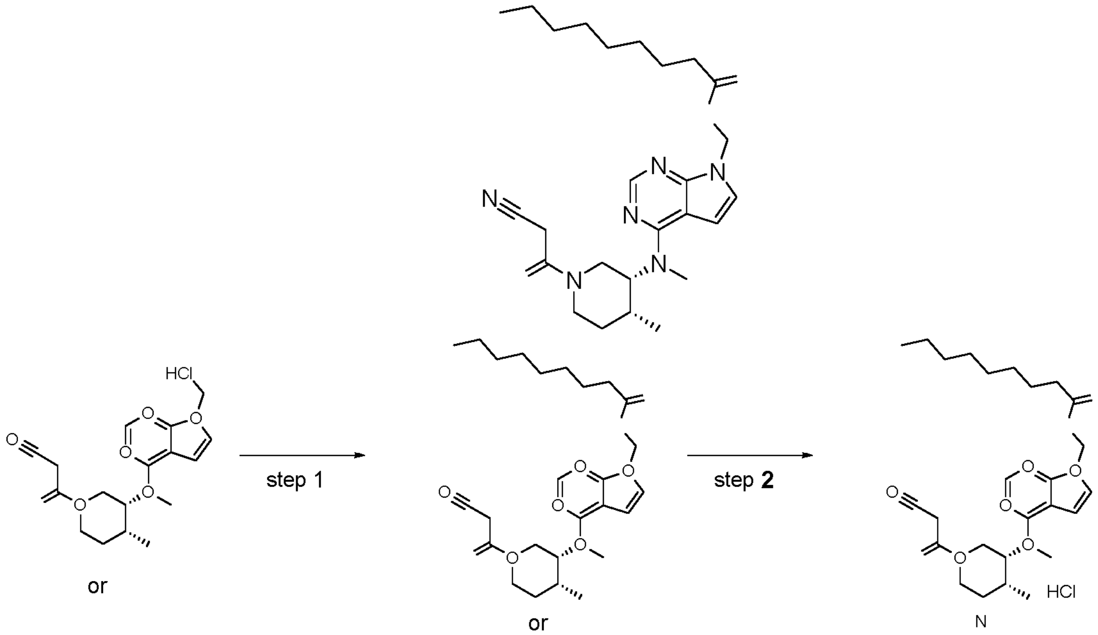


or

or

**7** (350 mg) was obtained from **1e** by a two-step reaction with reference to the synthetic method of example **1,** two-step yield: 33%.

$^{1}$H NMR (400 MHz, DMSO-$d_6$) δ8.37-8.37 (m, 1H), 7.52-7.49 (m, 1H), 6.85 (br s, 1H), 6.19-6.17 (m, 2H), 4.70 (br s, 1H), 4.18-3.71 (m, 5H), 3.42-3.40 (m, 1H), 3.32 (s, 3H), 2.42-2.37 (m, 1H), 2.29 (t, *J* = 7.6 Hz, 2H), 1.90-1.86 (m, 1H), 1.62-1.46 (m, 3H), 1.24-1.17 (m, 10H), 1.07-1.02 (m, 3H), 0.83-0.82 (m, 3H).

MS-ESI calcd.for $[M+H]^+$483, found 483.

**Example 8**

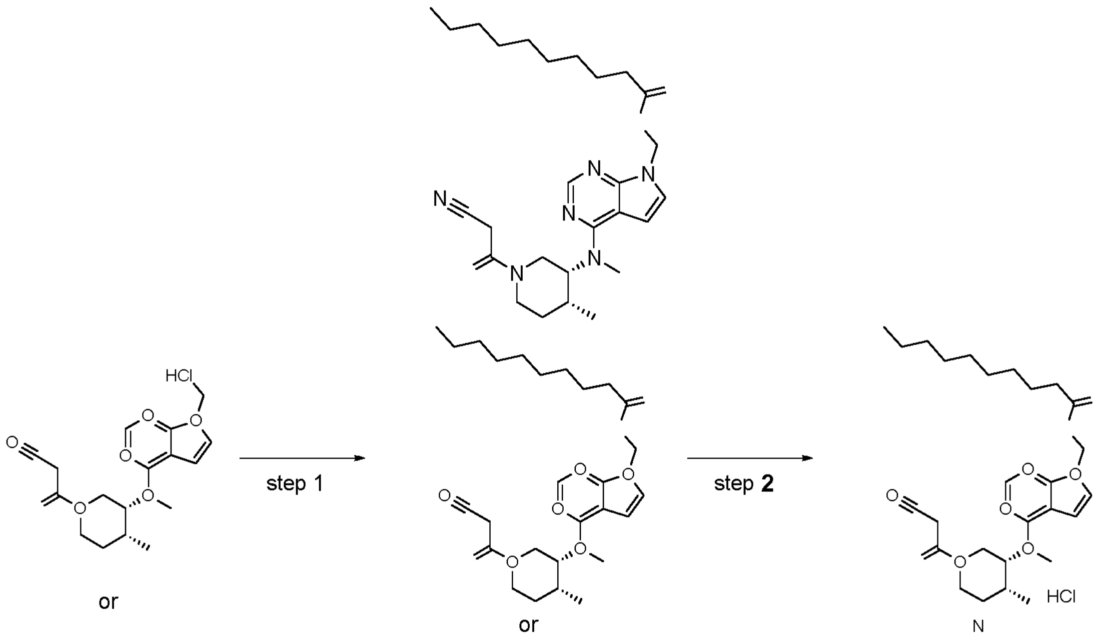


or

or

**8** (350 mg) was obtained from **1e** by a two-step reaction with reference to the synthetic method of example **1,** two-step yield: 36%.

$^1$H NMR(400 MHz, DMSO-$d_6$) δ8.38-8.34 (m, 1H), 7.52-7.50 (m, 1H), 6.85 (br s, 1H), 6.18-6.17 (m, 2H), 4.69 (br s, 1H), 4.18-3.68 (m, 5H), 3.42-3.40 (m, 1H), 3.32 (s, 3H), 2.42-2.37 (m, 1H), 2.29 (t, $J$ = 7.2 Hz, 2H), 1.92-1.83(m, 1H), 1.62-1.46 (m, 3H), 1.24-1.18 (m, 12H), 1.07-0.98 (m, 3H), 0.86-0.83 (m, 3H).

MS-ESI calcd for [M+H]$^+$497, found 497.

## Example 9

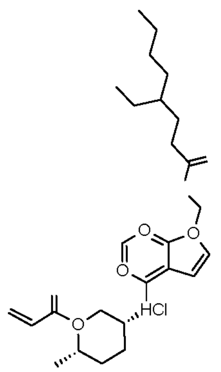

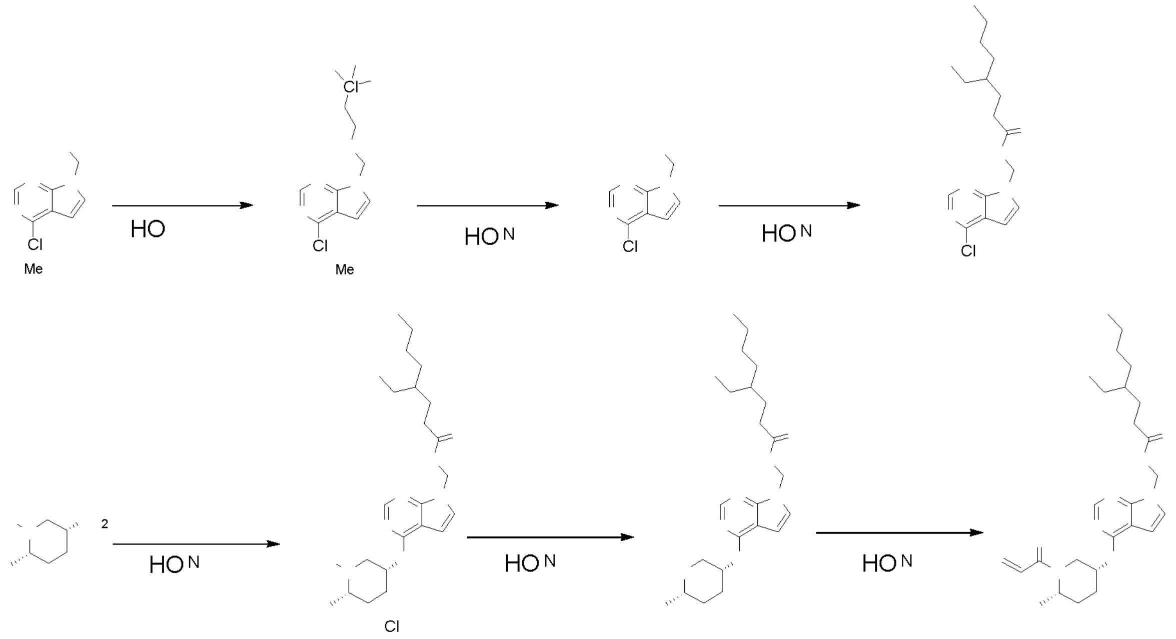

**Step 1**

**9a** (5.00 g, 32.89 mmol) was dissolved in *N,N*-dimethylformamide (30 mL), 60% w/w sodium hydride (1.58 g, 36.18 mmol) was added to the above solution at 0°C under $N_2$ atmosphere. After stirring for 0.5 h at 0°C, 2-(trimethylsilyl)ethoxymethyl chloride (6.00 g, 36.18 mmol) was added to the resultant suspension, the mixture was slowly warmed up to room temperature and and continued to stir for 1 h. Water (100 mL) was added to quench the reaction, the mixture was extracted with ethyl acetate (100 mL × 3)and the organic phase was washed with brine (100 mL × 1), dried over anhydrous sodium sulfate, filtered and concentrated under reduced pressure to obtain the residue, which was purified by column chromatography (ethyl acetate: hexanes = 0-100%) to give **9b** (5.10 g), yield: 55%.

$^1$H NMR (400 MHz, $CDCl_3$) δ 8.66 (s, 1H), 7.39 (d, *J* = 3.6 Hz, 1H), 6.66 (d, *J* = 3.6 Hz, 1H), 5.64 (s, 2H), 3.52 (t, *J* = 8.4 Hz, 2H), 0.90 (t, *J* = 8.4 Hz, 2H), -0.06 (s, 9H).

MS-ESI calcd.for $[M+H]^+$284, found 284.

**Step 2**

**9b** (2.00 g, 7.07 mmol) was dissolved in dichloromethane (10 mL), trifluoroacetic acid (8.80 g, 70.67 mmol) was added to the above solution, the mixture was stirred at room temperature for 16 h. The reaction solution was diluted with water (50 mL), extracted with dichloromethane (50 mL × 3)and the organic phase was washed with brine (50 mL × 1), dried over anhydrous sodium sulfate, filtered and concentrated under reduced pressure to obtain **9c** (1.20 g), yield: 92%.

$^1$H NMR (400 MHz, DMSO-$d_6$) δ 8.67 (s, 1H), 7.79 (d, *J* = 3.6 Hz, 1H), 6.68 (d, *J* = 3.6 Hz, 1H), 5.63 (s, 2H).

MS-ESI calcd.for $[M+H]^+$184, found 184.

**Step 3**

**9c** (2.00 g, 7.07 mmol), 1-(3-dimethylaminopropyl)-3-ethylcarbodiimide hydrochloride (2.50 g, 13.11 mmol) and 4-dimethylaminopyridine (1.60 g, 13.11 mmol) were dissolved in dichloromethane (30 mL). After stirring for 0.5 h at room temperature, 4-ethyloctanoic acid (1.19 g, 13.11 mmol) was added to the above solution, the mixture was stirred at room temperature for 16 h. The reaction solution was diluted with water (100 mL), extracted with ethyl acetate (100 mL × 3), the organic phase was washed with brine (100 mL × 1), dried over anhydrous sodium sulfate, filtered and concentrated under reduced pressure to obtain the residue, which was purified by column chromatography (ethyl acetate: hexanes = 0-100%) to give **9d** (1.21 g), yield: 55%.

$^1$H NMR (400 MHz, $CDCl_3$) δ 8.70 (s, 1H), 7.49 (d, *J* = 3.6 Hz, 1H), 6.63 (d, *J* = 3.6 Hz, 1H), 6.22 (s, 2H), 2.34-2.27 (m, 2H), 1.59-1.50 (m, 2H), 1.25-1.13 (m, 9H), 0.85 (t, *J* = 5.4 Hz, 3H), 0.78 (t, *J* = 7.2 Hz, 3H).

**Step 4**

**9e** (0.27 g, 0.98 mmol), *N,N*-diisopropylethylamine (0.25 g, 1.96 mmol) and **9d** (0.33 g, 1.08 mmol) were dissolved in dimethyl sulfoxide (5 mL), and the resultant solution was stirred at 100 °C for 16 h. The reaction solution was diluted with water (50 mL), extracted with ethyl acetate (50 mL × 3) and the organic phase was washed with brine (50 mL × 1), dried over anhydrous sodium sulfate, filtered and concentrated under reduced pressure to obtain the residue, which was purified by column chromatography (ethyl acetate: hexanes = 0-100%) to give **9f** (0.35 g), yield: 66%.

$^1$H NMR (400 MHz, $CDCl_3$) δ 8.35 (s, 1H), 7.41-7.30 (m, 5H), 7.13 (d, *J* = 3.6 Hz, 1H), 6.38 (d, *J* = 3.6 Hz, 1H), 6.14 (s, 2H), 5.23-5.10 (m, 2H), 4.58-4.45 (m, 2H), 2.79-2.67 (m, 1H), 2.30 (t, *J* = 8.0 Hz, 2H), 2.00-1.83 (m, 4H), 1.66 (d, *J* = 10.8 Hz, 2H), 1.58-1.53 (m, 2H), 1.27-1.17 (m, 12H), 0.85 (t, *J* = 6.8 Hz, 3H), 0.79 (t, *J* = 7.2 Hz, 3H).

**Step 5**

**9f** (0.35 g, 0.64 mmol) was dissolved in tetrahydrofuran (5 mL), 10% wet palladium carbon (0.15 g) was added to the above solution, and the reaction was evacuated and backfilled with $H_2$ three times, stirred at room temperature for 2 h. The reaction solution was filtered through a pad of celite and the filtrate was concentrated under reduced pressure to give **9h** (0.26 g), yield: 97%.

$^1$H NMR (400 MHz, $CDCl_3$) δ 8.38 (s, 1H), 7.14 (d, *J* = 3.6 Hz, 1H), 6.44 (d, *J* = 3.6 Hz, 1H), 6.14 (s, 2H), 6.12 (s, 1H), 4.39 (s, 1H), 3.18-2.93 (m, 2H), 2.76-2.66 (m, 1H), 2.33-2.23 (m, 2H), 2.10-1.99 (m, 2H), 1.66-1.45 (m, 6H), 1.26-1.16 (m, 9H), 1.10 (d, *J* = 6.0, 3H), 0.85 (t, *J* = 6.8 Hz, 3H), 0.79 (t, *J* = 7.2 Hz, 3H).

MS-ESI calcd.for $[M+H]^+$416, found 416.

**Step 6**

**9h** (0.22 g, 0.52 mmol) was dissolved in tetrahydrofuran (10 mL), 0.52 N aqueous sodium bicarbonate (5 mL) was added to the above solution, the reaction solution was cooled down to 0°C and acryloyl chloride (0.06 g, 0.62 mmol) was added dropwise, the mixture was slowly warmed up to room temperature and continued to stir for 2 h. The reaction solution was diluted with water (50 mL), extracted with ethyl acetate (50 mL × 3) and the organic phase was washed with brine (50 mL × 1), dried over anhydrous sodium sulfate, filtered and concentrated under reduced pressure to obtain the residue, which was purified by column chromatography (ethyl acetate: hexanes = 0-100%) to give **9** (0.21 g), yield: 87%.

$^1$H NMR (500 MHz, DMSO-$d_6$) δ 8.20 (s, 1H), 7.36 (d, *J* = 7.5 Hz, 1H), 7.23 (d, *J* = 4.0 Hz, 1H), 6.75 (dd, *J* = 16.5, 10.5 Hz, 1H), 6.66 (d, *J* = 4.0 Hz, 1H), 6.11 (s, 2H), 6.07 (dd, *J* = 16.5, 2.5 Hz, 1H), 5.65 (dd, *J* = 10.5, 2.5 Hz, 1H), 4.86-4.15 (m, 2H), 4.11-4.02 (m, 1H), 2.97-2.60 (m, 1H), 2.27 (t, *J* = 7.5 Hz, 2H), 1.89-1.65 (m, 4H), 1.49-1.43 (m, 2H), 1.22-1.13 (m, 12H), 0.83 (t, *J* = 7.0 Hz, 3H), 0.75 (t, *J* = 7.5 Hz, 3H).

MS-ESI calcd.for $[M+H]^+$470, found 470.

**Example 10**

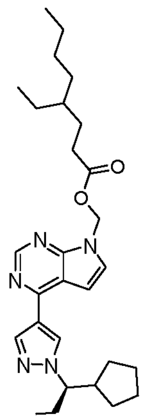

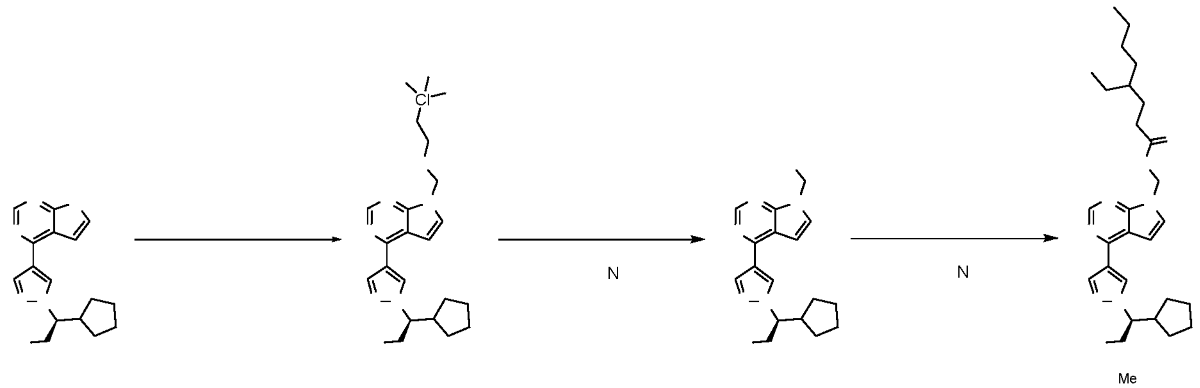

**Step 1**

**10a** (48 mg, 0.16 mmol) and N,N diisopropylethylamine (30 mg, 0.24 mmol) were dissolved in dichloromethane (3 mL)and stirred at room temperature for 5 min, 2-(trimethylsilyl)ethoxymethyl chloride (39 mg, 0.24 mmol) was added to the above solution, and the mixture was stirred for 3 h at room temperature. The reaction solution was concentrated under reduced pressure to give the residue, which was purified by column chromatography (ethyl acetate: hexanes = 0-100%) to give **10b** (62 mg), yield: 91%.

$^1$H NMR (500 MHz, DMSO-*d*$_6$) δ 8.82 (d, *J* = 0.8 Hz, 1H), 8.75 (s, 1H), 8.39 (s, 1H), 7.77 (d, *J* = 3.5 Hz, 1H), 7.09 (d, *J* = 3.5 Hz, 1H), 5.63 (s, 2H), 4.54 (td, *J* = 9.5, 4.0 Hz, 1H), 3.57-3.48 (m, 2H), 3.30-3.16 (m, 2H), 2.45-2.35 (m, 1H), 1.85-1.22 (m, 8H), 0.83 (t, *J* = 8.5 Hz, 2H), -0.10 (s, 9H).

MS-ESI calcd.for $[M+H]^+$437, found 437.

**Step 2**

**10b** (58 mg, 0.13 mmol) was dissolved in dichloromethane (2 mL), trifluoroacetic acid (379 mg, 3.33 mmol) was added to the above solution, the mixture was stirred at room temperature for 6 h. The reaction solution was diluted with saturated aqueous sodium bicarbonate (20 mL), extracted with dichloromethane (20 mL × 3)and the organic phase was dried over anhydrous sodium sulfate, filtered and concentrated under reduced pressure to obtain **10c** (48 mg), yield: 99%.

**Step 3**

**10c** (36 mg, 0.11 mmol), 1-(3-dimethylaminopropyl)-3-ethylcarbodiimide hydrochloride (41 mg, 0.21 mmol) and 4-dimethylaminopyridine (26 mg, 0.21 mmol) were dissolved in dichloromethane (3 mL). After stirring for 0.5 h at room temperature, 4-ethyloctanoic acid (37 mg, 0.21 mmol) was added to the above solution, the mixture was stirred at room temperature for 2 h. The reaction solution was concentrated under reduced pressure to obtain the residue, which was purified by column chromatography (ethyl acetate: hexanes = 0-100%) to give **10** (37 mg), yield: 71%.

$^1$H NMR (400 MHz, $CDCl_3$) δ 8.89 (s, 1H), 8.34 (s, 1H), 8.30 (s, 1H), 7.49 (d, *J* = 3.6 Hz, 1H), 6.77 (d, *J* = 3.6 Hz, 1H), 6.25 (s, 2H), 4.27 (td, *J* = 9.4, 3.2Hz, 1H), 3.18-3.07 (m, 1H), 3.00-2.90 (m, 1H), 2.64-2.55 (m, 1H), 2.34-2.29 (m, 2H), 2.05-1.92 (m, 2H), 1.73-1.54 (m, 8H), 1.22-1.16 (m, 9H), 0.84 (t, *J* = 7.0 Hz, 3H), 0.78 (t, *J* = 7.2 Hz, 3H).

MS-ESI calcd.for $[M+H]^+$491, found 491.

**Example 11**

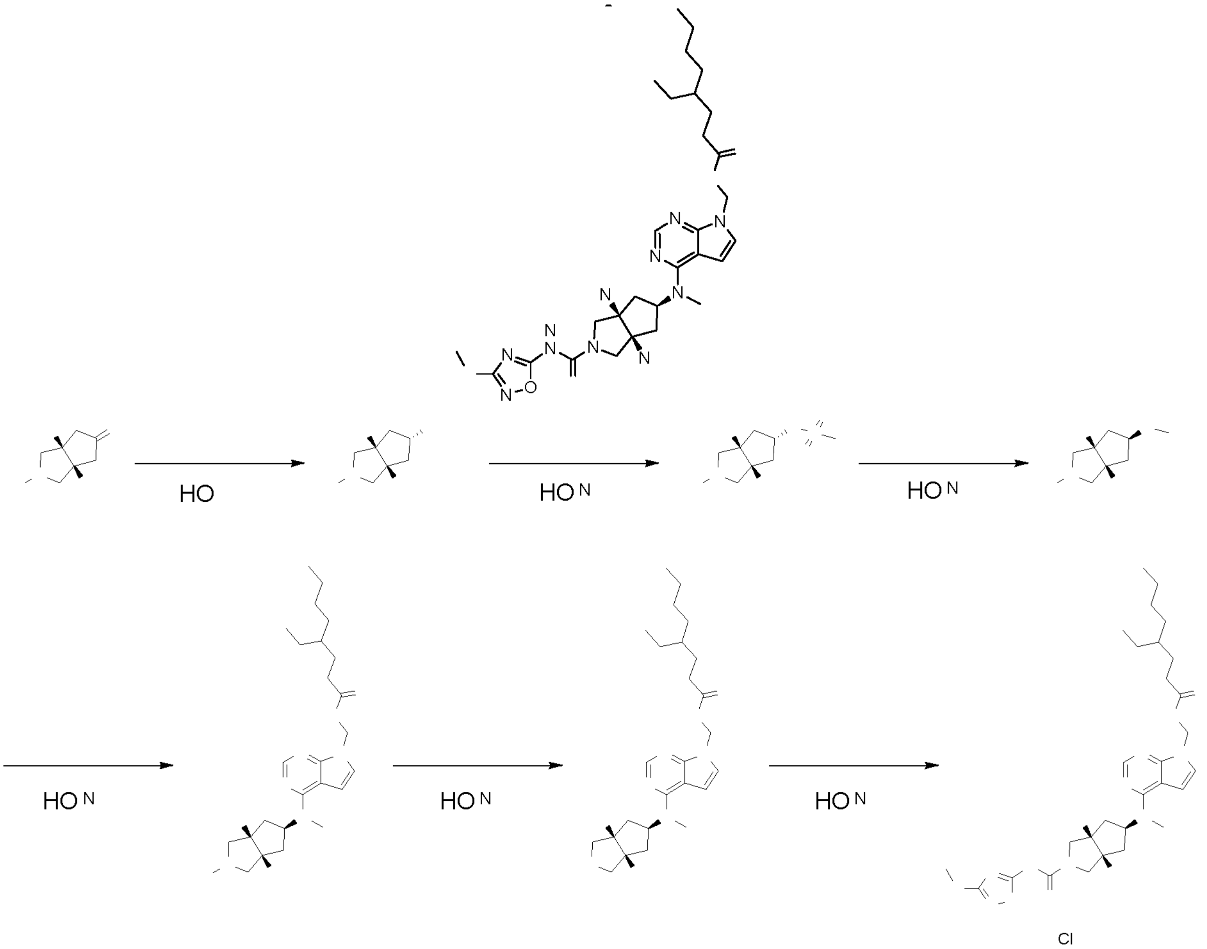


**Step** 1

**11a** (0.99 g, 4.40 mmol) was dissolved in methanol (10 mL), sodium borohydride (0.20 g, 5.29 mmol) was added to the above solution at 0°C under $N_2$ atmosphere, the mixture was slowly warmed up to room temperature and continued to stir for 1 h. The reaction solution was diluted with brine (50 mL), extracted with dichloromethane (50 mL × 3)and the organic phase was dried over anhydrous sodium sulfate, filtered and concentrated under reduced pressure to obtain **11b** (1.01 g), yield: 99%.

$^1$H NMR (400 MHz, $CDCl_3$) δ 4.30 (p, *J* = 6.4 Hz, 1H), 3.54-3.45 (m, 2H), 3.38-3.30 (m, 2H), 2.62-2.57 (m, 2H), 2.24-2.10 (m, 2H), 1.53-1.48 (m, 2H), 1.45 (s, 9H).

**Step 2**

**11b** (0.80 g, 3.52 mmol) was dissolved in dichloromethane (3 mL), *N,N*-diisopropylethylamine (0.91 g, 7.05 mmol) and methanesulfonic anhydride (1.23 g, 7.05 mmol) were added sequentially to the above solution under $N_2$ atmosphere, the mixture was stirred at room temperature for 3 h. The reaction solution was diluted with water (25 mL), extracted with ethyl acetate (25 mL × 3) and the organic phase was washed with brine (50 mL × 1), dried over anhydrous sodium sulfate, filtered and concentrated under reduced pressure to obtain the residue, which was purified by column chromatography (ethyl acetate: hexanes = 0-100%) to give **11c** (0.90 g), yield: 84%.

$^1$H NMR (400 MHz, $CDCl_3$) δ 5.11 (p, *J* = 6.0 Hz, 1H), 3.54 (s, 1H), 3.36 (s, 1H), 3.05-2.96 (m, 3H), 2.68-2.66 (m, 2H), 2.36-2.29 (m, 2H), 2.06-1.95 (m, 2H), 1.89-1.82 (m, 2H), 1.25 (s, 9H).

**Step 3**

**11c** (0.90 g, 2.95 mmol) was dissolved in 30% methylamine methanol solution (10 mL) in a schlenk tube, the mixture was sealed and stirred at 80°C for 7 h. The reaction solution was cooled to room temperature and diluted with water (100 mL), extracted with ethyl acetate (100 mL × 3) and the organic phase was washed with brine (50 mL × 1), dried over anhydrous sodium sulfate, filtered and concentrated under reduced pressure to obtain the residue, which was purified by column chromatography (methanol: dichloromethane = 0-100%) to give **11d** (0.68 g), yield: 86%.
$^1$H NMR (400 MHz, $CDCl_3$) δ 3.53-3.48 (m, 2H), 3.15-3.12 (m, 2H), 2.95-2.87 (m, 2H), 2.76 (s, 2H), 2.66 (s, 3H), 2.18-2.11 (m, 2H), 2.00-1.95 (m, 2H), 1.44 (s, 9H).

**Step 4**

**11d** (0.68 g, 2.01 mmol), **9d** (0.54 g, 2.01 mmol) and N,N diisopropylethylamine (0.52 g, 4.03 mmol) were dissolved in*N*-methylpyrrolidone (8 mL) in a microwave tube, the reaction was stirred at 150°C in the microwave for 3 h. The reaction solution was cooled down to room temperature and diluted with water (50 mL), extracted with ethyl acetate (50 mL × 3) and the organic phase was washed with brine (50 mL × 1), dried over anhydrous sodium sulfate, filtered and concentrated under reduced pressure to obtain the residue, which was purified by column chromatography (ethyl acetate: hexanes = 0-100%) to give **11e** (0.65 g), yield: 60%.
$^1$H NMR (400 MHz, $CDCl_3$) δ 8.36 (s, 1H), 7.13 (d, *J* = 4.0 Hz, 1H), 6.52 (d, *J* = 4.0 Hz, 1H), 6.15 (s, 2H), 5.57-5.47 (m, 1H), 3.61 (s, 2H), 3.32-3.10 (m, 5H), 2.83 (s, 2H), 2.32-2.25 (m, 2H), 2.05-1.93 (m, 2H), 1.92-1.83 (m, 2H), 1.57-1.52 (m, 2H), 1.48 (s, 9H), 1.25-1.16 (m, 9H), 0.85 (t, *J* = 6.8 Hz, 3H), 0.79 (t, *J* = 7.2 Hz, 3H).
MS-ESI calcd.for $[M+H]^+$542, found 542.

**Step 5**

**11e** (0.65 g, 1.20 mmol) was dissolved in dichloromethane (20 mL), trifluoroacetic acid (2.74 g, 24.03 mmol) was added to the above solution, the mixture was stirred at room temperature for 3 h. The reaction solution was concentrated under reduced pressure to obtain **11f** (0.80 g), yield: 99%.
MS-ESI calcd.for $[M+H]^+$442, found 442.

**Step 6**

**11f** (0.80 g, 1.2 mmol), triethylamine (0.97 g, 9.60 mmol) and phenyl (3-methoxy-1,2,4-thiadiazol-5-yl) carbamate (0.33 g, 1.32 mmol) were dissolved in tetrahydrofuran (10 mL), and the mixture was stirred at 70°C for 5 h. The reaction solution was diluted with water (50 mL), extracted with ethyl acetate (50 mL × 3) and the organic phase was washed with saturated salt water (50 mL × 1), dried over anhydrous sodium sulfate, filtered and concentrated under reduced pressure to obtain the residue, which was purified by column chromatography (ethyl acetate: hexanes = 0-100%) to give **11** (0.56 g), yield: 79%.
$^1$H NMR (400 MHz, DMSO-$d_6$) δ 11.58 (s, 1H), 8.17 (s, 1H), 7.23 (d, *J* = 4.0 Hz, 1H), 6.64 (d, *J* = 4.0 Hz, 1H), 6.09 (s, 2H), 5.44 (p, *J* = 9.0 Hz, 1H), 3.90 (s, 3H), 3.74-3.61 (m, 2H), 3.44-3.35 (m, 2H), 3.15 (s, 3H), 2.90 (s, 2H), 2.25 (t, *J* = 7.6 Hz, 2H), 2.05-1.97 (m, 2H), 1.82-1.73 (m, 2H), 1.48-1.37 (m, 2H), 1.20-1.07 (m, 9H), 0.80 (t, *J* = 7.2 Hz, 3H), 0.73 (t, *J* = 7.2 Hz, 3H).
MS-ESI calcd.for $[M+H]^+$599, found 599.

Example 12

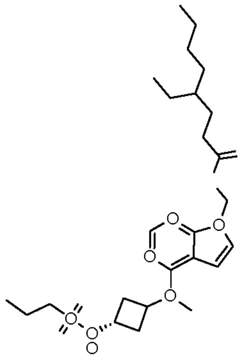

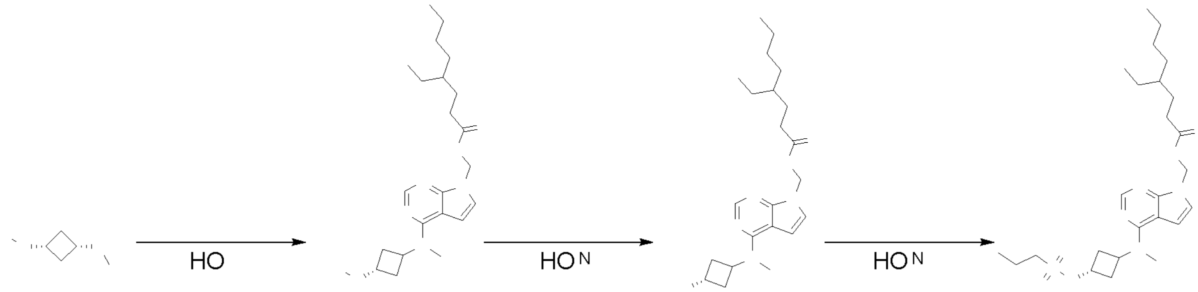

**Step** 1

**12a** (195 mg, 0.83 mmol), *N,N*-diisopropylethylamine (215 mg, 1.67 mmol) and **9d** (337 mg, 1.00 mmol) were dissolved in dimethyl sulfoxide (6 mL), and the mixture was stirred at 100°C for 7 h. The reaction solution was diluted with water (50 mL), extracted with ethyl acetate (50 mL × 3) and the organic phase was washed with brine (50 mL × 1), dried over anhydrous sodium sulfate, filtered and concentrated under reduced pressure to obtain the residue, which was purified by column chromatography (ethyl acetate: hexanes = 0-100%) to give **12b** (420 mg), yield: 94%.

$^1$H NMR (400 MHz, DMSO-$d_6$) δ 8.19 (s, 1H), 7.63 (d, *J* = 8.0 Hz, 1H), 7.41-7.31 (m, 5H), 7.30 (d, *J* = 4.0 Hz, 1H), 6.75 (d, *J* = 4.0 Hz, 1H), 6.11 (s, 2H), 5.02 (s, 2H), 4.96-4.85 (m, 1H), 3.88-3.77 (m, 1H), 3.25 (s, 3H), 2.57-2.51 (m, 2H), 2.29-2.18 (m, 4H), 1.47-1.39 (m, 2H), 1.34-0.91 (m, 9H), 0.80 (t, *J* = 7.0 Hz, 3H), 0.73 (t, *J* = 7.0 Hz, 3H).

MS-ESI calcd.for $[M+H]^+$536, found 536.

**Step 2**

**12b** (0.42 g, 0.79 mmol) was dissolved in tetrahydrofuran (12 mL), 10% wet palladium carbon (0.20 g) was added and the reaction was evacuated and backfilled with $H_2$ three times, stirred at room temperature for 1.5 h. The reaction solution was filtered through a pad of celite and the filtrate was concentrated under reduced pressure to obtain the residue, which was purified by column chromatography (ethyl acetate: hexanes = 0-100%) to give **12c** (0.30 g), yield: 95%.

$^1$H NMR (400 MHz, DMSO-$d_6$) δ 8.17 (s, 1H), 7.29 (d, *J* = 4.0 Hz, 1H), 6.71 (d, *J* = 4.0 Hz, 1H), 6.11 (s, 2H), 4.83-4.73 (m, 1H), 3.26 (s, 3H), 3.14-3.05 (m, 1H), 2.47-2.41 (m, 2H), 2.26 (t, *J* = 7.6 Hz, 2H), 2.03-1.93 (m, 2H), 1.46-1.40 (m, 2H), 1.19-1.06 (m, 9H), 0.80 (t, *J* = 7.0 Hz, 3H), 0.73 (t, *J* = 7.2 Hz, 3H).

MS-ESI calcd.for $[M+H]^+$402, found 402.

**Step 3**

**12c** (298 mg, 0.74 mmol) was dissolved in dichloromethane (10 mL), triethylamine (150 mg, 14.9 mmol) was added to the above solution at 0°C under $N_2$ atmosphere, and 1-propanesulfonyl chloride (127 mg, 0.89 mmol) was added dropwise to the above solution, the mixture was slowly warmed up to room temperature and and continued to stir for 2 h. The reaction solution was concentrated under reduced pressure to obtain the residue, which was purified by column chromatography (ethyl acetate: hexanes = 0-100%) to give **12** (260 mg), yield: 69%.

$^1$H NMR (500 MHz, DMSO-$d_6$) δ 8.19 (s, 1H), 7.50 (d, *J* = 9.0 Hz, 1H), 7.31 (d, *J* = 4.0 Hz, 1H), 6.76 (d, *J* = 4.0 Hz, 1H), 6.11 (s, 2H), 4.88 (p, *J* = 9.5 Hz, 1H), 3.62-3.53 (m, 1H), 3.25 (s, 3H), 2.96-2.89 (m, 2H), 2.62-2.56 (m, 2H), 2.28-2.18 (m, 4H), 1.73-1.62 (m, 2H), 1.46-1.39 (m, 2H), 1.24-1.05 (m, 9H), 0.97 (t, *J* = 7.5 Hz, 3H), 0.80 (t, *J* = 7.0 Hz, 3H), 0.73 (t, *J* = 7.5 Hz, 3H).

MS-ESI calcd.for $[M+H]^+$508, found 508.

**Example 13**

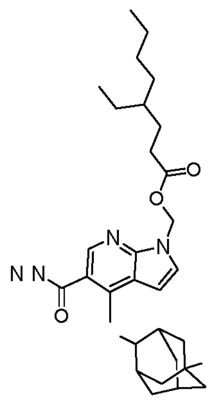

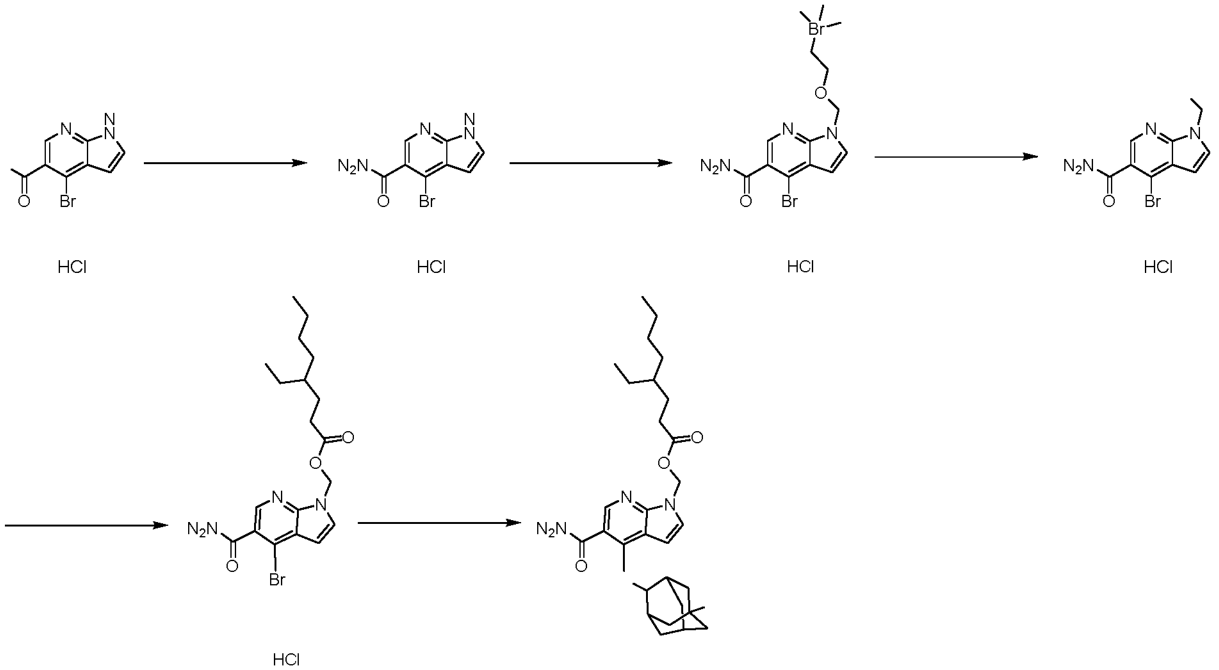

**Step 1**

**13a** (0.85 g, 4.34 mmol) and *N,N'*-carbonyldiimidazole (1.05 g, 6.51 mmol) were dissolved in *N,N*-dimethylformamide (8 mL) under $N_2$ atmosphere, and the mixture was stirred at room temperature for 2 h, the reaction solution was cooled to 0°C and 28% ammonia hydroxide solution (1.3 mL) was added dropwise to the above solution, the mixture was slowly warmed up to room temperature and continued to stir for 1 h. The reaction solution was diluted with dichloromethane (50 mL), filtered and washed with water (50 mL), the filter cake was collected and dried under vacuum to give **13b** (0.77 g), yield: 90%.

$^1H$ NMR (400 MHz, DMSO-$d_6$) δ 12.10 (s, 1H), 8.29 (s, 1H), 7.89 (s, 1H), 7.64 (d, *J* = 3.6 Hz, 1H), 7.62 (s, 1H), 6.56 (d, *J* = 3.6 Hz, 1H).

MS-ESI calcd.for $[M+H]^+$ 196, found 196.

**Step 2**

**13b** (0.72 g, 3.68 mmol) and N,N diisopropylethylamine (0.71 g, 5.52 mmol) were dissolved in *N,N*-dimethylformamide (10 mL) under $N_2$ atmosphere, 2-(trimethylsilyl)ethoxymethyl chloride (0.92 g, 5.52 mmol) was added dropwise to the above solution and the reaction mixture was stirred at room temperature for 1 h. The reaction solution was diluted

with water (50 mL), extracted with ethyl acetate (50 mL × 3) and the organic phase was washed with brine (50 mL × 1), dried over anhydrous sodium sulfate, filtered and concentrated under reduced pressure to obtain the residue, which was purified by column chromatography (ethyl acetate: hexanes = 0-100%) to give **13c** (1.12 g), yield: 93%.

$^{1}$H NMR (500 MHz, DMSO-$d_6$) $\delta$ 8.35 (s, 1H), 7.96 (s, 1H), 7.83 (d, *J* = 3.6 Hz, 1H), 7.70 (s, 1H), 6.66 (d, *J* = 3.6 Hz, 1H), 5.64 (s, 2H), 3.51 (t, *J* = 6.4 Hz, 2H), 0.82 (t, *J* = 6.4 Hz, 2H), - 0.09 (s, 9H).

MS-ESI calcd.for [M+H]$^+$ 326, found 326.

**Step 3**

**13c** (1.12 g, 3.45 mmol) was dissolved in dichloromethane (20 mL), trifluoroacetic acid (7.86 g, 68.92 mmol) was added to the above solution, the mixture was stirred at room temperature for 20 h. The reaction solution was concentrated under reduced pressure to obtain **13d** (1.80 g), yield: 95%.

$^{1}$H NMR (400 MHz, DMSO-$d_6$) $\delta$ 8.35 (s, 1H), 7.94 (s, 1H), 7.75 (d, *J* = 3.6 Hz, 1H), 7.67 (s, 1H), 6.62 (d, *J* = 3.6 Hz, 1H), 5.63 (s, 2H).

MS-ESI calcd.for [M+H]$^+$226, found 226.

**Step 4**

4-ethyloctanoic acid (0.97 g, 5.60 mmol), 1-(3-dimethylaminopropyl)-3-ethylcarbodiimide hydrochloride (1.08 g, 5.60 mmol) and 4-dimethylaminopyridine (1.70 mg, 14.00 mmol) were dissolved in dichloromethane (30 mL). After stirring for 10 min at room temperature, **13d** (1.60 g, 2.80 mmol) was added to the above solution, the mixture was stirred at room temperature for 2 h. The reaction solution was concentrated under reduced pressure to obtain the residue, which was purified by column chromatography (ethyl acetate: hexanes = 0-100%) to give **13e** (750 mg), yield: 75%.

$^{1}$H NMR (400 MHz, DMSO-$d_6$) $\delta$ 8.38 (s, 1H), 7.96 (s, 1H), 7.79 (d, *J* = 3.6 Hz, 1H), 7.73-7.69 (m, 1H), 6.68 (d, *J* = 3.6 Hz, 1H), 6.25 (s, 2H), 2.27 (t, *J* = 7.6 Hz, 2H), 1.47-1.39 (m, 2H), 1.25-1.10 (m, 9H), 0.81 (t, *J* = 7.0 Hz, 3H), 0.72 (t, *J* = 7.2 Hz, 3H).

MS-ESI calcd.for [M+H]$^+$380, found 380.

**Step 5**

**13e** (0.55 g, 1.45 mmol), trans-4-amino-1-hydroxy-adamantane (0.48 g, 2.90 mmol) and *N*,*N*-diisopropylethylamine (0.37 g, 2.90 mmol) were dissolved in*N*-methylpyrrolidone (15 mL) in a microwave tube, the reaction was stirred at 150°C in the microwave for 1 h. The reaction solution was diluted with water (100 mL), extracted with ethyl acetate (100 mL × 3) and the organic phase was washed with brine (200 mL × 1), dried over anhydrous sodium sulfate, filtered and concentrated under reduced pressure to obtain the residue, which was purified by column chromatography (ethyl acetate: hexanes = 0-100%) to give **13** (0.35 g), yield: 47%.

$^{1}$H NMR (400 MHz, DMSO-$d_6$) $\delta$ 10.11 (d, *J* = 8.0 Hz, 1H), 8.43 (s, 1H), 7.88 (s, 1H), 7.29 (d, *J* = 3.6 Hz, 1H), 7.12 (s, 1H), 6.49 (d, *J* = 3.6 Hz, 1H), 6.20-6.05 (m, 2H), 4.50 (s, 1H), 4.10 (d, *J* = 8.0 Hz, 1H), 2.25 (t, *J*= 7.6 Hz, 2H), 2.13 (s, 2H), 2.04 (s, 1H), 1.88-1.78 (m, 4H), 1.70-1.63 (m, 4H), 1.46-1.36 (m, 4H), 1.24-1.09 (m, 9H), 0.81 (t, *J* = 7.0 Hz, 3H), 0.72 (t, *J* = 7.2 Hz, 3H).

MS-ESI calcd.for [M+H]$^+$511, found 511.

**Example 14**

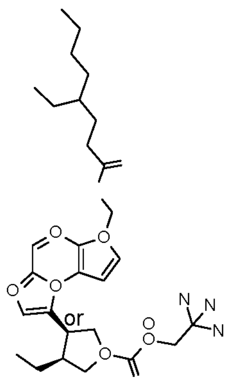

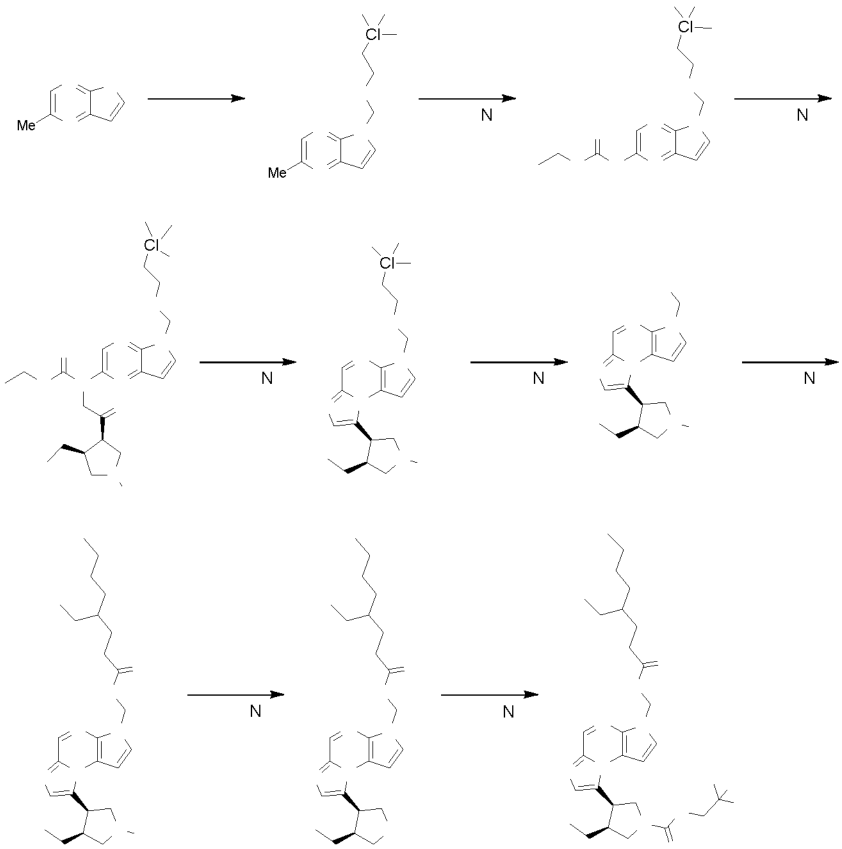


**Step 1**

**14a** (1.00 g, 5.05 mmol) was dissolved inN,N-dimethylformamide (10 mL), sodium hydride (60%, dispersion in mineral oil) (224 mg, 6.06 mmol) was added to the above solution in one batch at 0°C under $N_2$ atmosphere. After stirring for 0.5 h at 0°C, 2-(trimethylsilyl)ethoxymethyl chloride (1.01 g, 6.06 mmol) was added to the resultant suspension, the mixture was slowly warmed up to room temperature and and continued to stir for 3 h. Water (100 mL) was added to quench the reaction, the mixture was extracted with ethyl acetate (100 mL × 3)and the organic phase was washed with brine (100 mL × 1), dried over anhydrous sodium sulfate, filtered and concentrated under reduced pressure to obtain the residue, which was purified by column chromatography (ethyl acetate: hexanes = 0-100%) to give **14b** (1.60 g), yield: 96%.

$^1$H NMR (400 MHz, DMSO-$d_6$) δ 8.45 (s, 1H), 8.15 (d, *J* = 3.6 Hz, 1H), 6.74 (d, *J* = 3.6 Hz, 1H), 5.62 (s, 2H), 3.55-3.45 (m, 2H), 0.82-0.78 (m, 2H), -0.11 (s, 9H).

**Step 2**

**14b** (1.50 g, 4.57 mmol), ethyl carbamate (0.81 g, 9.15 mmol), potassium carbonate (1.89 g, 13.71 mmol), 4,5-bis(diphenylphosphino)-9,9-dimethyloxanthene (0.53 g, 0.91 mmol) and palladium acetate (0.10 g, 0.46 mmol) were added sequentially to dioxane (30 mL) under $N_2$ atmosphere, the mixture was stirred at 115°C for 3 h. The reaction solution was cooled down to room temperature and filtered through a pad of celite and the filtrate was concentrated under reduced pressure to obtain the residue, which was purified by column chromatography (ethyl acetate: hexanes = 0-100%) to give **14c** (0.90 g), yield: 58%.

$^1H$ NMR (400 MHz, DMSO-$d_6$) δ 10.17 (s, 1H), 8.74 (s, 1H), 7.97 (d, *J* = 3.6 Hz, 1H), 6.56 (d, *J* = 3.6 Hz, 1H), 5.59 (s, 2H), 4.16 (q, *J* = 6.4 Hz, 2H), 3.50 (t, *J* = 8.0 Hz, 2H), 1.25 (t, *J* = 7.2 Hz, 3H), 0.81 (d, *J* = 8.0, 2H), -0.11 (s, 9H).

**Step 3**

**14c** (900 mg, 2.68 mmol) was dissolved in *N*,*N*-dimethylacetamide (10 mL), lithium tert-butoxide (214 mg, 2.68 mmol) was added to the above solution at 0°C under $N_2$ atmosphere. After stirring for 0.5 h at 0°C, the reaction solution was cooled down to -10°C followed by dropwise addition a solution of (3R,4S)-3-(2-bromoacetyl)-4-ethyl-1-pyrrolidinecarboxylic acid phenylmethyl ester (948 mg, 2.68 mmol) in *N*,*N*-dimethylacetamide (5 mL), the mixture was stirred at -10°C for 0.5 h. Water (100 mL) was added to quench the reaction, the mixture was extracted with ethyl acetate (100 mL × 3)and the organic phase was washed with brine (100 mL × 1), dried over anhydrous sodium sulfate, filtered and concentrated under reduced pressure to obtain the residue, which was purified by column chromatography (ethyl acetate: hexanes = 0-100%) to give**14d** (1.20 g), yield: 75%.

$^1H$ NMR (400 MHz, DMSO-$d_6$) δ 8.61 (s, 1H), 7.99 (dd, *J* = 6.4, 3.6 Hz, 1H), 7.36-7.29 (m, 5H), 6.52 (dd, *J* = 30.4, 3.6 Hz, 1H), 5.61 (s, 2H), 5.06-5.05 (m, 2H), 4.84 (s, 2H), 4.18-4.12 (m, 2H), 3.59-3.42 (m, 6H), 3.20-3.16 (m, 1H), 2.46-2.38 (m, 1H), 1.47-1.39 (m, 1H), 1.20-1.15 (m, 3H), 0.91-0.85 (m, 4H), 0.81 (t, *J* = 8.0 Hz, 2H), -0.11 (s, 9H).

MS-ESI calcd.for $[M+H]^+$610, found 610.

**Step 4**

**14c** (1.10 g, 1.81 mmol), trifluoroacetic anhydride (1.89 g, 9.03 mmol) and pyridine (0.43 g, 5.43 mmol) weredissolved in acetonitrile (20 mL) under $N_2$ atmosphere, and the resultant solution was stirred at 75°C for 2 h. The reaction solution was diluted with water (100 mL), extracted with ethyl acetate (100 mL × 3) and the organic phase was washed with brine (100 mL × 1), dried over anhydrous sodium sulfate, filtered and concentrated under reduced pressure to obtain the residue, which was purified by column chromatography (ethyl acetate: hexanes = 0-100%) to give **14e** (0.61 g), yield: 65%.

$^1H$ NMR (400 MHz, DMSO-$d_6$) δ 8.66 (s, 1H), 7.67 (d, *J* = 3.6 Hz, 1H), 7.61 (d, *J* = 4.8 Hz, 1H), 7.41-7.31 (m, 5H), 7.09 (t, *J* = 4.0 Hz, 1H), 5.67 (s, 2H), 5.18-5.09 (m, 2H), 4.41-4.33 (m, 1H), 3.93-3.710 (m, 3H), 3.55 (t, *J*= 8.0 Hz, 2H), 3.31-3.22 (m, 1H), 2.58-2.53 (m, 1H), 1.08-0.99 (m, 1H), 0.89-0.78 (m, 3H), 0.61-0.57 (m, 3H), -0.13 (s, 9H).

MS-ESI calcd.for $[M+H]^+$520, found 520.

**Step 5**

**14e** (560 mg, 1.08 mmol) was dissolved in dichloromethane (20 mL), trifluoroacetic acid (4 mL) was added to the above solution, the mixture was stirred at room temperature for 20 h. The reaction solution was concentrated under reduced pressure to obtain **14f** (660 mg), yield: 99%.

MS-ESI calcd.for $[M+H]^+$420, found 420.

**Step 6**

4-ethyloctanoic acid (371 mg, 2.16 mmol), 1-(3-dimethylaminopropyl)-3-ethylcarbodiimide hydrochloride (412 mg, 2.16 mmol) and 4-dimethylaminopyridine (695 mg, 5.40 mmol) were dissolved in dichloromethane (20 mL). After stirring for 10 min at room temperature, **14f** (660 mg, 1.08 mmol) was added to the resultant solution, the mixture was stirred at room temperature for 2 h. The reaction solution was concentrated under reduced pressure to obtain the residue, which was purified by column chromatography (ethyl acetate: hexanes = 0-100%) to give **14g** (502 mg), yield: 81%.

$^1H$ NMR (400 MHz, DMSO-$d_6$) δ 8.69 (s, 1H), 7.65-7.62 (m, 2H), 7.42-7.31 (m, 5H), 7.13 (t, *J* = 4.0 Hz, 1H), 6.29 (s, 2H), 5.18-5.08 (m, 2H), 4.39-4.31 (m, 1H), 3.91-3.71 (m, 3H), 3.33-3.28 (m, 1H), 2.58-2.53 (m, 1H), 2.29 (t, *J* = 7.6 Hz, 2H), 1.42 (q, *J* = 6.8 Hz, 2H), 1.13-1.08 (m, 4H), 1.06-1.01 (m, 5H), 0.90-0.81 (m, 2H), 0.76-0.66 (m, 6H), 0.64-0.58 (m, 3H).

MS-ESI calcd.for $[M+H]^+$574, found 574.

**Step 7**

**14g** (502 mg, 0.88 mmol) was dissolved in tetrahydrofuran (20 mL), 10% wet palladium carbon (500 mg) was added to the above solution, and the reaction was evacuated and backfilled with $H_2$ three times, stirred at room temperature for 20 h. The reaction solution was filtered through a pad of celite and the filtrate was concentrated under reduced pressure to obtain the residue, which was purified by column chromatography (ethyl acetate: hexanes = 0-100%) to give **14h** (213 mg), yield: 55%.

$^1$H NMR (400 MHz, DMSO-$d_6$) δ 8.72 (s, 1H), 7.87 (s, 1H), 7.68 (d, *J* = 3.6 Hz, 1H), 7.14 (d, *J* = 3.6 Hz, 1H), 6.31 (s, 2H), 4.37 (q, *J* = 7.6 Hz, 1H), 3.69-3.65 (m, 1H), 3.55-3.54 (m, 2H), 3.05-3.00 (m, 1H), 2.62-2.56 (m, 1H), 2.31 (t, *J* = 7.6 Hz, 2H), 1.45 (q, *J* = 6.4 Hz, 2H), 1.16-1.00 (m, 9H), 0.96-0.85 (m, 2H), 0.77 (q, *J* = 7.2 Hz, 3H), 0.70 (q, *J* = 7.2 Hz, 3H), 0.62 (t, *J* = 7.2 Hz, 3H).

MS-ESI calcd.for $[M+H]^+$440, found 440.

**Step 8**

*N*,*N*-carbonyldiimidazole (116 mg, 0.70 mmol), triethylamine (70 mg, 0.70 mmol) and trifluoroethylamine (58 mg, 0.58 mmol) were dissolved in dichloromethane (3 mL) under $N_2$ atmosphere, after stirring for 0.5 h at room temperature, a solution of **14h** (170 mg, 0.39 mmol) dissolved in dichloromethane (2 mL) was added dropwiseto the above solution, the mixture was stirred at room temperature for 3 h. The reaction solution was concentrated under reduced pressure to obtain the residue, which was purified by column chromatography (ethyl acetate: hexanes = 0-100%) to give **14** (182 mg), yield: 83%.

$^1$H NMR (400 MHz, DMSO-$d_6$) δ 8.69 (s, 1H), 7.63 (d, *J* = 3.6 Hz, 1H), 7.53 (s, 1H), 7.15 (d, *J* = 3.6 Hz, 1H), 6.97 (t, *J* = 6.4 Hz, 1H), 6.29 (s, 2H), 4.34 (q, *J* = 6.4 Hz, 1H), 3.88-3.66 (m, 5H), 3.28-3.24 (m, 1H), 2.58-2.54 (m, 1H), 2.29 (t, *J* = 7.6 Hz, 2H), 1.43 (q, *J* = 6.4 Hz, 2H), 1.14-1.03 (m, 9H), 0.86-0.61 (m, 11H).

MS-ESI calcd.for $[M+H]^+$565, found 565.

**Example 15**

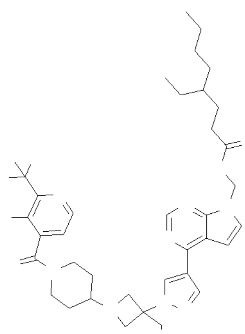

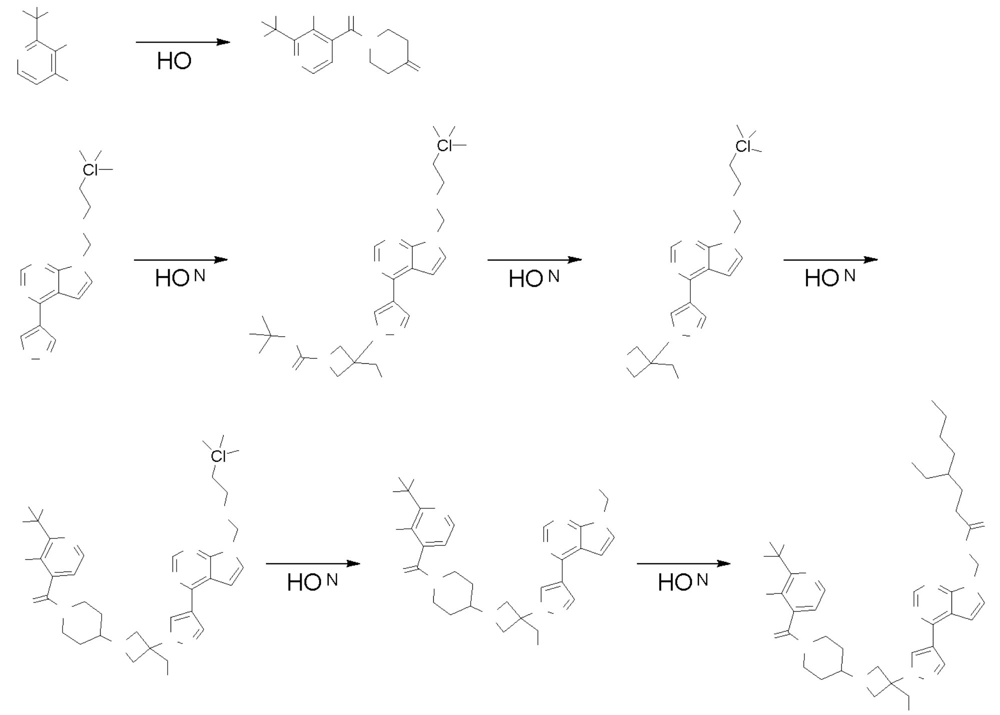


**Step 1**

**15a** (0.45 g, 2.15 mmol), HATU (1.23 g, 3.23 mmol), *N,N*-diisopropylethylamine (0.82 g, 6.45 mmol) and 4-oxopiperidinium chloride (0.44 g, 3.23 mmol) were dissolved in *N,N*-dimethylacetamide (5 mL) under $N_2$ atmosphere, the mixture was stirred at room temperature for 1 h. The reaction solution was diluted with water (50 mL), extracted with ethyl acetate (50 mL × 3) and the organic phase was washed with brine (50 mL × 1), dried over anhydrous sodium sulfate, filtered and concentrated under reduced pressure to obtain the residue, which was purified by column chromatography (ethyl acetate: hexanes = 0-100%) to give **15b** (0.60 g), yield: 96%.

$^1$H NMR (400 MHz, DMSO-$d_6$) δ 8.71 (d, *J* = 4.8 Hz, 1H), 8.01 (t, *J* = 4.8 Hz, 1H), 3.94 (s, 2H), 3.58 (t, *J* = 6.4 Hz, 2H), 2.55-2.52 (m, 2H), 2.38-2.36 (m, 2H).

MS-ESI calcd.for $[M+H]^+$291, found 291.

**Step 2**

**15c** (1.00 g, 3.17 mmol) and tert-butyl 3-(cyanomethylene)azetidine-1-carboxylate (0.74 g, 3.80 mmol) were dissolved in acetonitrile (20 mL) under $N_2$ atmosphere, 1,8-diazabicyclo[5.4.0]undec-7-ene (0.58 g, 3.80 mmol) was added dropwise to the above solution, the mixture was stirred at room temperature for 3 h. The reaction solution was concentrated under reduced pressure to obtain the residue, which was purified by column chromatography (ethyl acetate: hexanes = 0-100%) to give **15d** (1.38 g), yield: 86%.

$^1$H NMR (400 MHz, DMSO-$d_6$) δ 8.93 (s, 1H), 8.78 (s, 1H), 8.47 (s, 1H), 7.80 (d, *J* = 4.0 Hz, 1H), 7.20 (d, *J* = 3.6 Hz, 1H), 5.64 (s, 2H), 4.51 (d, *J* = 9.6 Hz, 2H), 4.22 (d, *J* = 9.6 Hz, 2H), 3.67 (s, 2H), 3.53 (t, *J* = 7.6 Hz, 2H), 1.41 (s, 9H), 0.82 (d, *J* = 8.0 Hz, 2H), - 0.11 (s, 9H).

MS-ESI calcd.for $[M+H]^+$510, found 510.

**Step 3**

**15d** (1.38 g, 2.71 mmol) was dissolved in hydrogen chloride dioxane solution (4.02 M, 20 mL), the mixture was stirred at room temperature for 1 h. The reaction solution was concentrated under reduced pressure to obtain **15e** (1.30 g), yield: 99%.

$^1$H NMR (400 MHz, DMSO-$d_6$) δ 10.06 (s, 1H), 9.83 (s, 1H), 9.32 (s, 1H), 8.95 (s, 1H), 8.75 (s, 1H), 8.03 (d, *J* = 4.0 Hz, 1H), 7.44 (d, *J* = 4.0 Hz, 1H), 5.70 (s, 2H), 4.74-4.68 (m, 2H), 4.39-4.37 (m, 2H), 3.94 (s, 2H), 3.55 (t, *J* = 7.6 Hz, 2H), 0.84 (t, *J* = 8.0 Hz, 2H), -0.09 (s, 9H).

MS-ESI calcd.for $[M+H]^+$410, found 410.

**Step 4**

**15e** (130 mg, 0.26 mmol), triethylamine (52 mg, 0.52 mmol) and **15b** (113 mg, 0.39 mmol) were dissolved in dichloromethane (5 mL) under $N_2$ atmosphere, after stirring for 5 min at room temperature, sodium triacetoxyborohydride (83 mg, 0.39 mmol) was addedto the above solution, the mixture was stirred at room temperature for 1 h. The reaction solution was diluted with the saturated aqueous sodium bicarbonate (25 mL), extracted with ethyl acetate (25 mL x 3) and the organic phase was washed with brine (25 mL × 1), dried over anhydrous sodium sulfate, filtered and concentrated under reduced pressure to obtain the residue, which was purified by column chromatography (ethyl acetate: hexanes = 0-100%) to give **15f** (100 mg), yield: 56%.

$^1$H NMR (400 MHz, DMSO-$d_6$) δ 8.85 (s, 1H), 8.77 (s, 1H), 8.67 (d, *J* = 4.8 Hz, 1H), 8.44 (s, 1H), 7.91 (t, *J* = 4.8 Hz, 1H), 7.80 (d, *J* = 3.6 Hz, 1H), 7.18 (d, *J* = 3.6 Hz, 1H), 5.64 (s, 2H), 4.12-4.07 (m, 1H), 3.76 (d, *J*= 8.0 Hz, 2H), 3.61-3.51 (m, 6H), 3.11-3.05 (m, 1H), 2.59-2.54 (m, 1H), 1.80-1.60 (m, 2H), 1.34-1.23 (m, 4H), 0.83 (t, *J* = 8.0 Hz, 2H), - 0.11 (s, 9H).

MS-ESI calcd.for $[M+H]^+$684, found 684.

**Step 5**

**15f** (95 mg, 0.14 mmol) was dissolved in dichloromethane (5 mL), trifluoroacetic acid (1 mL) was added to the above solution, the mixture was stirred at room temperature for 24 h. The reaction solution was concentrated under reduced pressure to obtain **15g** (115 mg), yield: 99%.

MS-ESI calcd.for $[M+H]^+$584, found 584.

**Step 6**

4-ethyloctanoic acid (46 mg, 0.27 mmol), 1-(3-dimethylaminopropyl)-3-ethylcarbodiimide hydrochloride (52 mg, 0.27 mmol) and 4-dimethylaminopyridine (83 mg, 0.68 mmol) were dissolved in dichloromethane (5 mL). After stirring for 10 min at room temperature, **15g** (660 mg, 1.08 mmol) was added to the above solution, the mixture was stirred at room temperature for 1 h. The reaction solution was concentrated under reduced pressure to obtain the residue, which was purified by column chromatography (ethyl acetate: hexanes = 0-100%) to give **15** (65 mg), yield: 73%.

$^1$H NMR (400 MHz, DMSO-$d_6$) δ 8.86 (s, 1H), 8.80 (s, 1H), 8.67 (d, *J* = 4.4 Hz, 1H), 8.45 (s, 1H), 7.91 (t, *J* = 4.8 Hz, 1H), 7.75 (d, *J* = 3.6 Hz, 1H), 7.20 (d, *J* = 3.6 Hz, 1H), 6.24 (s, 2H), 4.10-4.07 (m, 1H), 3.75 (d, *J* = 8.0 Hz, 2H), 3.60-3.56 (m, 4H), 3.44-3.40 (m, 1H), 3.28-3.21 (m, 1H), 3.11-3.05 (m, 1H), 2.61-2.53 (m, 1H), 2.29 (t, *J* = 7.6 Hz, 1H), 1.80-1.58 (m, 2H), 1.44 (q, *J* = 4.8 Hz, 2H), 1.23-1.09 (m, 11H), 0.77 (t, *J* = 7.0 Hz, 3H), 0.70 (t, *J* = 7.2 Hz, 3H).

MS-ESI calcd.for $[M+H]^+$738, found 738.

**Example 16**

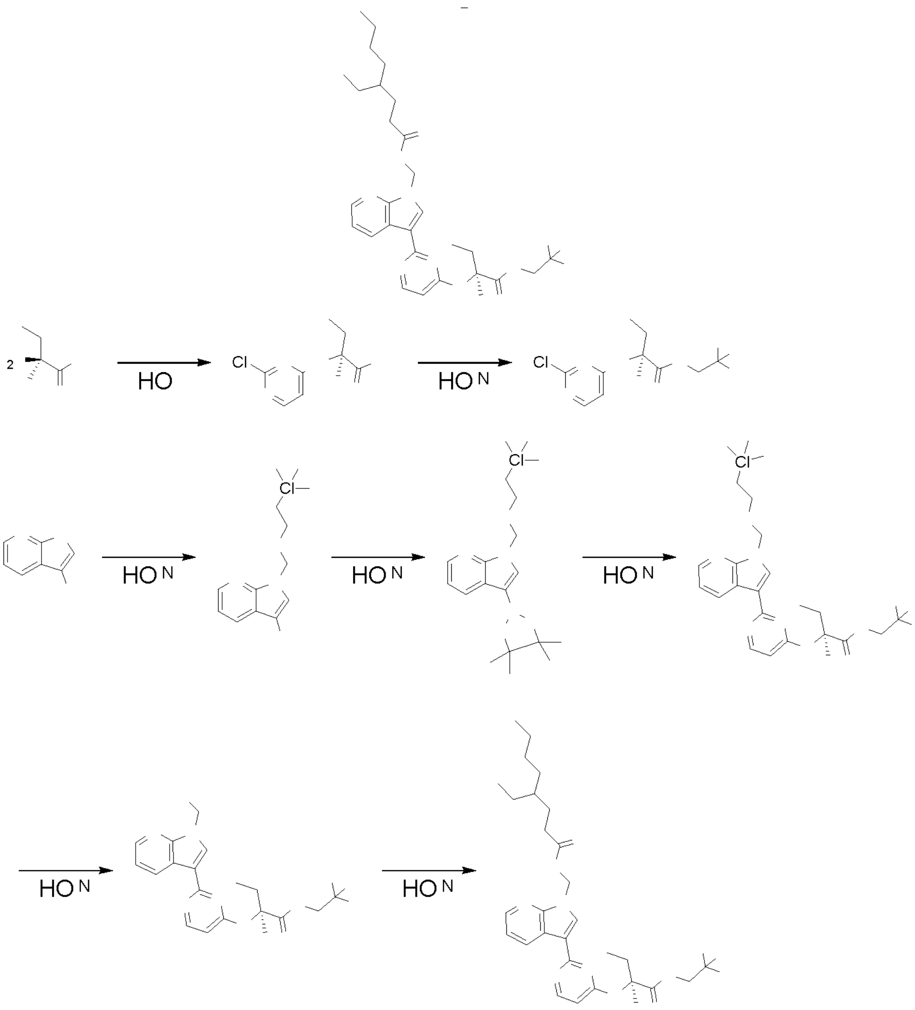


**Step 1**

**16a** (1.00 g, 8.55 mmol), 2,4-dichloropyrimidine (1.27 g, 8.55 mmol) and potassium carbonate (1.75 g, 12.83 mmol) were dissolved in isopropanol (30 mL), the mixture was stirred at 100°C for 8 h. The reaction solution was cooled down to room temperature and concentrated under reduced pressure to obtain the residue, which was purified by column chromatography (methanol : dichloromethane = 0-100%) to give **16b** (1.50 g), yield: 79%.

$^1$H NMR (400 MHz, DMSO-$d_6$) $\delta$ 12.51 (s, 1H), 7.98 (s, 1H), 7.94 (d, *J* = 6.0 Hz, 1H), 6.60 (d, *J* = 6.0 Hz, 1H), 2.03-1.93 (m, 1H), 1.89-1.80 (m, 1H), 1.44 (s, 3H), 0.82 (t, *J* = 7.4 Hz, 3H).

MS-ESI calcd.for $[M+H]^+$230, found 230.

**Step 2**

**16b** (1.00 g, 4.37 mmol), 2,2,2-trifluoroethylamine (0.65 g, 6.55 mmol) and *N,N*-diisopropylethylamine (0.84 g, 6.55 mmol) were dissolved in *N*,*N*-dimethylformamide (30 mL) under $N_2$ atmosphere, the reaction solution was cooled down to 0°C and 50% propylphosphonic anhydride (50% w/w, ethyl acetate solution, 4.20 g, 6.55 mmol) was added dropwise to the resultant solution and the mixture was slowly warmed up to room temperature and continued to stir for 17 h. The reaction solution was diluted with water (100 mL), extracted with ethyl acetate (100 mL × 3), the organic phase was washed with brine (100 mL × 1), dried over anhydrous sodium sulfate, filtered and concentrated under reduced pressure to obtain the residue, which was purified by column chromatography (ethyl acetate: hexanes = 0-100%) to give **16c** (0.50 g), yield: 37%.

$^1$H NMR (400 MHz, DMSO-$d_6$) $\delta$ 8.23 (s, 1H), 7.95 (d, *J* = 4.8 Hz, 1H), 7.81 (s, 1H), 6.61-6.59 (m, 1H), 3.84-3.76 (m, 2H), 1.97-1.89 (m, 1H), 1.87-1.78 (m, 1H), 1.42 (s, 3H), 0.76 (t, *J* = 7.6 Hz, 3H).

MS-ESI calcd.for $[M+H]^+$311, found 311.

**Step 3**

**16d** (1.00 g, 5.07 mmol) and *N,N*-diisopropylethylamine (0.98 g, 7.61 mmol) were dissolved in dichloromethane (20 mL) under $N_2$ atmosphere, 2-(trimethylsilyl)ethoxymethyl chloride (1.26 g, 7.61 mmol) was added dropwise to the resultant solution and the reaction mixture was stirred at room temperature for 3 h. The reaction solution was concentrated under reduced pressure to obtain the residue, which was purified by column chromatography (ethyl acetate: hexanes = 0-100%) to give **16e** (1.30 g), yield: 77%.

$^1H$ NMR (400 MHz, DMSO-$d_6$) δ 8.36 (dd, *J* = 4.8, 1.6 Hz, 1H), 7.92 (s, 1H), 7.89 (dd, *J* = 8.0, 1.6 Hz, 1H), 7.26 (dd, *J* = 8.0, 4.8 Hz, 1H), 5.62 (s, 2H), 3.50 (t, *J*= 8.0 Hz, 2H), 0.80 (t, *J* = 8.0 Hz, 2H), -0.12 (s, 9H).

MS-ESI calcd.for $[M+H]^+$327, found 327.

**Step 4**

**16e** (1.05 g, 3.22 mmol), bis(pinacolato)diboron (2.45 g, 9.66 mmol), potassium acetate (0.95 g, 9.66 mmol) and chloro(2-dicyclohexylphosphino-2',4',6'-triisopropyl-1,1'-biphenyl)[2-(2'-amino-1,1'-biphenyl)]palladium(II) (0.25 g, 0.32 mmol) were added sequentially to dioxane (20 mL) under $N_2$ atmosphere, the mixture was stirred at 100°C for 2 h. The reaction solution was cooled to room temperature and filtered through a pad of celite and the filtrate was concentrated under reduced pressure to obtain the residue, which was purified by column chromatography (ethyl acetate: hexanes = 0-100%) to give **16f** (0.80 g), yield: 67%.

$^1H$ NMR (400 MHz, DMSO-$d_6$) δ 8.29 (dd, *J* = 4.8, 1.6 Hz, 1H), 8.11 (dd, *J* = 8.0, 1.6 Hz, 1H), 7.98 (s, 1H), 7.20 (dd, *J* = 8.0, 4.8 Hz, 1H), 5.63 (s, 2H), 3.51 (t, *J* = 8.0 Hz, 2H), 1.31 (s, 12H), 0.80 (t, *J* = 8.0 Hz, 2H), -0.11 (s, 9H).

MS-ESI calcd.for $[M+H]^+$375, found 375.

**Step 5**

**16f** (675 mg, 1.80 mmol), **16c** (700 mg, 2.26 mmol), potassium carbonate (623 mg, 4.56 mmol) and chloro(2-dicyclohexylphosphino-2',4',6'-triisopropyl-1,1'-biphenylyl)[2-(2'-amino-1,1'-biphenyl)]palladium(II) (177 mg, 0.23 mmol) were added sequentially to a mixture of dioxane (20 mL) and water (4 mL) under $N_2$ atmosphere, the mixture was stirred at 100°C for 2 h. The reaction solution was cooled to room temperature and filtered through a pad of celite and the filtrate was concentrated under reduced pressure to obtain the residue, which was purified by column chromatography (ethyl acetate: hexanes = 0-100%) to give **16g** (230 mg), yield: 24%.

$^1H$ NMR (400 MHz, DMSO-$d_6$) δ 8.75 (d, *J* = 8.0 Hz, 1H), 8.31-8.30 (m, 2H), 8.19 (s, 1H), 8.14 (d, *J* = 5.6 Hz, 1H), 7.36 (s, 1H), 7.22 (dd, *J* = 8.0, 4.8 Hz, 1H), 6.44 (s, 1H), 5.68 (s, 2H), 3.87-3.66 (m, 2H), 3.53 (t, *J* = 8.0 Hz, 2H), 2.12-2.02 (m, 1H), 1.89-1.79 (m, 1H), 1.49 (s, 3H), 0.85-0.80 (m, 5H), -0.11 (s, 9H).

MS-ESI calcd.for $[M+H]^+$523, found 523.

**Step 6**

**16g** (220 mg, 0.42 mmol) was dissolved in dichloromethane (20 mL), trifluoroacetic acid (4 mL) was added to the above solution, the mixture was stirred at room temperature for 16 h. The reaction solution was concentrated under reduced pressure to obtain **16h** (290 mg), yield: 99%.

MS-ESI calcd.for $[M+H]^+$423, found 423.

**Step 7**

4-ethyloctanoic acid (290 mg, 0.90 mmol), 1-(3-dimethylaminopropyl)-3-ethylcarbodiimide hydrochloride (172 mg, 0.90 mmol) and 4-dimethylaminopyridine (274 mg, 2.25 mmol) were dissolved in dichloromethane (15 mL). After stirring for 10 min at room temperature, **16h** (290 mg, 0.45 mmol) was added to the resultant solution, the mixture was stirred at room temperature for 2 h. The reaction solution was concentrated under reduced pressure to obtain the residue, which was purified by column chromatography (ethyl acetate: hexanes = 0-100%) to give **16** (139 mg), yield: 53%.

$^1H$ NMR (400 MHz, DMSO-$d_6$) δ 8.76 (d, *J* = 8.0 Hz, 1H), 8.33 (dd, *J* = 4.8, 1.6 Hz, 1H), 8.30 (s, 1H), 8.23 (s, 1H), 8.14 (d, *J* = 5.6 Hz, 1H), 7.42 (s, 1H), 7.26 (dd, *J* = 8.0, 4.8 Hz, 1H), 6.46 (s, 1H), 6.28 (s, 2H), 3.91-3.67 (m, 2H), 2.27 (t, *J* = 7.6 Hz, 2H), 2.11-2.00 (m, 1H), 1.89-1.80 (m, 1H), 1.49 (s, 3H), 1.13-1.07 (m, 9H), 0.86-0.75 (m, 8H), 0.68 (t, *J* = 7.2 Hz, 3H).

MS-ESI calcd.for $[M+H]^+$577, found 577.

**Example 17 Skin-Pampa assay**

1. Hydrate the Skin-Pampa membrane with hydration solution overnight.
2. Dissolve each compound to be tested in DMSO and prepare 0.5mL 20mM solutions of the test compounds.Take 50uL of solutions of the test compounds to the corresponding 5mL of donor solution and mix well.
3. Add 200 μL of the sample solutionto the donor (bottom) plateandadd 200 μL of acceptor solution into the wells of the acceptor (TOP )plate , and 12 wells are incubated in parallel for each sample.
4. After 7 hours of incubation, 100 μL of the sample in each of the donor plateand 100 μL of the sample in each of the acceptor plate were sampled and then added with 200 μL of dilution (50% acetonitrile aqueous solution), mixed by vortexing and centrifuged, 200 μL of the supernatant was used for HPLCcontent detection.
5. The Skin-Pampa parameters are calculated according to the following equation

$$R = 1 - \frac{C_D(t)}{C_D(0)} - \frac{V_A}{V_D}\frac{C_A(t)}{C_D(0)} \quad (1)$$

$$\log Pe = \log\left[-\frac{2.303 V_D}{A(t - t_{LAG})}\left(\frac{V_A}{V_A + V_D}\right)\log\left[1 - \left(\frac{V_A + V_D}{V_D(1 - R)}\right)\frac{C_A(t)}{C_D(0)}\right]\right] \quad (2)$$

Pe - effective permeability coefficient;
$V_A$ - the volume of acceptor well (ml);
$V_D$ - the volume of donorwell (ml);
A - the filtration area ($cm^2$);
t - the incubation time (s);
$t_{LAG}$ - the steady-state time (s);
$C_D(t)$ - the concentration of the compound in donorwell at t;
$C_A(t)$ - the concentration of the compound in acceptor well at t;
$C_D(0)$ - the concentration at the compound in donorwellat 0.

Table 6. Summary of Skin-Pampa assay results

| | Clog P | LogPe | Pe($10^{-6}$cm/s) | Multiplicity (relative to the prototype drug) |
|---|---|---|---|---|
| Piroxicam (positive control) | 1.89 | -5.73±0.06 | 1.84±0.24 | - |
| Tofacitinib | 1.55 | -6.45±0.14 | 0.36±0.12 | - |
| Example 1 | 4.44 | -5.80±0.11 | 1.57±0.50 | 4.31-4.46 |
| Example 4 | 5.37 | -5.50±0.17 | 3.13±1.15 | 8.25-8.92 |
| Example 5 | 6.56 | -5.56±0.16 | 2.78±1.13 | 6.88-8.15 |
| Example 7 | 4.97 | -5.53±0.08 | 2.98±0.56 | 7.38-10.08 |
| Example 8 | 5.50 | -5.66±0.08 | 2.19±0.35 | 5.29-7.67 |
| Itacitinib | 0.83 | -6.88±0.34 | 0.13±0.17 | - |
| Example 15 | 4.53 | -5.90±0.20 | 1.26±0.48 | 5.8-19.5 |
| Upadacitinib | 2.26 | -6.96±0.20 | 0.11±0.05 | - |
| Example 14 | 6.51 | -6.09±0.19 | 0.81±0.28 | 6.81-8.83 |
| Peficitinib | 1.82 | -7.06±0.33 | 0.11±0.06 | - |
| Example 13 | 5.80 | -6.01±0.19 | 1.07±0.39 | 8.59-15.6 |

Conclusion: The compounds synthesized by the invention has excellent transdermal properties in transdermal

experiments in rats and are suitable for the preparation of topical formulations.

All documents referred to in the present invention are incorporated by reference herein as if each document is individually incorporated by reference. Further, it should be understood that upon reading the above teaching of the present invention, various modifications or modifications may be made to the present invention by those skilled in the art, and those equivalents also fall within the scope defined by the appended claims of the present application.

## Claims

1. A prodrug molecule of a pharmaceutical compound G', and pharmaceutically acceptable salts, hydrates or solvates thereof, **characterized in that** the hydrophobicity coefficient CLogP of the drug molecule G' is less than 4; and the prodrug molecule has the structure shown in formula (I):

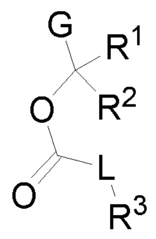

(I)

wherein, G is a partial structural fragment formed by losing H atoms in the drug molecule G', and connects to

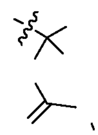

via any N, O or S atoms within the molecule;

$R^1$ and $R^2$ are each independently selected from the group consisting of H, D, substituted or unsubstituted C1-C6 alkyl, substituted or unsubstituted C1-C6 heteroalkyl, substituted or unsubstituted C3-C8 cycloalkyl, and substituted or unsubstituted 3-8 membered heterocyclyl, or $R^1$ and $R^2$, together with the carbon atoms connected thereto, form a C3-C8 carbocycle or heterocycle;

L is selected from the group consisting of chemical bond, substituted or unsubstituted C1-C6 alkylene, and substituted or unsubstituted C1-C6 heteroalkylene;

$R^3$ is selected from the group consisting of substituted or unsubstituted C1-C20 alkyl (linear or branched), substituted or unsubstituted C3-C20 cycloalkyl, substituted or unsubstituted C1-C20 heteroalkyl, substituted or unsubstituted 3-20 membered heterocyclyl, and substituted or unsubstituted C6-C14 aryl, or $R^3$ is connected with $R^1$ or $R^2$ so as to form a substituted or unsubstituted 5-20 membered lactone ring or heterolactone ring, wherein the heterolactone ring refers to the cyclic backbone of the lactone ring comprising 1-3 heteroatoms selected from the group consisting of N, O and $S(O)_p$;

wherein, the heteroalkyl refers to one or more carbon atoms on the carbon chain being replaced by heteroatoms selected from the group consisting of N, O and $S(O)_p$;

the heterocyclyl comprises 1-3 heteroatoms selected from the group consisting of N, O and $S(O)_p$;

p is selected from 0, 1 or 2;

unless otherwise specified, the "substituted" means being substituted by one or more (e.g. 2, 3, 4, etc.) substituents selected from the group consisting of deuterium, halogen, C1-C6 alkyl, halogenated C1- C6 alkyl, C1-C6 alkoxy, halogenated C1-C6 alkoxy, C3-C8 cycloalkyl, halogenated C3-C8 cycloalkyl, C3-C8 heterocyclyl, oxo, -CN, hydroxyl, amino, carboxyl, amide, sulfonamide, sulfonyl, and a group which is unsubstituted or substituted by one or more substituents, wherein the group is selected from C6-C10 aryl, halogenated C6-C10 aryl, 5-10 membered heteroaryl having 1-3 heteroatoms selected from N, S and O, halogenated 5-10 membered heterocyclyl having 1-3 heteroatoms selected from N, S and O; and the substituents selected from the group consisting of: halogen, C1-C6 alkyl, C1-C6 alkoxy, and =O.

2. The prodrug molecule of claim 1, or pharmaceutically acceptable salts, hydrates or solvates thereof, wherein, the prodrug molecule G' is selected from the group consisted of JAK inhibitors, MEK inhibitors, and BTK inhibitors.

3. The prodrug molecule of any one of claims 1-2, or pharmaceutically acceptable salts, hydrates or solvates thereof, wherein, the JAK inhibitor is selected from the group consisting of INCB-52793, ATI-502, deuterium-modified ruxolitinib analog, ATI-501, R-348, NS-018, Jaktinib, KL-130008, DTRMHS-07, WXSH-0150, TQ05105, WXFL10203614, and a molecule selected from the following group, or the pharmaceutically acceptable salts, hydrates, or solvates thereof:

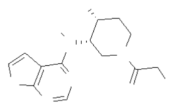
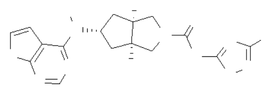
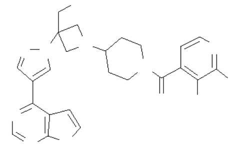
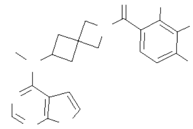
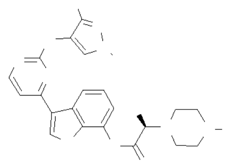
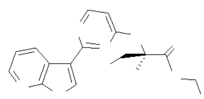
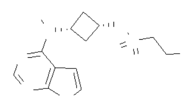
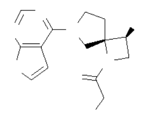
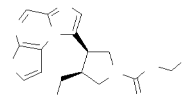
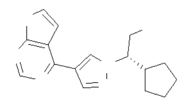
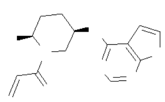
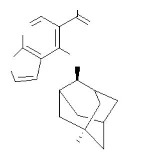
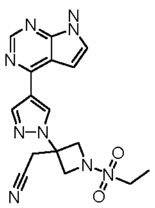
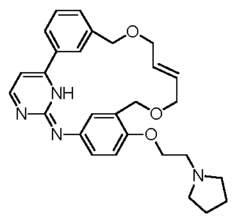
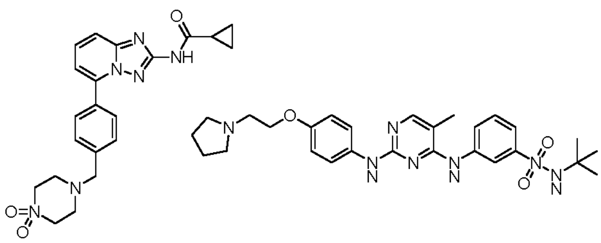
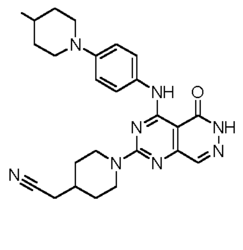
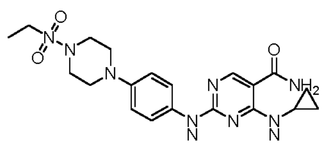
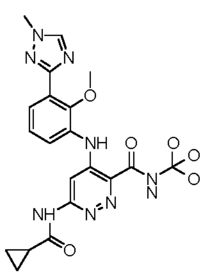
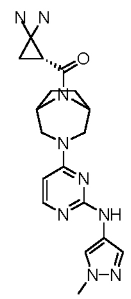
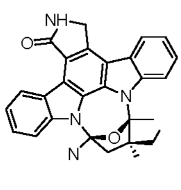
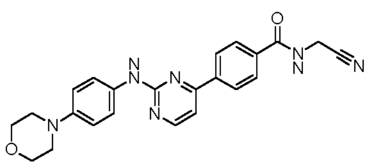
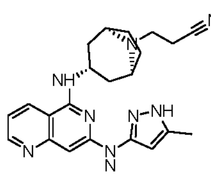
and
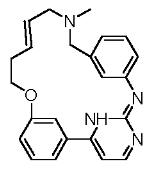

4. The prodrug molecule of any one of claims 1-2, or pharmaceutically acceptable salts, hydrates or solvates thereof, wherein, the MEK inhibitor is selected from the following group, or pharmaceutically acceptable salts, hydrates, or

solvates thereof:

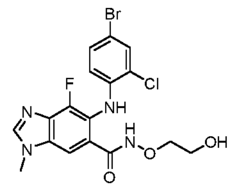
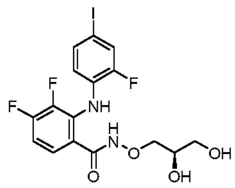
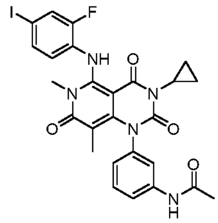
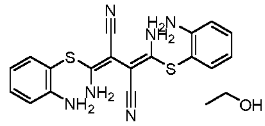

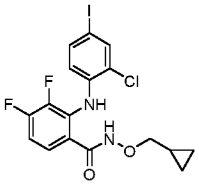
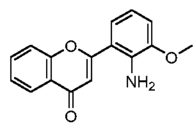
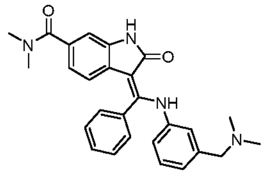
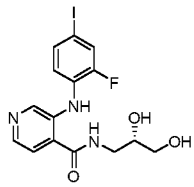

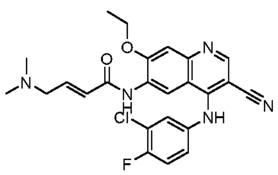
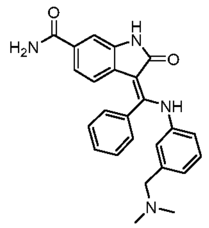
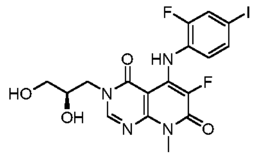
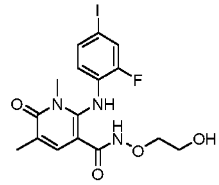

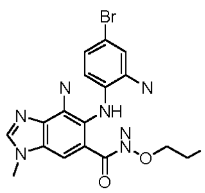
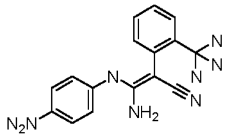
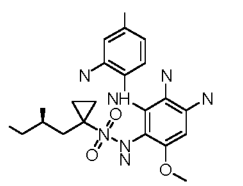
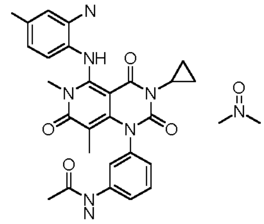

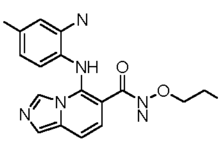
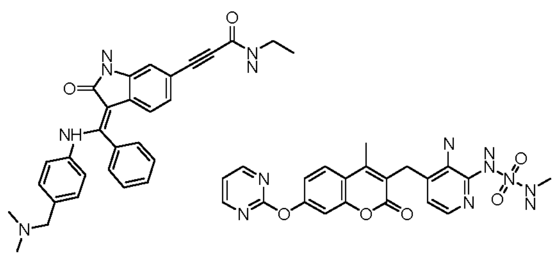
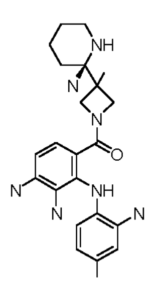

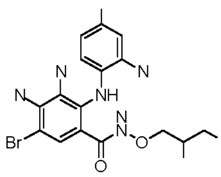
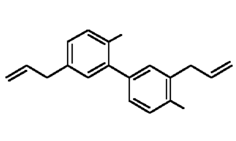
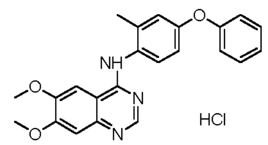
and
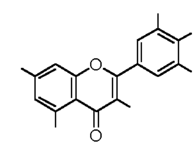
.

**5.** The prodrug molecule of any one of claims 1-2, or pharmaceutically acceptable salts, hydrates or solvates thereof, wherein, the BTK inhibitor is selected from the following group, or pharmaceutically acceptable salts, hydrates, or solvates thereof:

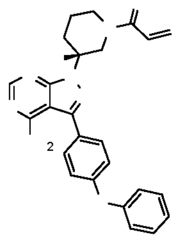
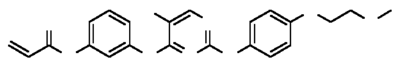
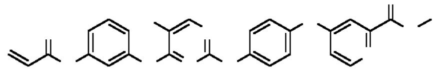

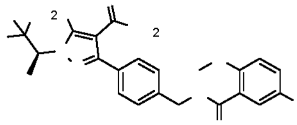
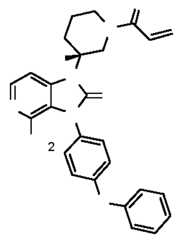
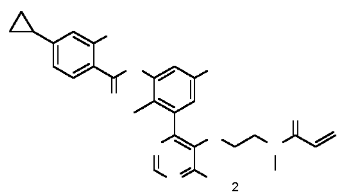
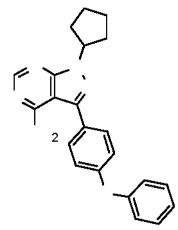

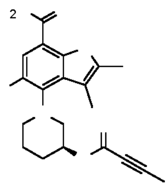
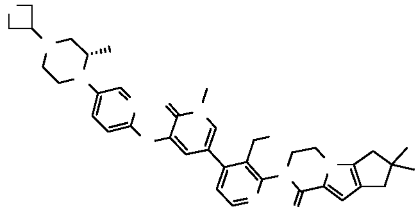
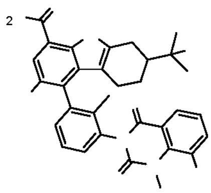
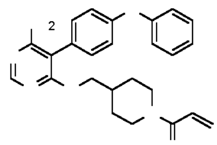

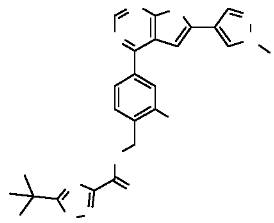
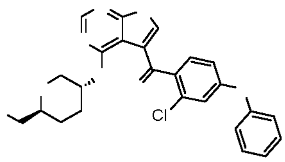
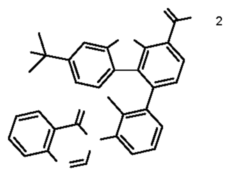
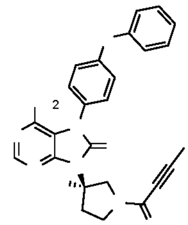

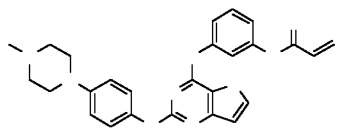
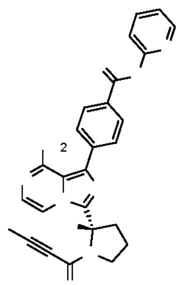
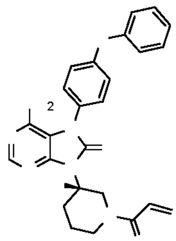
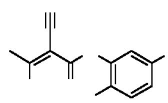

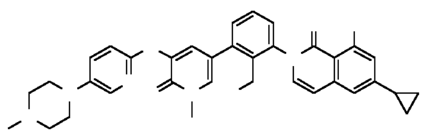
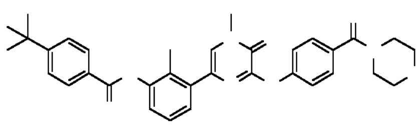

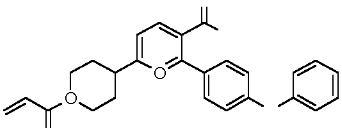

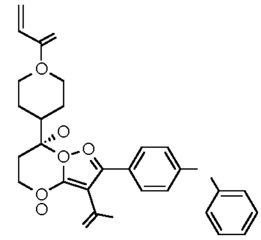

and

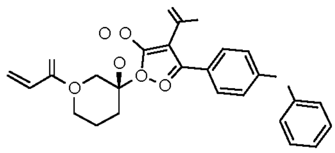

.

6. The prodrug molecule of any one of claims 1-5, or pharmaceutically acceptable salts, hydrates or solvates thereof, wherein, $R^1$ and $R^2$ are each independently H, D, or C1-C6 alkyl, L is chemical bond or C1-C6 alkylene, and $R^3$ is selected from the group consisting of C1-C20 alkyl, C3-C20 cycloalkyl, C6-C14 aryl; wherein the alkyl, alkylene, cycloalkyl, and aryl can be optionally substituted by substituents selected from the group consisting of halogen, and C1-C4 alkyl.

7. The prodrug molecule of any one of claims 1-6, or pharmaceutically acceptable salts, hydrates or solvates thereof, wherein, $R^1$ and $R^2$ are each independently H, L is chemical bond, and $R^3$ is selected from the group consisting of C1-C20 alkyl; wherein the alkyl can be optionally substituted by substituents selected from the group consisting of halogen, and C1-C4 alkyl.

8. The prodrug molecule of any one of claims 1-7, or pharmaceutically acceptable salts, hydrates or solvates thereof, wherein, G group is selected from the group consisting of:

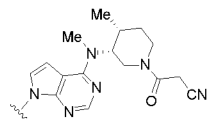

Tofacitinib

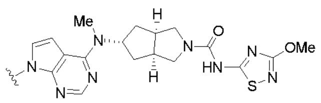

SHR-0302

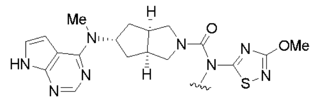

SHR-0302

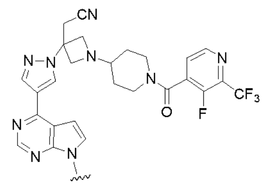

Itacitinib

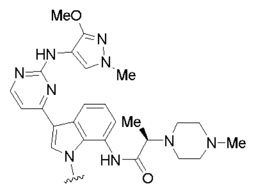

AZD-4205
2091134-68-6

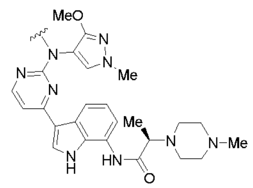

AZD-4205
2091134-68-6

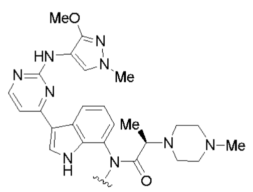

AZD-4205
2091134-68-6

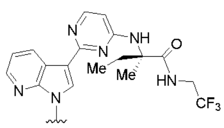

Decemotinib

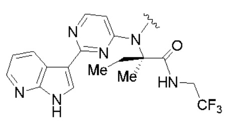

Decemotinib

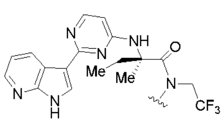

Decemotinib

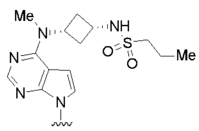

PF-04965842(Abrocitinib)

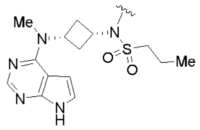

PF-04965842(Abrocitinib)

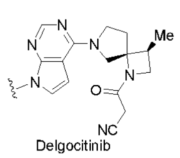

Delgocitinib

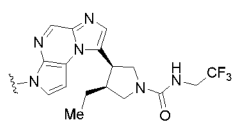

Upadacitinib

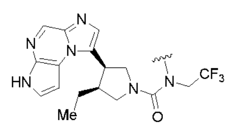

Upadacitinib

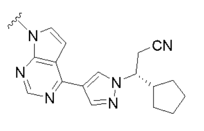

Ruxolitinib

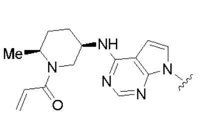

PF-06651600

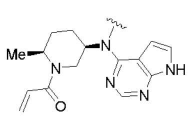

PF-06651600

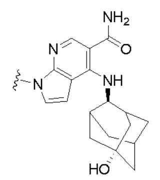

Peficitinib

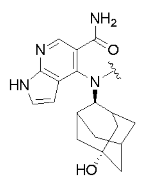

Peficitinib

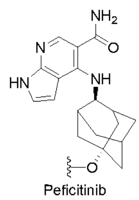
Peficitinib

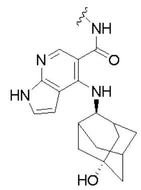
Peficitinib

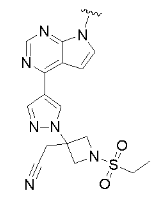
Baricitinib

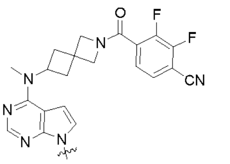

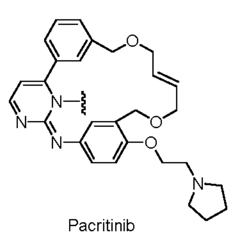
Pacritinib

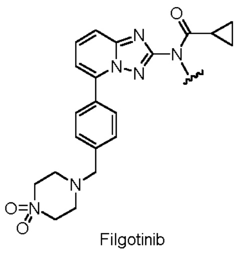
Filgotinib

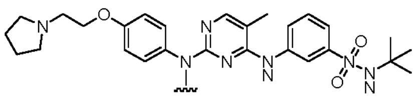
Fedratinib

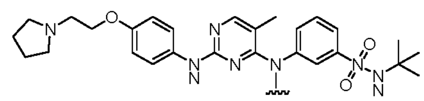
Fedratinib

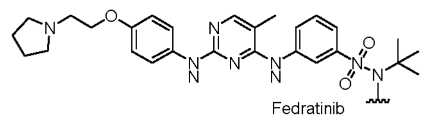
Fedratinib

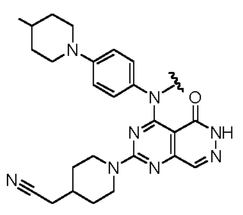

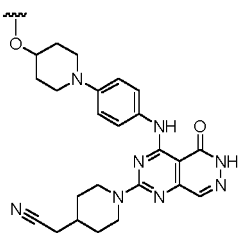

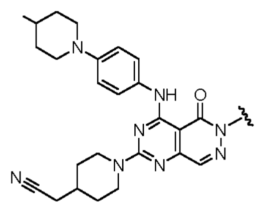

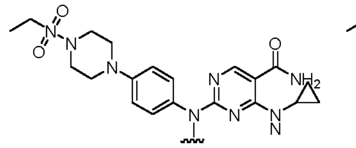
Cerdulatinib

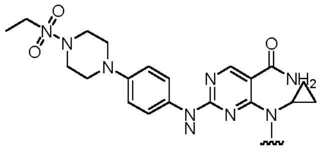
Cerdulatinib

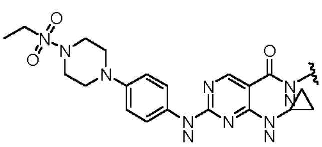
Cerdulatinib

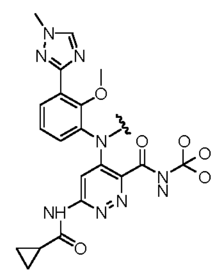

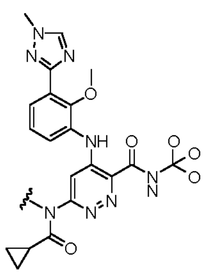

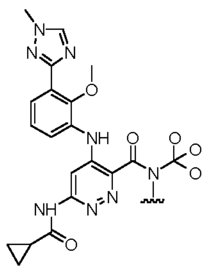

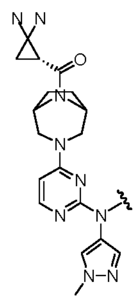

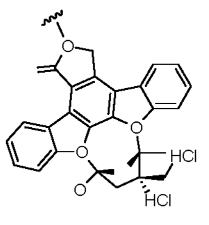

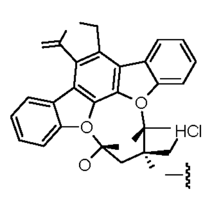

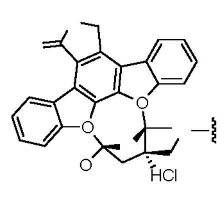

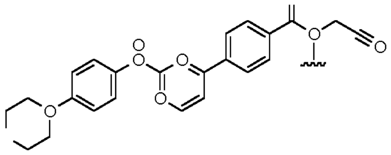
Momelotinib

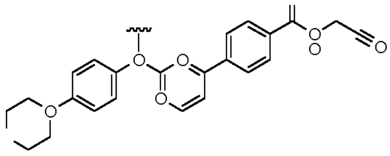
Momelotinib

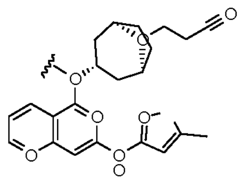

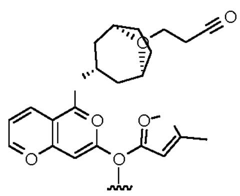

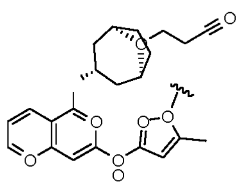

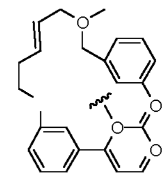

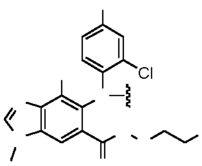
Selumetinib

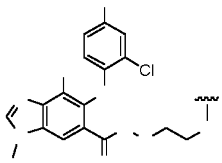
Selumetinib

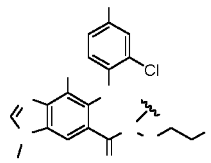
Selumetinib

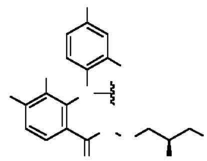
Mirdametinib

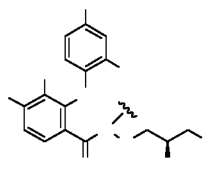
Mirdametinib

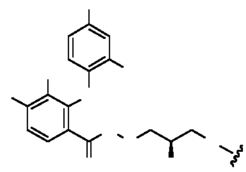
Mirdametinib

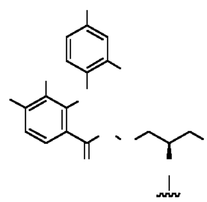
Mirdametinib

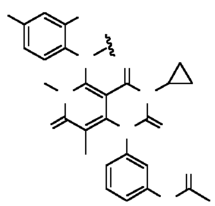

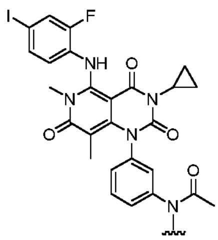
Trametinib (GSK1120212)

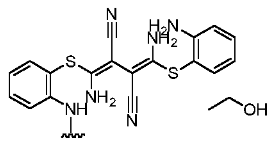
U0126

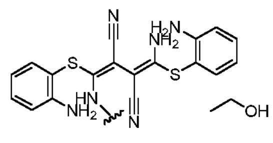
U0126

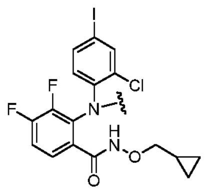
PD184352 (CI-1040)

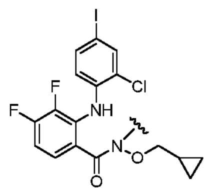
PD184352 (CI-1040)

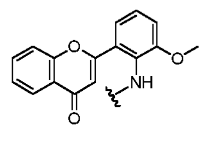
PD98059

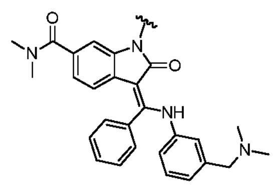
BIX 02189

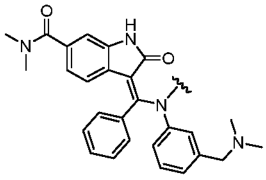
BIX 02189

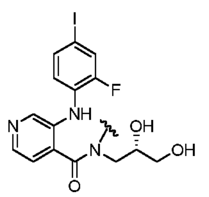
Pimasertib (AS-703026)

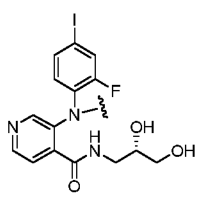
Pimasertib (AS-703026)

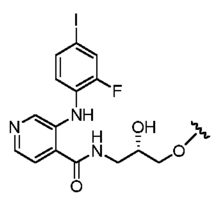
Pimasertib (AS-703026)

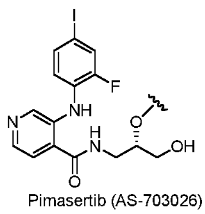
Pimasertib (AS-703026)

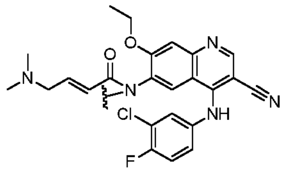
Pelitinib (EKB-569)

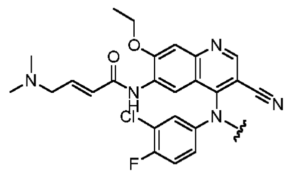
Pelitinib (EKB-569)

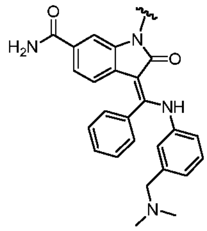
BIX 02188

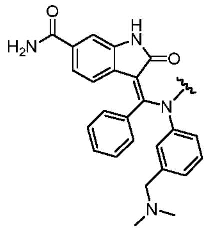
BIX 02188

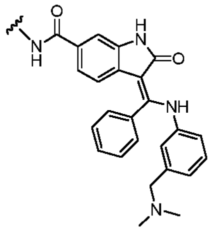
BIX 02188

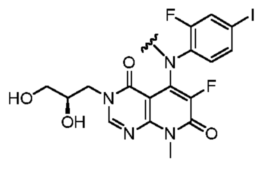
TAK-733

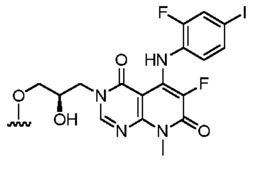
TAK-733

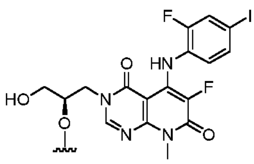
TAK-733

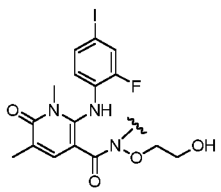
AZD8330

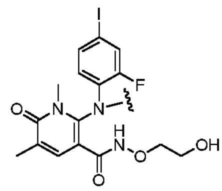
AZD8330

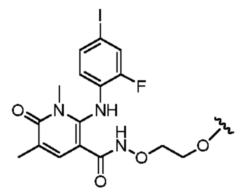
AZD8330

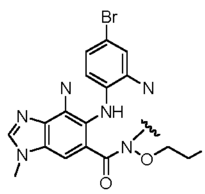
Binimetinib (MEK162)

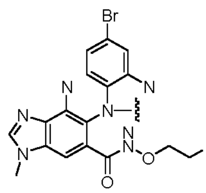
Binimetinib (MEK162)

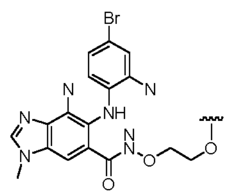
Binimetinib (MEK162)

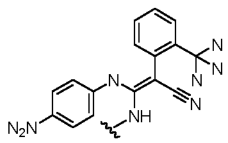

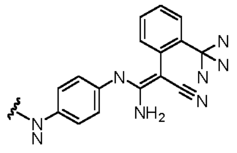

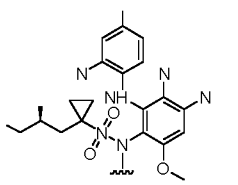
Cerdulatinib

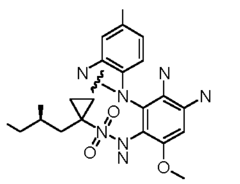
Cerdulatinib

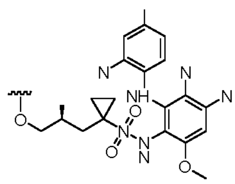
Cerdulatinib

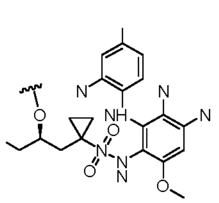
Cerdulatinib

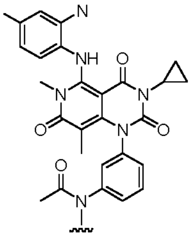

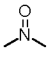

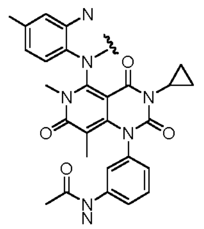

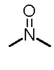

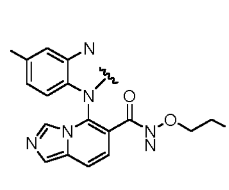
Binimetinib

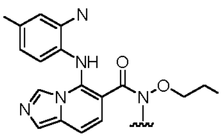
Binimetinib

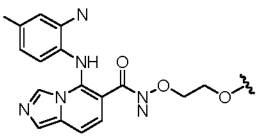
Binimetinib

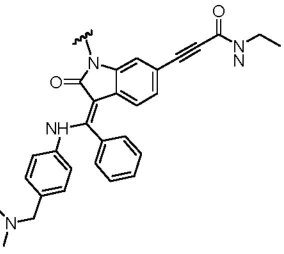

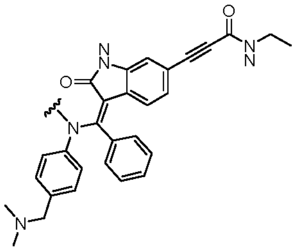

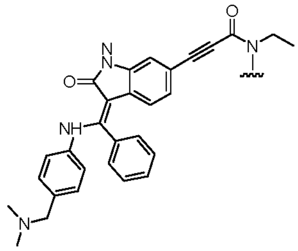

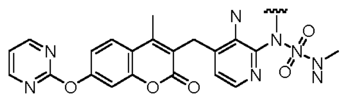
Cerdulatinib

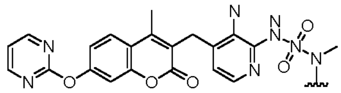
Cerdulatinib

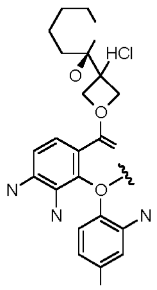
(GDC-0973)

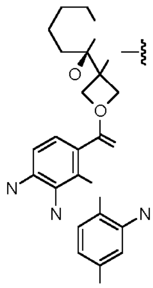
(GDC-0973)

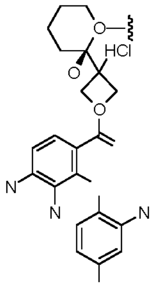
(GDC-0973)

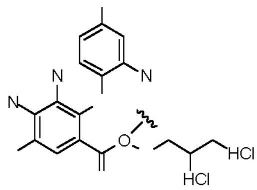

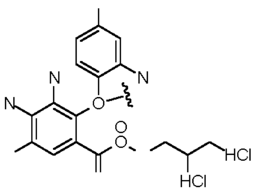

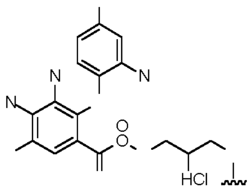

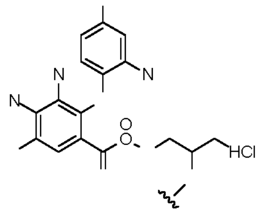

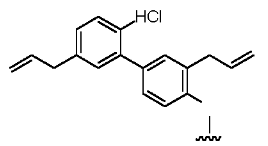
293100)

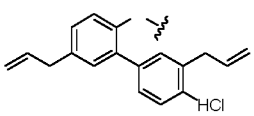
293100)

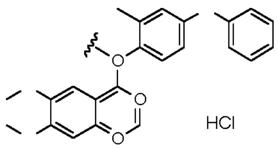
HCl

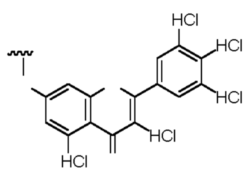
Myricetin

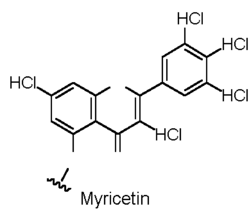
Myricetin

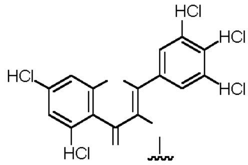
Myricetin

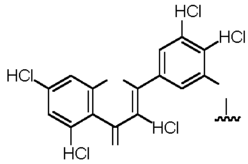
Myricetin

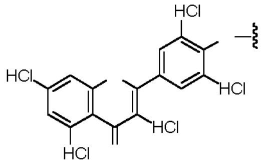
Myricetin

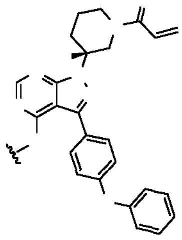

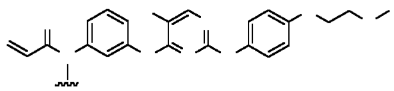

Filgotinib

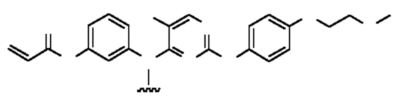

Filgotinib

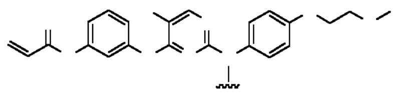

Filgotinib

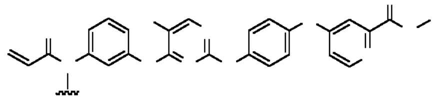

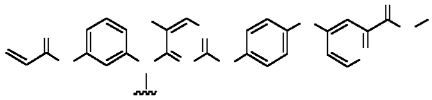

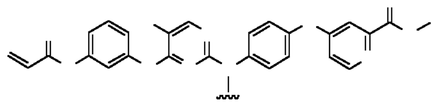

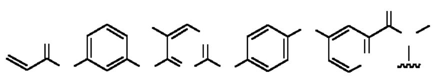

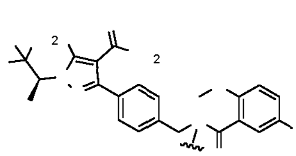

Selumetinib

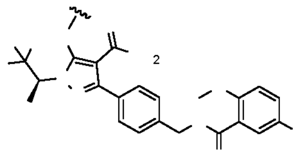

Pirtobrut

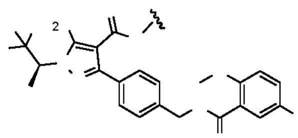

Selumetinib

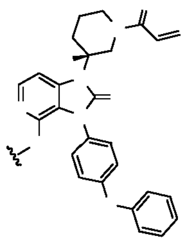

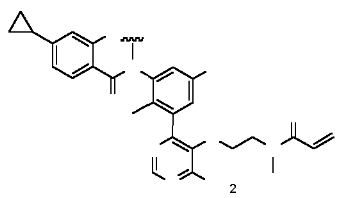

Remibrutinib
(LOU064)

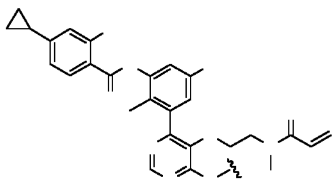

Remibrutinib (LOU064)

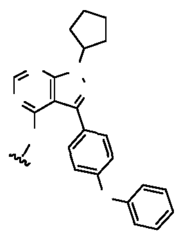

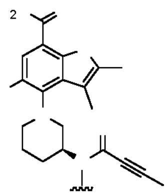

Branebrutinib (BMS-986195)

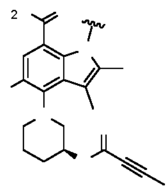

Branebrutinib (BMS-986195)

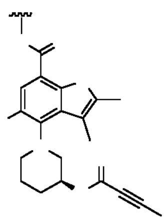

Branebrutinib (BMS-986195)

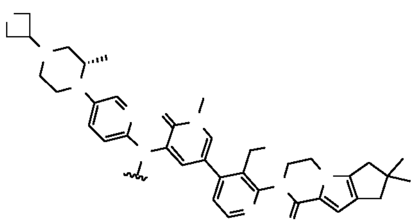

Remibrutinib

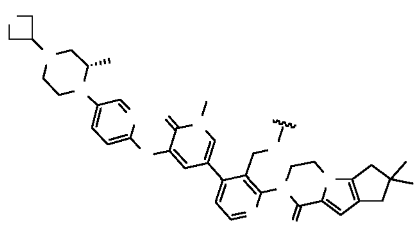

Remibrutinib

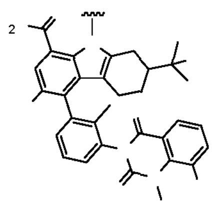
Mirdametinib

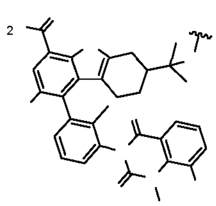
Mirdametinib

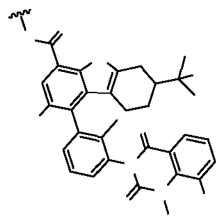
Mirdametinib

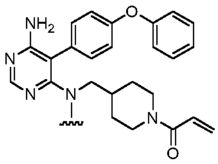
Evobrutinib (M-2951)

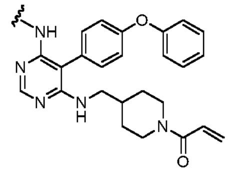
Evobrutinib (M-2951)

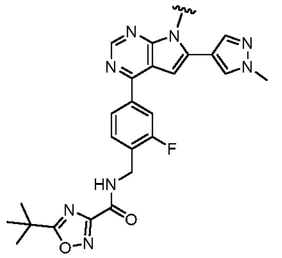
CAS: 2230724-66-8

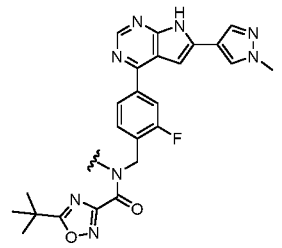
CAS: 2230724-66-8

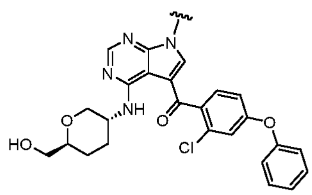
Nemtabrutinib (ARQ 531)

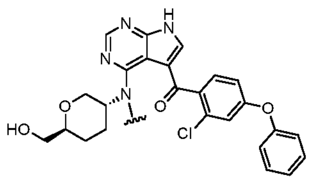
Nemtabrutinib (ARQ 531)

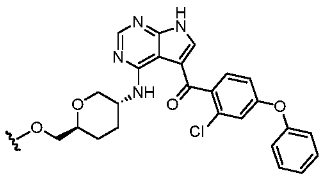
Nemtabrutinib (ARQ 531)

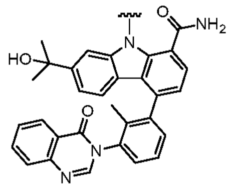
BMS-935177

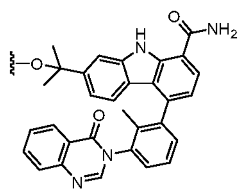
BMS-935177

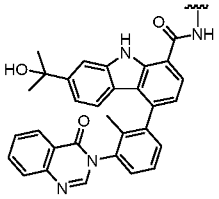
BMS-935177

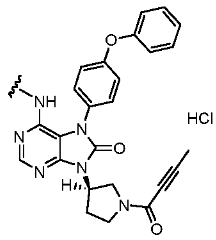
HCl

tirabrutinib(ONO-4059) hydrochloride

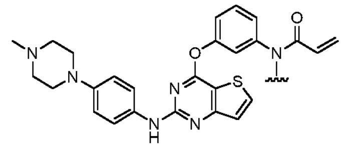
Olmutinib (BI 1482694)

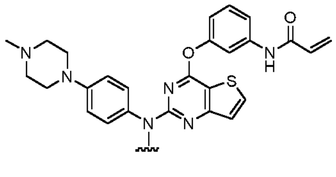
Olmutinib (BI 1482694)

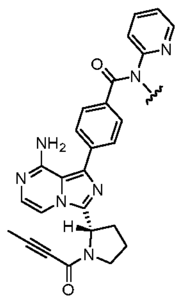
Acalabrutinib (ACP-196)

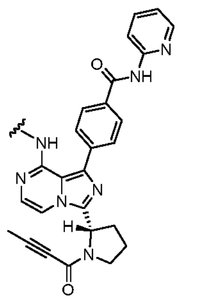
Acalabrutinib (ACP-196)

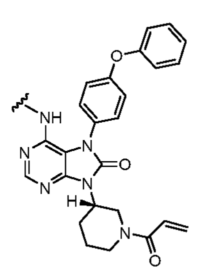
ONO-4059 analogue

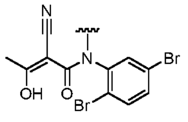
LFM-A13

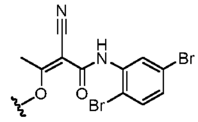
LFM-A13

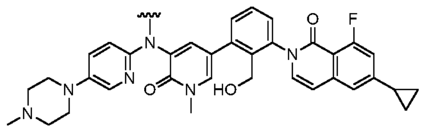
RN486

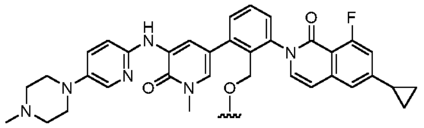
RN486

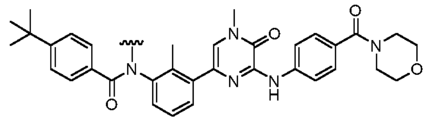
CGI1746

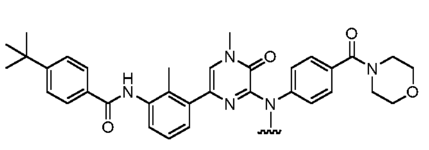
CGI1746

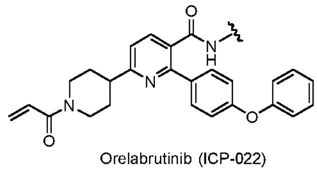
Orelabrutinib (ICP-022)

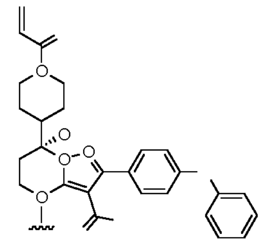
Zanubrutinib

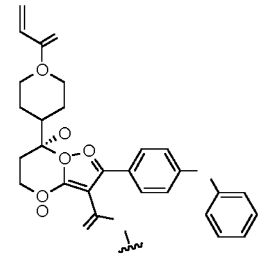
Zanubrutinib

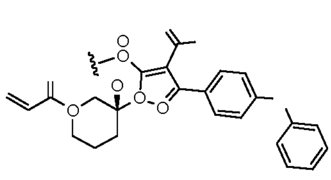
and
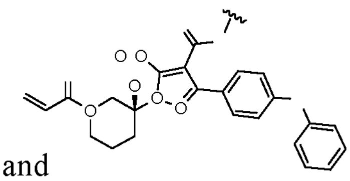
.

9. The prodrug molecule of any one of claims 1-8, or pharmaceutically acceptable salts, hydrates or solvates thereof, wherein, G group is selected from the group consisting of:

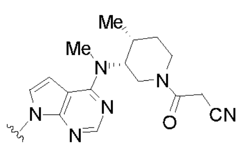
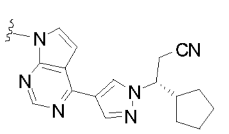
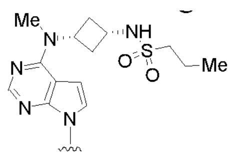
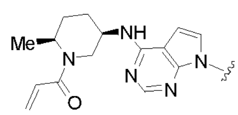
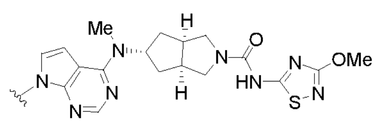
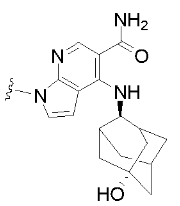
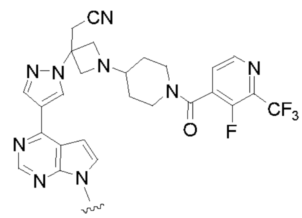

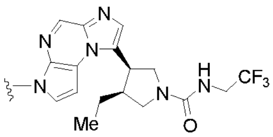
and
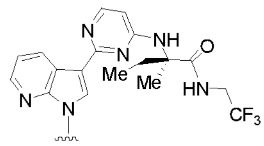

**10.** The prodrug molecule of any one of claims 1-9, or pharmaceutically acceptable salts, hydrates or solvates thereof, wherein, the compound of formula (I) has the structure shown in formula (IIB) or (IIC):

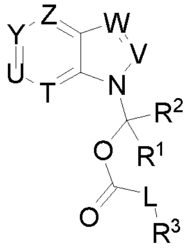
**(IIB)**

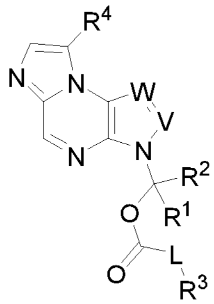
**(IIC)**

wherein,

Z, T, U, V, and W are each independently N or $CR^4$;
Y is N or $CR^5$;
wherein, $R^4$ and $R^5$ are each independently selected from the group consisting of H, halogen, -CN, $-C(O)NH_2$, and

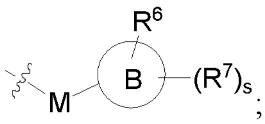
;

M is selected from the group consisting of chemical bond, C(O), C(O)O, S(O), $S(O)_2$, $NR^8$, 5-7-membered heteroaryl, and 5-7-membered heteroaryl $(CHR^8)$-;
ring B is selected from the group consisting of 5-7 membered heteroaryl, 4-10 membered heterocyclyl, C4-C10 cycloalkyl, or 4-10 membered heterocyclyl substituted with 4-10 membered heterocyclyl (wherein, the heteroaryl, cycloalkyl, or heterocyclyl comprises monocyclic, fused, spiro or bridged ring);
$R^8$ is selected from the group consisting of H, C1-C4 alkyl, and C2-C6 cyanoalkyl;
$R^6$ is selected from the group consisting of C1-C4 alkyl, C2-C6 cyanoalkyl, - $C(O)CH_2CN$, $-C(O)CH=CH_2$, $-C(O)NHR^7$, $-NHS(O)_2R^7$, $-NHC(R^8)_2C(O)NHR^7$, and - $C(O)NHR^7$;
$R^7$ is selected from the group consisting of -OH, halogen, C1-C6 alkyl, C1-C6 haloalkyl, C1-C6 alkoxy, 5-7 membered heteroaryl, and C2-C6 cyanoalkyl; wherein, the heteroaryl may be substituted with one or more substituents selected from the group consisting of -OH, halogen, C1-C6 alkyl, C1-C6 haloalkyl, and C1-C6 alkoxy;
s is selected from 0 or 1.

**11.** The prodrug molecule of claim 10, wherein,

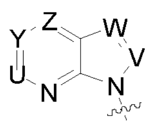

is selected from the following structure:

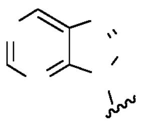 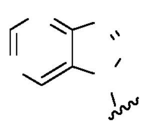 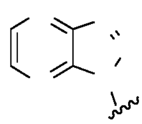 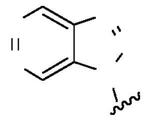,

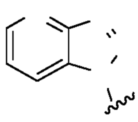 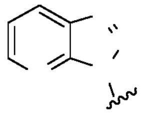 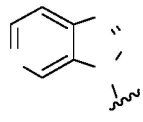 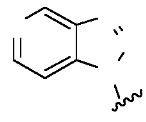, and 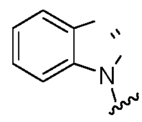.

**12.** The prodrug molecule of any one of claims 1-11, or pharmaceutically acceptable salts, hydrates or solvates thereof, wherein, the compound of formula (I) has the structure shown in formula (IIA):

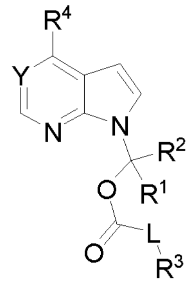

**(IIA)**

wherein,

Y is N or C-C(O)$NH_2$;
$R^4$ is selected from the group consisting of

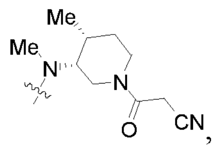, 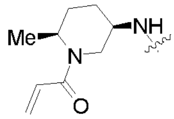, 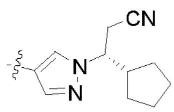,

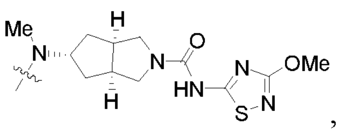, 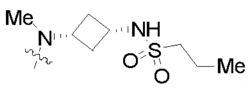, 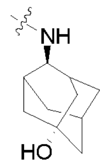, and 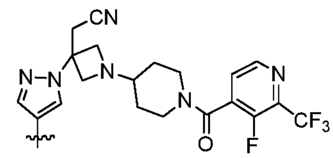.

**13.** The prodrug molecule of any one of claims 1-12, or pharmaceutically acceptable salts, hydrates or solvates thereof, wherein, the compound of formula (I) has the structure selected from the group consisting of:

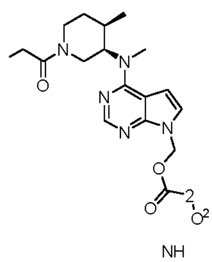 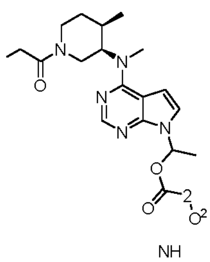 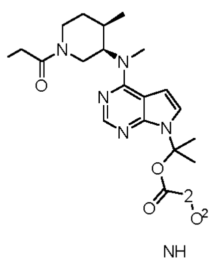

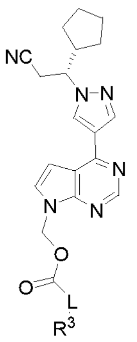
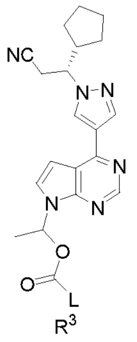
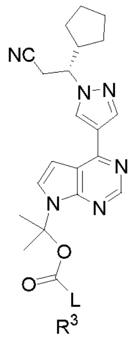

II-A II-B II-C

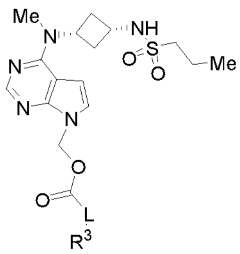
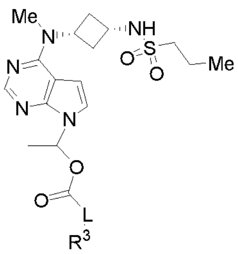
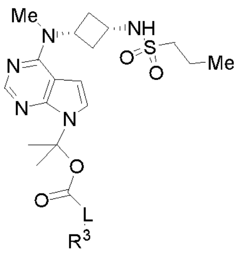

III-A III-B III-C

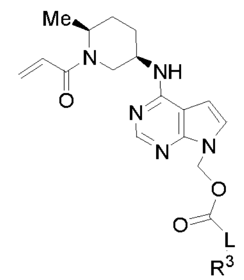
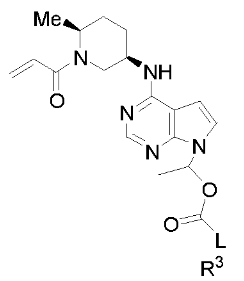
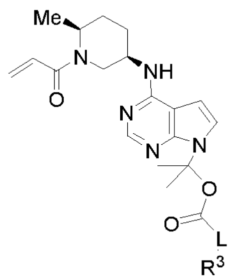

IV-A IV-B IV-C

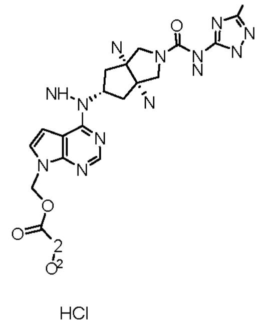
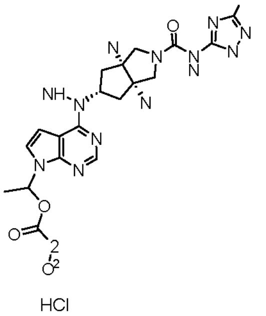
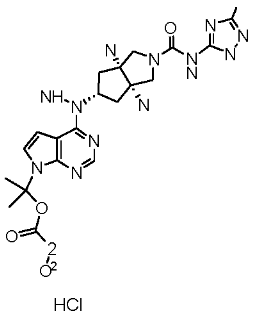

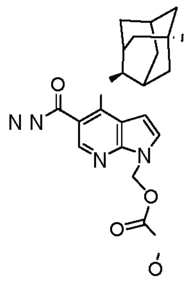

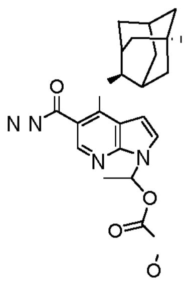

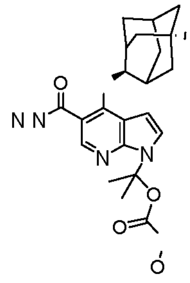

VI-C

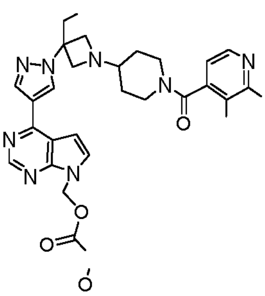

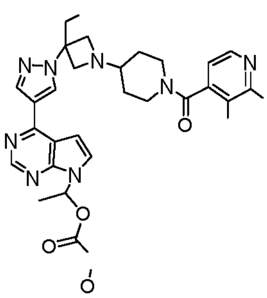

VII-B

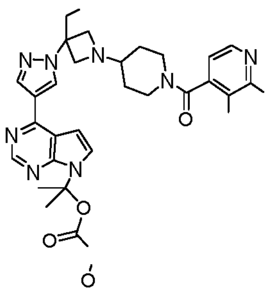

VII-B

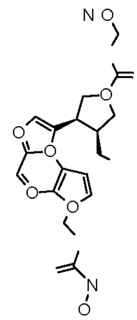

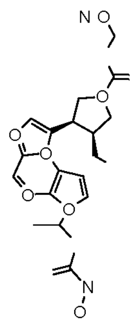

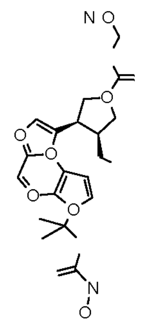

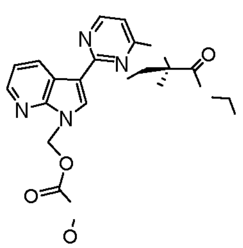

VI-C

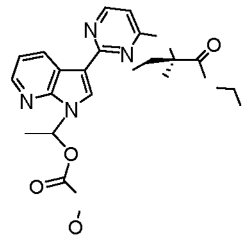

VI-C

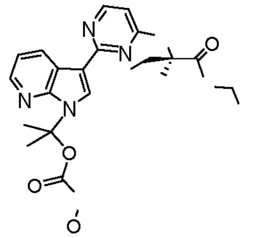

VI-C

.

**14.** The prodrug molecule of any one of claims 1-13, or pharmaceutically acceptable salts, hydrates or solvates thereof, wherein, the compound of formula (I) has the structure selected from the group consisting of:

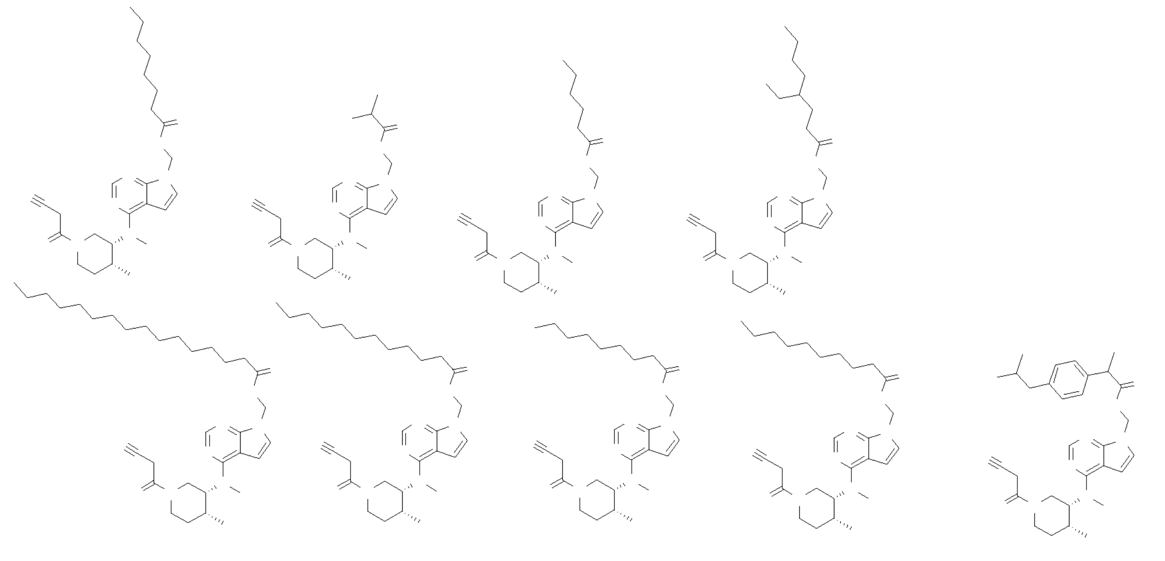

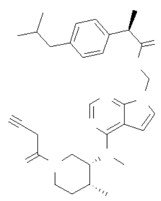

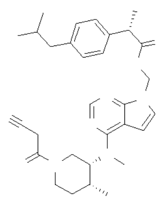

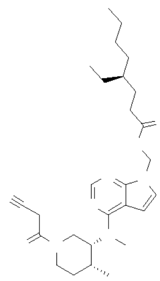

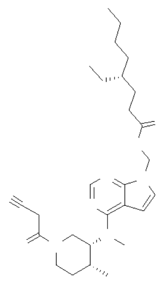

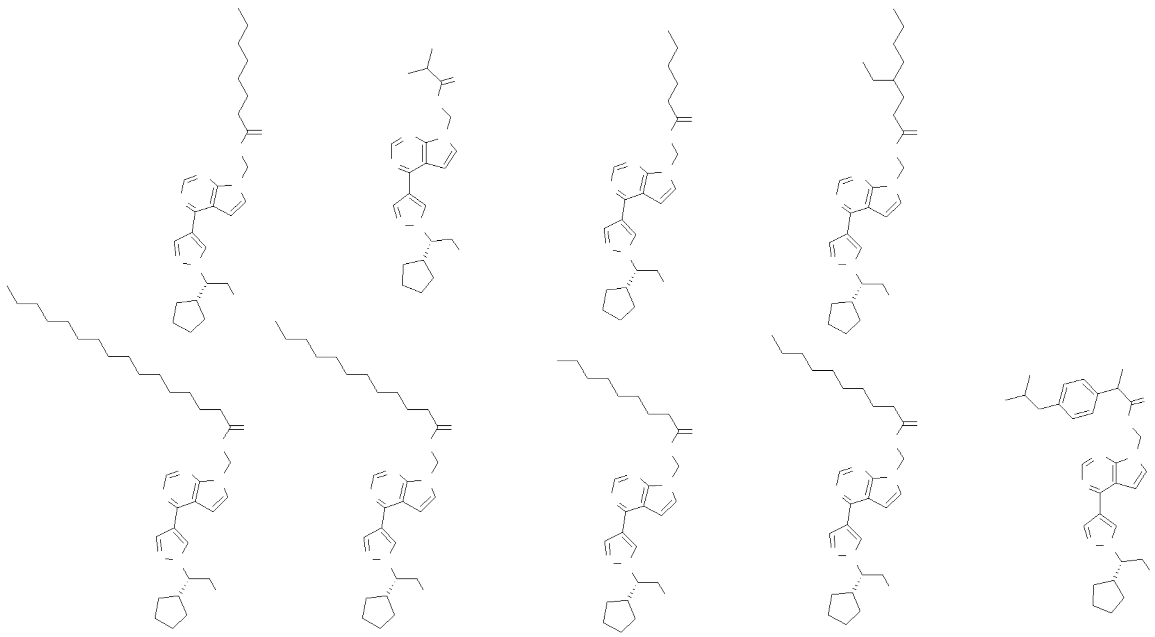

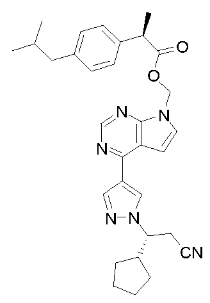

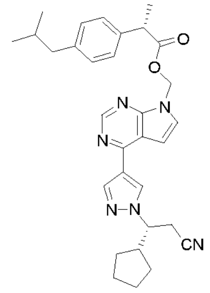

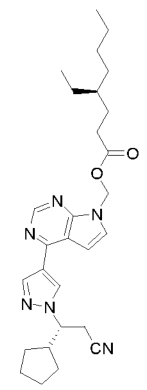

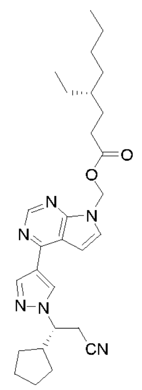

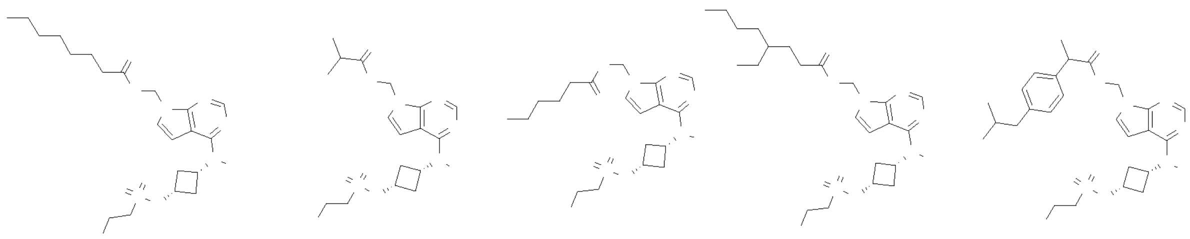

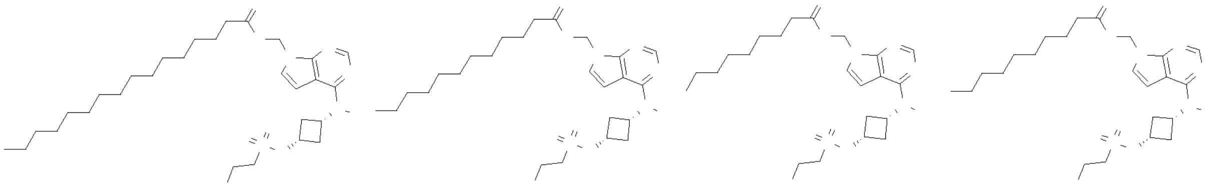

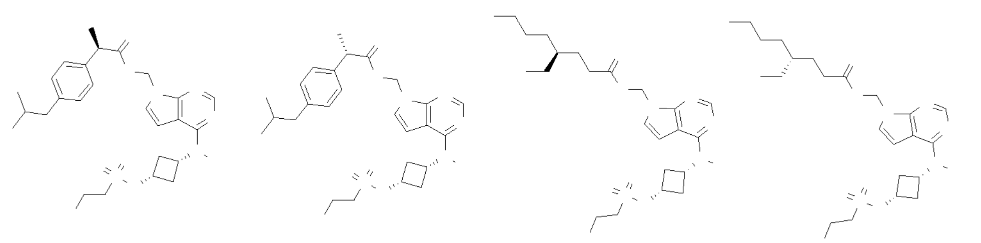

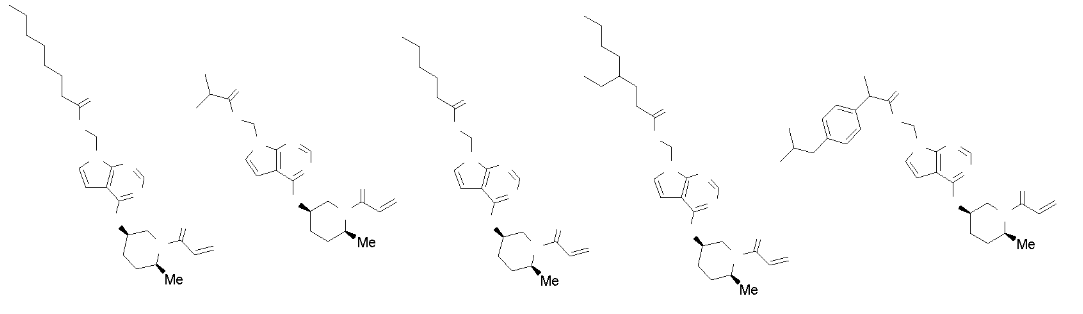

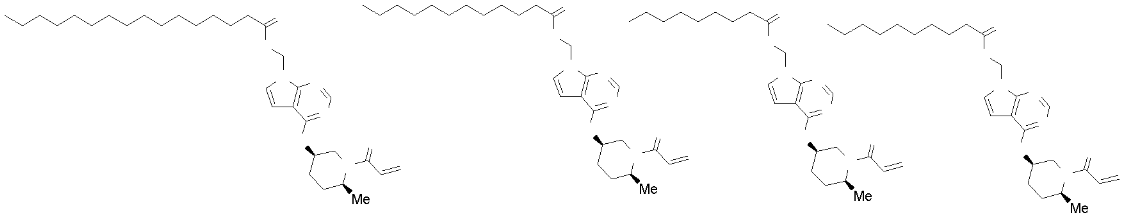

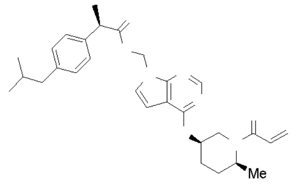

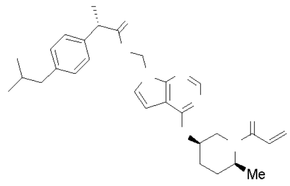

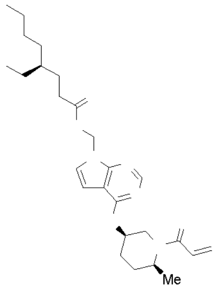

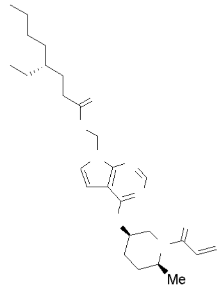

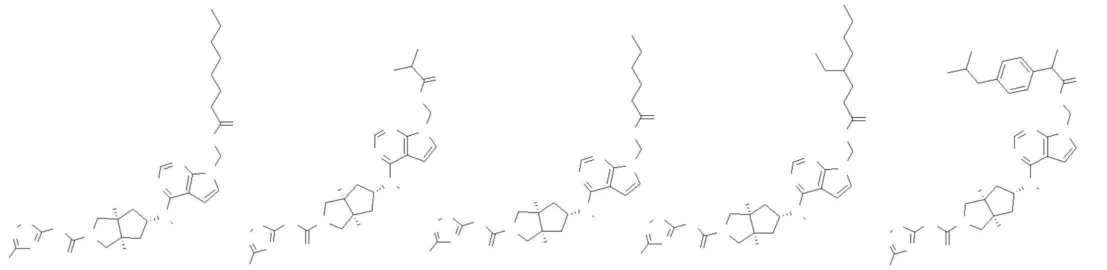

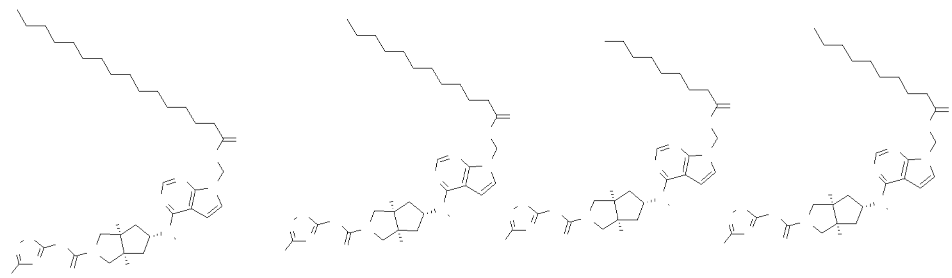

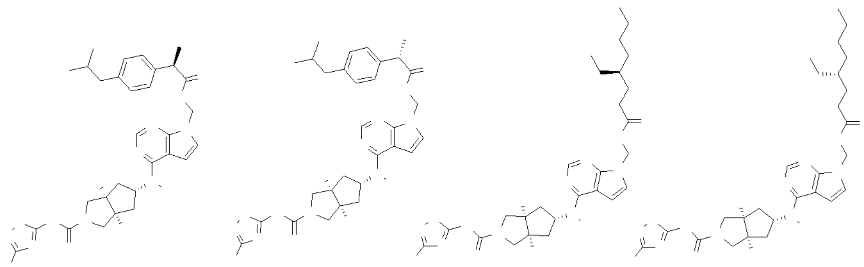

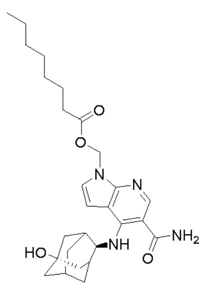

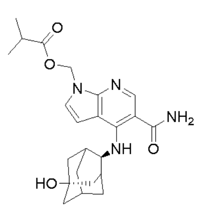

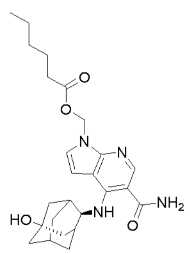

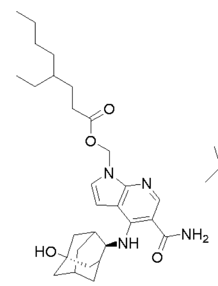

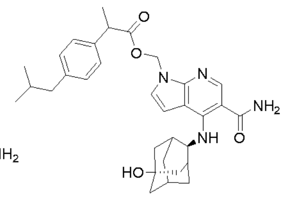

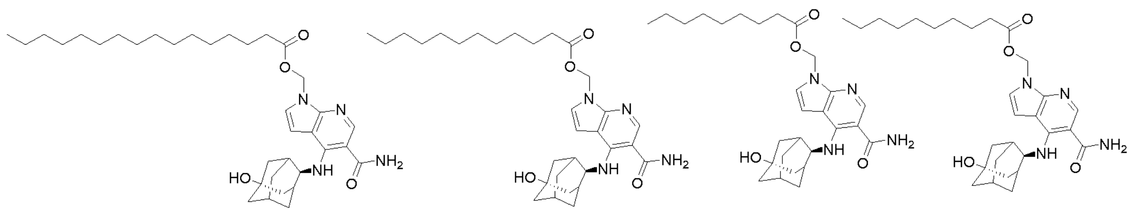

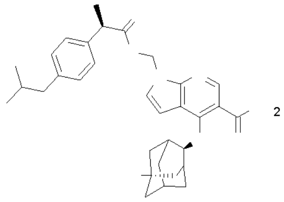

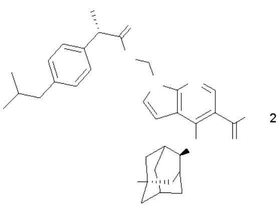

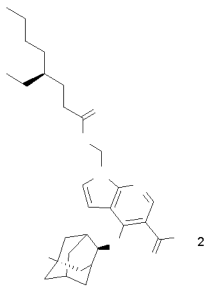

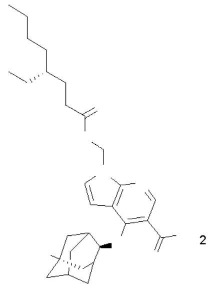

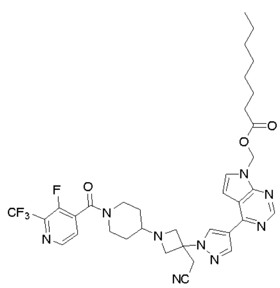

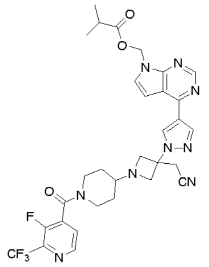

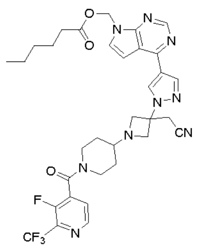

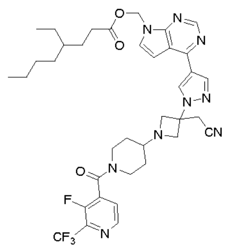

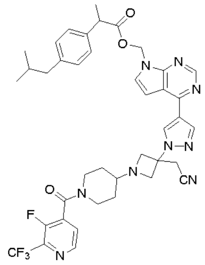

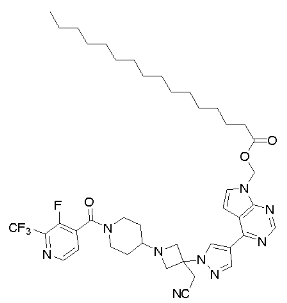

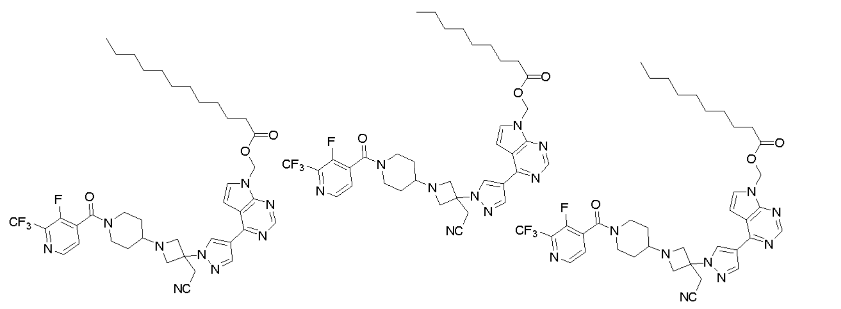

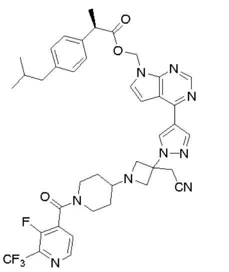

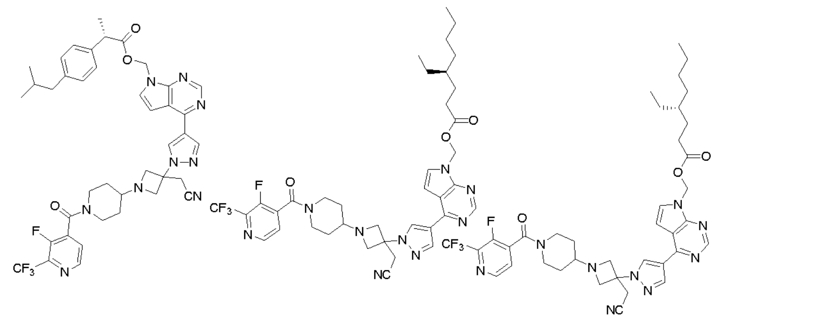

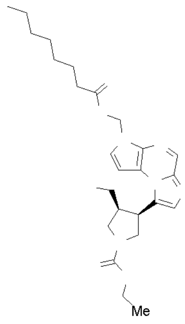
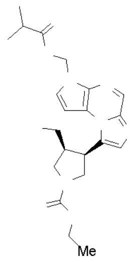
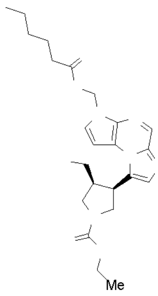
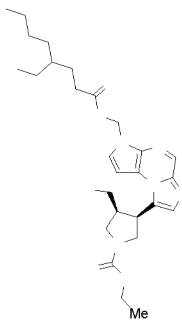
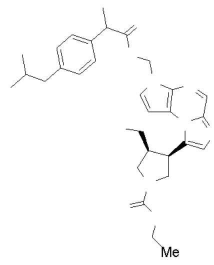

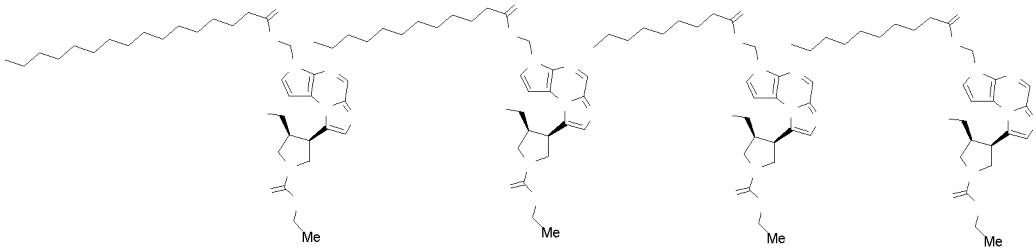

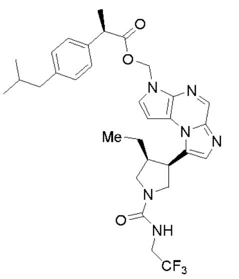
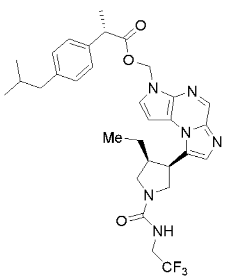
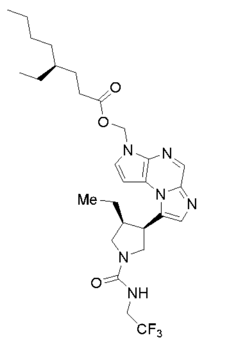
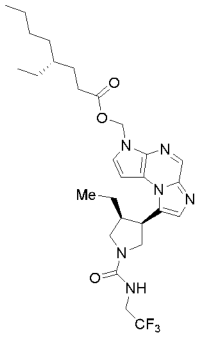

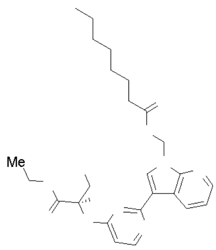
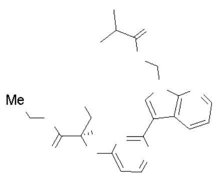
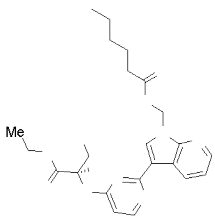
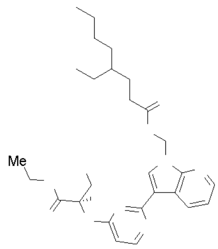
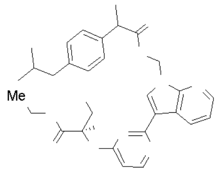

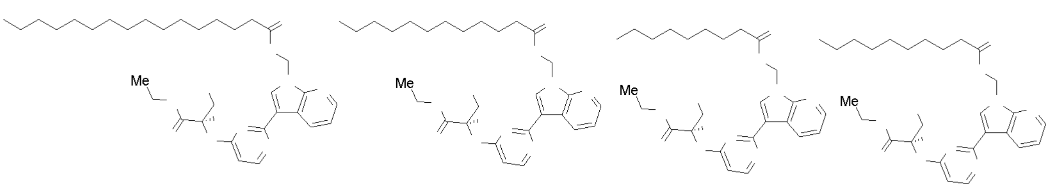

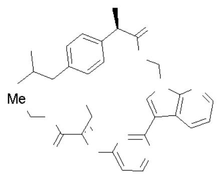 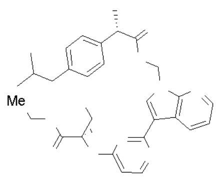 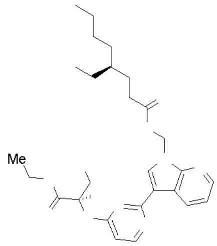 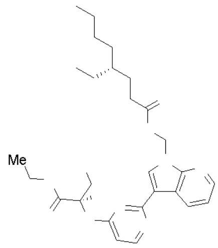

15. The prodrug molecule of any one of claims 1-14, or pharmaceutically acceptable salts, hydrates or solvates thereof, wherein, the compound of formula (I) has the structure selected from the group consisting of:

| | | | |
|---|---|---|---|
| 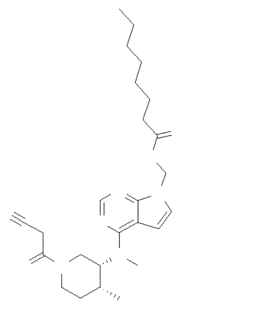 | 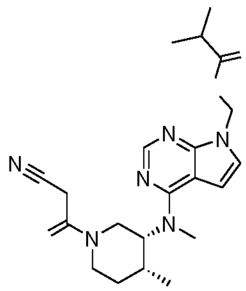 | 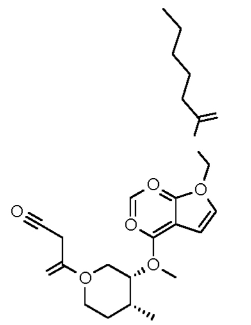 | 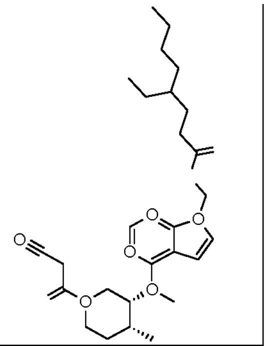 |
| 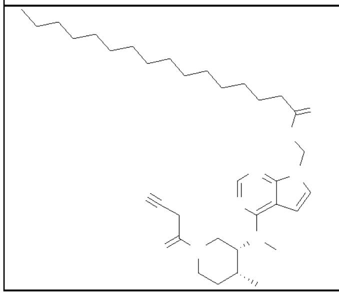 | 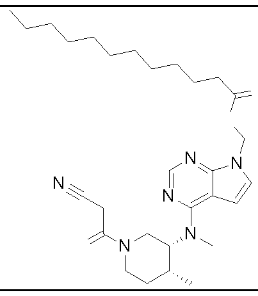 | 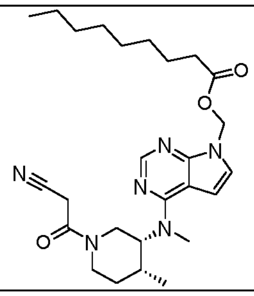 | 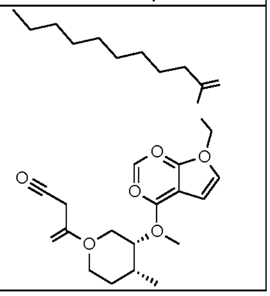 |

| | | | |
|---|---|---|---|
| 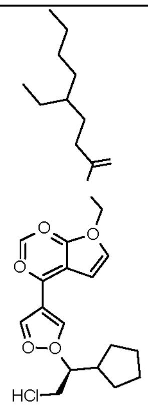 | 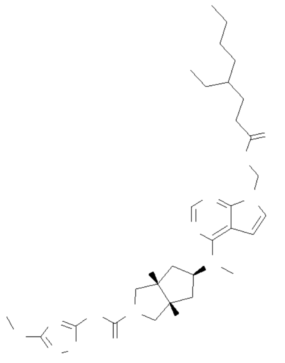 | 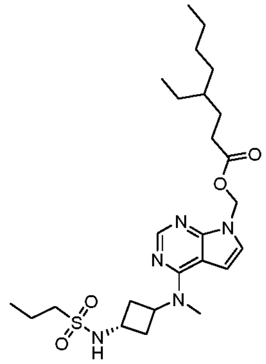 | 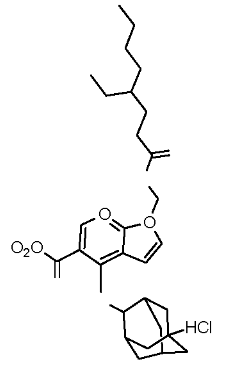 |
| 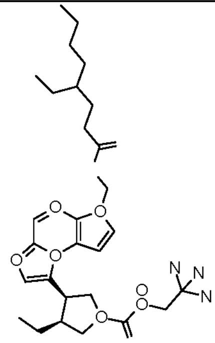 | 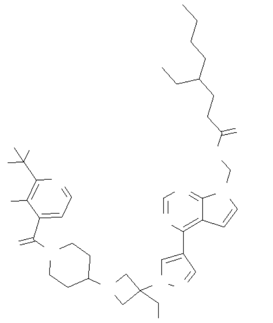 | 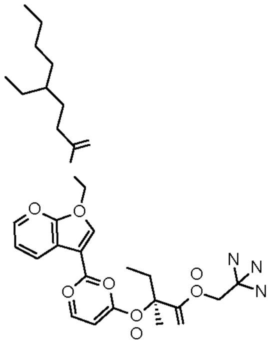 | 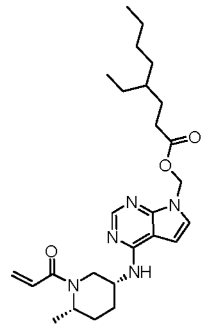 |

**16.** A method for preparing a compound of claim 12, including steps of:

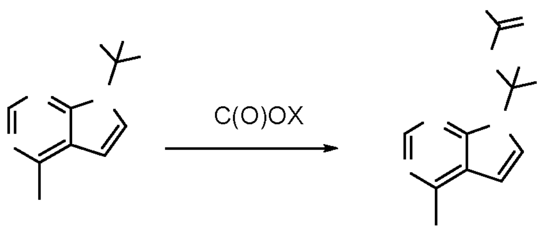


compound of formula 2e reacts with $R^3C(O)X$ in an inert solvent to obtain compound of formula (IIA); wherein Y is N or C-C(O)$NH_2$, X is OH or activating group (preferably halogen or OC(O)$R^3$) and each of the remaining groups is as defined in claim 12.

**17.** An intermediate of formula 2e:

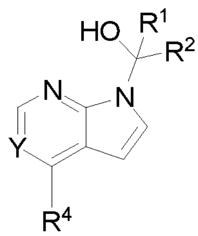

2e ;

wherein, Y is N or C-C(O)$NH_2$; each of the remaining groups is as defined in claim 12.

**18.** A pharmaceutical composition, comprising pharmaceutically acceptable carriers and the compound of any one of

claims 1-13, or pharmaceutically acceptable salts, or hydrates or solvates thereof.

**19.** Use of the compound of any one of claims 1-15, or pharmaceutically acceptable salts or hydrates thereof, in the preparation of a pharmaceutical composition for the treatment or prevention of a disease associated with the activity or expression of JAK kinase.

**20.** A topical drug delivery formulation, comprising:

the compound of any one of claims 1-15;
optional skin penetration enhancer, preferably, the skin penetration enhancer is selected from the group consisting of surfactants, dimethylsulfoxide and its analogs, azones, pyrrolidone derivatives, alcohols, ethers, fatty acids and fatty acid esters, and combinations thereof; and
optional supporting layer;
preferably, the drug is present in a single phase or in multiple phases, in solution or suspension; the formulation is administered as a solution, suspension, gel, emulsion, cream or foam.

**21.** A method for improving the membrane permeability of drug molecule G', comprising steps of: the drug molecule G' is modified so as to introduce fragment

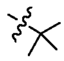

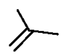,

into the molecule

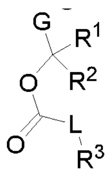

to form (I) with CLogP > 4, while the CLogP of the modified prodrug molecule (I) is increased by at least 1 over the CLogP of the drug molecule G'.

**22.** The method according to claim 21, wherein, the Skin-Pama Pe value of the prodrug

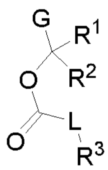

molecule ([1]) is increased by 2-100 folds over the parent drug molecule G'.

**INTERNATIONAL SEARCH REPORT**

International application No.
**PCT/CN2022/075714**

**A. CLASSIFICATION OF SUBJECT MATTER**

C07D 403/14(2006.01)i; A61P 37/02(2006.01)i; A61K 47/54(2017.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CJFD, REGISTRY, CAPLUS, MARPAT: sub-structure search based on all-level general formulae and embodiments, 明慧医药, 明慧医药(杭州)有限公司, skin-pampa, pampa, 吡罗昔康, tofacitinib, itacitinib, upadacitinib, Clog P, LogPe, 有效渗透系数, 透皮, 透膜, 外用, 前药, JAK, Transdermal, Permeable, membrane, prodrug, topical, Effective permeability coefficient

**C. DOCUMENTS CONSIDERED TO BE RELEVANT**

| Category* | Citation of document, with indication, where appropriate, of the relevant passages | Relevant to claim No. |
|---|---|---|
| A | WO 2020176859 A1 (PURETECH LYT INC) 03 September 2020 (2020-09-03)<br>embodiments | 1-22 |
| Y | WO 2020191477 A1 (INTEGRATED NANOTHERAPEUTICS INC) 01 October 2020 (2020-10-01)<br>embodiment 5, compounds INT-D081, 082, description paragraphs 31-32, 49-50, 104-105, 385, 387-390, table 6 | 1-22 |
| X | WO 2010083283 A2 (INCYTE CORP.) 22 July 2010 (2010-07-22)<br>description paragraphs 1046, 1198, compounds (R)-12, 20, (R)-21 | 1-13 |
| A | CN 110734428 A (JIAXING TEKELUO BIOTECHNOLOGY CO., LTD.) 31 January 2020 (2020-01-31)<br>embodiments | 1-22 |
| X | CN 111763209 A (CHIA TAI TIANQING PHARMACEUTICAL GROUP CO., LTD.) 13 October 2020 (2020-10-13)<br>embodiment 4, compound XIV | 17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 April 2022** | **27 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

INTERNATIONAL SEARCH REPORT

International application No.
**PCT/CN2022/075714**

**C. DOCUMENTS CONSIDERED TO BE RELEVANT**

| Category* | Citation of document, with indication, where appropriate, of the relevant passages | Relevant to claim No. |
|---|---|---|
| Y | 钱晶晶 等 (QIAN, Jingjing et al.). "前体药物技术在经皮给药系统中的应用 (Application of Prodrug Technology in Transdermal Drug Delivery System)" *中国医药工业杂志 (Chinese Journal of Pharmaceuticals),* No. 08, 10 August 2011 (2011-08-10), parts 1, 4 and 6 | 1-22 |
| PX | WO 2022012492 A1 (COVAL BIOPHARMA SHANGHAI CO LTD) 20 January 2022 (2022-01-20) embodiments, page 202, Intermediates | 17 |

Form PCT/ISA/210 (second sheet) (January 2015)

**INTERNATIONAL SEARCH REPORT**
**Information on patent family members**

International application No.
**PCT/CN2022/075714**

| Patent document cited in search report | Publication date (day/month/year) | Patent family member(s) | Publication date (day/month/year) |
|---|---|---|---|
| WO 2020176859 A1 | 03 September 2020 | CA 3130349 A1 | 03 September 2020 |
| | | EP 3930709 A1 | 05 January 2022 |
| | | AU 2020228662 A1 | 09 September 2021 |
| WO 2020191477 A1 | 01 October 2020 | CA 3131977 A1 | 01 October 2020 |
| | | CN 113853368 A | 28 December 2021 |
| | | EP 3941897 A1 | 26 January 2022 |
| | | AU 2020245715 A1 | 19 August 2021 |
| | | KR 20210142152 A | 24 November 2021 |
| WO 2010083283 A2 | 22 July 2010 | US 2018237442 A1 | 23 August 2018 |
| | | CO 6400137 A2 | 15 March 2012 |
| | | EA 201170935 A1 | 28 February 2012 |
| | | JP 2015091805 A | 14 May 2015 |
| | | CN 102348693 A | 08 February 2012 |
| | | CN 105669675 A | 15 June 2016 |
| | | JP 2021100930 A | 08 July 2021 |
| | | CA 2749483 A1 | 22 July 2010 |
| | | ZA 201104909 B | 28 November 2018 |
| | | MA 33038 B1 | 01 February 2012 |
| | | TN 2011000329 A1 | 27 March 2013 |
| | | MX 2011007102 A | 15 September 2011 |
| | | NZ 617077 A | 29 May 2015 |
| | | EP 2398774 A2 | 28 December 2011 |
| | | PL 2398774 T3 | 30 November 2017 |
| | | JO P20190231 A1 | 16 June 2017 |
| | | AU 2020201709 A1 | 26 March 2020 |
| | | TW 201524982 A | 01 July 2015 |
| | | BR PI1006794 A2 | 25 August 2015 |
| | | CL 2011001668 A1 | 03 February 2012 |
| | | IL 242752 A | 29 June 2017 |
| | | PE 20160788 A1 | 04 September 2016 |
| | | MY 163540 A | 15 September 2017 |
| | | US 2013253191 A1 | 26 September 2013 |
| | | CA 3121743 A1 | 22 July 2010 |
| | | AU 2010204772 A1 | 04 August 2011 |
| | | TW 201038575 A | 01 November 2010 |
| | | KR 20210130846 A | 01 November 2021 |
| | | SG 172468 A1 | 29 August 2011 |
| | | NZ 725585 A | 25 May 2018 |
| | | AU 2016216537 A1 | 01 September 2016 |
| | | EC SP11011268 A | 30 December 2011 |
| | | EA 201400575 A1 | 27 February 2015 |
| | | TW 202118766 A | 16 May 2021 |
| | | KR 20210022770 A | 03 March 2021 |
| | | CL 2013002617 A1 | 28 March 2014 |
| | | US 2013253193 A1 | 26 September 2013 |
| | | KR 20190083010 A | 10 July 2019 |
| | | SG 10201913261Q A | 30 March 2020 |
| | | US 2016257687 A1 | 08 September 2016 |
| | | US 2020002341 A1 | 02 January 2020 |
| | | TW 202146417 A | 16 December 2021 |

Form PCT/ISA/210 (patent family annex) (January 2015)

**INTERNATIONAL SEARCH REPORT**
**Information on patent family members**

International application No.
**PCT/CN2022/075714**

| Patent document cited in search report | | | Publication date (day/month/year) | Patent family member(s) | | | Publication date (day/month/year) |
|---|---|---|---|---|---|---|---|
| | | | | IL | 283038 | D0 | 30 June 2021 |
| | | | | PE | 02.11.2017 | A1 | 07 August 2017 |
| | | | | PT | 2398774 | T | 29 September 2017 |
| | | | | JP | 2020073491 | A | 14 May 2020 |
| | | | | IL | 242750 | A | 30 June 2019 |
| | | | | NZ | 706894 | A | 25 November 2016 |
| | | | | AR | 082747 | A1 | 09 January 2013 |
| CN | 110734428 | A | 31 January 2020 | WO | 2021078020 | A1 | 29 April 2021 |
| CN | 111763209 | A | 13 October 2020 | JP | 2019506379 | A | 07 March 2019 |
| | | | | US | 2019023712 | A1 | 24 January 2019 |
| | | | | ES | 2882118 | T3 | 01 December 2021 |
| | | | | EP | 3398952 | A1 | 07 November 2018 |
| | | | | CN | 108699063 | A | 23 October 2018 |
| | | | | WO | 2017114461 | A1 | 06 July 2017 |
| WO | 2022012492 | A1 | 20 January 2022 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington: The Science and Practice of Pharmacy. Mack Publishing Co, 1995